# EUROPEAN PATENT SPECIFICATION

(11) **EP 2 167 083 B1**
(45) Date of publication and mention of the grant of the patent: **28.10.2015**
(21) Application number: 08768192.0
(22) Date of filing: 06.06.2008
(51) Int. Cl.: A61K 31/44, A61K 31/541, A61P 25/24, C07D 401/04, C07D 403/04, C07D 405/04, C07D 409/04, C07D 417/04

(54) **1- HETEROARYL-3-AZABICYCLO[3.1.0]HEXANES, METHODS FOR THEIR PREPARATION AND THEIR USE AS MEDICAMENTS**
1- HETEROARYL-3-AZABICYCLO[3.1.0]HEXANE, VERFAHREN ZU IHRER HERSTELLUNG UND IHRE VERWENDUNG ALS MEDIKAMENTE
1- HÉTÉROARYL-3-AZABICYCLO[3.1.0]HEXANES, LEURS MÉTHODES DE PRÉPARATION ET LEUR UTILISATION COMME MÉDICAMENTS

(30) Priority: 06.06.2007 US 933631 P
(43) Date of publication of application: 31.03.2010
(73) Proprietor: Euthymics Bioscience, Inc., Cambridge, MA 02141 (US)
(72) Inventor: SKOLNICK, Phil, Edgewater, NJ 07020 (US); CHEN, Zhengming, Belle Meade, NJ 08520 (US)
(74) Representative: Bond, Christopher William
(86) International application number: PCT/US2008/007116
(87) International publication number: WO 2008/153937

(56) References cited:
- WO-A1-2005/080382
- WO-A1-2006/108701
- WO-A1-2007/006117
- WO-A1-2007/022933
- WO-A1-2007/022980
- WO-A2-2006/096810
- WO-A2-2007/022934
- ZHANG M ET AL: "Studies on the structure-activity relationship of bicifadine analogs as monoamine transporter inhibitors" BIOORGANIC & MEDICINAL CHEMISTRY LETTERS, PERGAMON, ELSEVIER SCIENCE, GB LNKD- DOI:10.1016/J.BMCL.2008.05.077, vol. 18, no. 13, 1 July 2008 (2008-07-01), pages 3682-3686, XP022716275 ISSN: 0960-894X [retrieved on 2008-05-23]
- SKOLNICK P ET AL: 'Antidepressant-like actions of DOV 21,947: a "triple" reuptake inhibitor' EUROPEAN JOURNAL OF PHARMACOLOGY vol. 461, 2003, pages 99 - 104, XP002306753

## Description

### Technical Field

The present invention relates to novel 1-heteroaryl-3-azabicyclo[3.1.0]hexanes, intermediates for the production thereof and methods for preparing, formulating, and using 1-heteroaryl-3-azabicyclo[3.1.0]hexanes.

### Background of the Invention

Phenyl substituted-3-azabicyclo[3.1.0]hexanes, exemplified by bicifadine (p-toly-3-azabicyclo[3.1.0]hexane) and 1-(3,4-dichlorophenyl)-3-azabicyclo[3.1.0]hexane have been reported to inhibit the uptake of biogenic amines (serotonin, norepinephrine and dopamine) that serve as neurotransmitters in the central nervous system. Basile, A.S., et al., . J. Pharmacol. Exp. Ther., 321:1208-1225 (2007). ; Skolnick, P. et al., Eur. J. Pharmacol. 461:99 (2003); Skolnick, P. et al., Life Sci. 73: 3175-3179 (2003); Skolnick, P., et al., CNS Drug Reviews (2006)] Related compounds have also been described in U.S. Patent Application Serial No. 11/371,178 filed on March 7, 2006 (published as US 2006-0223875 A1), which is related to and claims priority from US Provisional Applications 60/661,662, filed on March 8, 2005 and 60/701,562 filed on July 22, 2005. Biogenic amines are widely distributed throughout the central nervous system and have been implicated in a variety of neurological and psychiatric disorders ranging from acute and chronic pain syndromes, and neuropathic disorders, to affective disorders such as depression and anxiety. [Briley, M., Hum. Psychopharmacol. Clin. Exp. 19:S21-S25 (2004); Skolnick, P. in "Dopamine and glutamate in psychiatric disorders, " W. Schmidt, Editor; Humana Press, Totowa, Chapter 9, pp. 199-214 (2005)]. Compounds inhibiting the uptake of these neurotransmitters (resulting in increased concentrations of norepinephrine, serotonin and/or dopamine in the synaptic cleft) may therefore be useful in treating these disorders. [Skolnick, P., et al., CNS Drug Reviews (2006); Briley, M., Hum. Psychopharmacol. Clin. Exp. 19:S21-S25 (2004)]

The effectiveness of a neurotransmitter uptake inhibitor in treating a neuropsychiatric disorder depends upon, at least in part, its relative potency as an inhibitor of serotonin versus norepinephrine versus dopamine uptake. For example, serotonin selective reuptake inhibitors (SSRIs) are commonly prescribed as antidepressants but are not very effective in treating pain [Atkinson, J.H. et al., Pain 83:137-145 (1999)], while compounds that inhibit the uptake of norepinephrine and serotonin (NSRIs) are useful in treating both depression and pain. Compounds inhibiting the reuptake of all three biogenic amines may have certain therapeutic advantages (e.g., faster onset; superior side effect profile) than single or dual uptake inhibitors. [Skolnick, P. et al., Eur. J. Pharmacol. 461:99 (2003); Skolnick, P. et al., Life Sci. 73: 3175-3179 (2003); Skolnick, P. in "Dopamine and glutamate in psychiatric disorders, " W. Schmidt, Editor; Humana Press, Totowa, Chapter 9, pp. 199-214(2005)]

The ability of phenyl substituted azabicyclohexanes to inhibit biogenic amine uptake is effected through binding to a family of distinct but related transport proteins that have been identified and can readily be expressed as recombinant proteins. [Povlock, S.L. and Amara, S.G., in "Neurotransmitter transporters: structure, junction, and regulation," Reith MEA, Editor, Humana Press, Totowa, pp.1-28 (1997); Eshleman, A.J. et al., Journal of Pharmacology & Experimental Therapeutics 289:877-885 (1999)] The potency of a compound to inhibit biogenic amine uptake can be measured by standard neurochemical techniques [Skolnick, P. et al., Eur. J. Pharmacol. 461:99 (2003)] including radioligand binding and [³H]biogenic amine (e.g., serotonin) uptake using such recombinant proteins expressed in, for example, a mammalian cell line. Such techniques are well known to those skilled in the art.

Notwithstanding the foregoing, there remains a compelling need to identify additional compounds that inhibit the reuptake of one, two, or three of these biogenic amines for the treatment of a variety of neurological and psychiatric disorders.

### Summary of Exemplary Embodiments of the Invention

The present invention provides novel compounds capable of inhibiting the reuptake of multiple biogenic amines linked to neurological and psychiatric disorders, related compositions and said compounds and compositions for treating and managing a variety of neurological and psychiatric disorders, including depression and anxiety.

### Detailed Description of Exemplary Embodiments of the Invention

The present invention is as defined in the claims.

Aspects of the present invention are now described with reference to the following clauses:
1. A compound of the following Formula I: 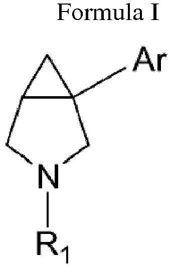 and pharmaceutically acceptable salts, enantiomers, polymorphs, solvates, or hydrates thereof, wherein:
   Ar is a heterocyclic aryl group selected from furan, methylfuran, pyrrole, imidazole, pyrazole, triazole, tetrazole, thiazole, isothiazole, methoxypyridine, pyridazine, pyrazine, triazine, indole, methylindole, benzofuran, benzothiophene, benzothiazole, isoquinoline, cinnoline, phthalazine, quinazoline, chromane and isochromane, and Ar is either unsubstituted or substituted with one or more substituents independently selected from fluoro, chloro, bromo, iodo, -NO₂, -CN,-NH₂, carboxy, C₂₋₈ alkenyl, C₂₋₈ alkynyl, halo(C₁₋₈)alkyl, hydroxy, trifluoromethyl, C₃₋₈ cycloalkyl, C₁₋₃ alkoxy, C₁₋₃ alkoxy(C₁₋₃)alkyl, carboxy(C₁₋₃)alkyl, C₁₋₃ alkanoyl, halo(C₁₋₃)alkoxyl, C₁₋₈ alkylamino, and di(C₁₋₈)alkylamino; and
   R₁ is selected from hydrogen, unsubstituted C₁₋₁₀ alkyl, C₃₋₈ cycloalkyl, C₂₋₁₀ alkenyl, and C₃₋₁₀ alkynyl, and substituted C₁₋₁₀ alkyl, C₃₋₁₀ alkenyl and C₃₋₁₀ alkynyl wherein the substituent is one or more of hydroxy, cyano, halogen, C₁₋₆ alkoxy, aryl substituted C₁₋₆ alkoxy, aryloxy, aryloxy substituted with one or more halogens, C₁₋₆ alkyl, C₁₋₆ alkyl independently substituted with one or more of cyano and halogen, C₁₋₄ alkoxy, and C₁₋₄ haloalkoxy.
2. The compound according to clause 1 and pharmaceutically acceptable salts, enantiomers, polymorphs, solvates, or hydrates thereof, wherein Ar is furan, methylfuran, fluorobenzothiophene, methoxypyridine, benzofuran, benzothiophene, chlorobenzothiophene, indole or methylindole and R₁ is hydrogen or unsubstituted C₁₋₁₀ alkyl.
3. The compound according to clause 2 and pharmaceutically acceptable salts, enantiomers, polymorphs, solvates, or hydrates thereof, wherein R₁ is hydrogen, methyl, ethyl, propyl or isopropyl.
4. The compound according to clause 1, wherein the compound is selected from the group consisting of:
   1-(5-methylfuran-2-yl)-3-azabicyclo[3.1.0]hexane;
   1-(6-methoxypyridin-3-yl)-3-azabicyclo[3.1.0]hexane;
   1-(6-methoxypyridin-3-yl)-3-methyl-3-azabicyclo[3.1.0]hexane;
   1-(benzofuran-2-yl)-3-methyl-3-azabicyclo[3.1.0]hexane;
   1-(benzofuran-3-yl)-3-azabicyclo[3.1.0]hexane;
   1-(benzofuran-3-yl)-3-methyl-3-azabicyclo[3.1.0]hexane;
   1-methyl-2-(3-methyl-3-azabicyclo[3.1.0]hexan-1-yl)-1*H*-indole;
   2-(3-ethyl-3-azabicyclo[3.1.0]hexan-1-yl)-1-methyl-1*H*-indole;
   2-(3-isopropyl-3-azabicyclo[3.1.0]hexan-1-yl)-1-methyl-1*H*-indole;
   1-methyl-5-(3-methyl-3-azabicyclo[3.1.0]hexan-1-yl)-1*H*-indole;
   5-(3-ethyl-3-azabicyclo[3.1.0]hexan-1-yl)-1-methyl-1*H*-indole;
   5-(3-isopropyl-3-azabicyclo[3.1.0]hexan-1-yl)-1-methyl-1*H*-indole;
   1-(5-chlorobenzo[*b*]thiophen-3-yl)-3-azabicyclo[3.1.0]hexane;
   1-(5-chlorobenzo[*b*]thiophen-2-yl)-3-methyl-3-azabicyclo[3.1.0]hexane;
   1-(5-chlorobenzo[*b*]thiophen-3-yl)-3-methyl-3-azabicyclo[3.1.0]hexane;
   1-(benzo[*b*]thiophen-2-yl)-3-aza-bicyclo[3.1.0]hexane;
   1-(benzo[*b*]thiophen-2-yl)-3-methyl-3-aza-bicyclo[3.1.0]hexane;
   1-(6-fluorobenzo[*b*]thiophen-2-yl)-3-aza-bicyclo[3.1.0]hexane;
   1-(6-fluorobenzo[*b*]thiophen-2-yl)-3-methyl-3-aza-bicyclo[3.1.0]hexane;
   1-(5-fluorobenzo[*b*]thiophen-2-yl)-3-aza-bicyclo[3.1.0]hexane;
   1-(5-fluorobenzo[*b*]thiophen-2-yl)-3-methyl-3-aza-bicyclo[3.1.0]hexane;
   N,N-dimethyl-2-(3-methyl-3-azabicyclo[3.1.0]hexane-1-yl)benzo[*b*]thiophen-5-amine;
   1-(5-bromobenzo[*b*]thiophen-2-yl)-3-azabicyclo[3.1.0]hexane;
   1-(5-bromobenzo[*b*]thiophen-2-yl)-3-methyl-3-azabicyclo[3.1.0]hexane;
   2-(3-azabicyclo[3.1.0]hexan-1-yl)-*N,N*-dimethylbenzo[b]thiophen-5-amine;
   2-(3-ethyl-3-azabicyclo[3.1.0]hexan-1-yl)-*N*,N-dimethylbenzo[*b*]thiophen-5-amine;
   2-(3-isopropyl-3-azabicyclo[3.1.0]hexan-1-yl)-*N,N*-dimethylbenzo[*b*]thiophen-5-amine;
   1-(6-bromobenzo[*b*]thiophen-2-yl)-3-azabicylco[3.1.0]hexane;
   1-(6-bromobenzo[*b*]thiophen-2-yl)-3-methyl-3-azabicyclo[3.1.0]hexane;
   2-(3-azabicyclo[3.1.0]hexan-1-yl)-*N,N*-dimethylbenzo[*b*]thiophen-6-amine;
   *N,N*-dimethyl-2-(3-methyl-3-azabicyclo[3.1.0]hexan-1-yl)benzo[*b*]thiophen-6-amine;
   2-(3-ethyl-3-azabicyclo[3.1.0]hexan-1-yl)-*N,N*-dimethylbenzo[*b*]thiophen-6-amine;
   2-(3-isopropyl-3-azabicyclo[3.1.0]hexan-1-yl)-*N,N*-dimethylbenzo[*b*]thiophen-6-amine; and
   2-(3-azabicyclo[3.1.0]hexan-1-yl)-benzo[*d*]thiazole;
   and pharmaceutically acceptable salts, enantiomers, polymorphs, solvates, or hydrates thereof.
5. The compound according to clause 1 selected from the group consisting of:
   (1*S*,5*S*)-1-(benzo[*b*]thiophen-2-yl)-3-azabicyclo[3.1.0]hexane;
   (1*R*,5*R*)-1-(benzo[*b*]thiophen-2-yl)-3-azabicyclo[3.1.0]hexane;
   (1*S*,5*S*)-1-(benzo[*b*]thiophen-2-yl)-3-methyl-3-azabicyclo[3.1.0]hexane;
   (1*R*,5*R*)-1-(benzo[*b*]thiophen-2-yl)-3-methyl-azabicyclo[3.1.0]hexane;
   (1*S*,5*S*)-1-(6-fluorobenzo[*b*]thiophen-2-yl)-3-aza-bicyclo[3.1.0]hexane; and
   (1*R*,5*R*)-1-(6-fluorobenzo[*b*]thiophen-2-yl)-3-aza-bicyclo[3.1.0]hexane;
   and pharmaceutically acceptable salts polymorphs, solvates, or hydrates thereof.
6. The compound according to clause 1 selected from the group consisting of:
   1-(5-methylfuran-2-yl)-3-azabicyclo[3.1.0]hexane;
   1-(6-methoxypyridin-3-yl)-3-azabicyclo[3.1.0]hexane;
   1-(6-methoxypyridin-3-yl)-3-methyl-3-azabicyclo[3.1.0]hexane;
   1-(benzofuran-2-yl)-3-methyl-3-azabicyclo[3.1.0]hexane;
   1-(benzofuran-3-yl)-3-azabicyclo[3.1.0]hexane;
   1-(benzofuran-3-yl)-3-methyl-3-azabicyclo[3.1.0]hexane;
   1-methyl-2-(3-methyl-3-azabicyclo[3.1.0]hexan-1-yl)-1*H*-indole;
   2-(3-ethyl-3-azabicyclo[3.1.0]hexan-1-yl)-1-methyl-1*H*-indole;
   2-(3-isopropyl-3-azabicyclo[3.1.0]hexan-1-yl)-1-methyl-1*H*-indole;
   1-methyl-5-(3-methyl-3-azabicyclo[3.1.0]hexan-1-yl)-1*H*-indole;
   5-(3-ethyl-3-azabicyclo[3.1.0]hexan-1-yl)-1-methyl-1*H*-indole;
   5-(3-isopropyl-3-azabicyclo[3.1.0]hexan-1-yl)-1-methyl-1*H*-indole;
   1-(5-chlorobenzo[*b*]thiophen-3-yl)-3-azabicyclo[3.1.0]hexane;
   1-(5-chlorobenzo[*b*]thiophen-2-yl)-3-methyl-3-azabicyclo[3.1.0]hexane;
   1-(5-chlorobenzo[*b*]thiophen-3-yl)-3-methyl-3-azabicyclo[3.1.0]hexane;
   1-(benzo[*b*]thiophen-2-yl)-3-aza-bicyclo[3.1.0]hexane;
   1-(benzo[*b*]thiophen-2-yl)-3-methyl-3-aza-bicyclo[3.1.0]hexane;
   1-(6-fluorobenzo[*b*]thiophen-2-yl)-3-aza-bicyclo[3.1.0]hexane;
   1-(6-fluorobenzo[*b*]thiophen-2-yl)-3-methyl-3-aza-bicyclo[3.1.0]hexane;
   1-(5-fluorobenzo[*b*]thiophen-2-yl)-3-aza-bicyclo[3.1.0]hexane;
   1-(5-fluorobenzo[*b*]thiophen-2-yl)-3-methyl-3-aza-bicyclo[3.1.0]hexane;
   N,N-dimethyl-2-(3-methyl-3-azabicyclo[3.1.0]hexane-1-yl)benzo[*b*]thiophen-5-amine;
   1-(5-bromobenzo[*b*]thiophen-2-yl)-3-azabicyclo[3.1.0]hexane;
   1-(5-bromobenzo[*b*]thiophen-2-yl)-3-methyl-3-azabicyclo[3.1.0]hexane;
   2-(3-azabicyclo[3.1.0]hexan-1-yl)-*N,N*-dimethylbenzo[*b*]thiophen-5-amine;
   2-(3-ethyl-3-azabicyclo[3.1.0]hexan-1-yl)-*N,N*-dimethylbenzo[*b*]thiophen-5-amine;
   2-(3-isopropyl-3-azabicyclo[3.1.0]hexan-1-yl)-*N,N*-dimethylbenzo[*b*]thiophen-5-amine;
   1-(6-bromobenzo[*b*]thiophen-2-yl)-3-azabicylco[3.1.0]hexane;
   1-(6-bromobenzo[*b*]thiophen-2-yl)-3-methyl-3-azabicyclo[3.1.0]hexane;
   2-(3-azabicyclo[3.1.0]hexan-1-yl)-*N,N*-dimethylbenzo[*b*]thiophen-6-amine;
   *N,N*-dimethyl-2-(3-methyl-3-azabicyclo[3.1.0]hexan-1-yl)benzo[*b*]thiophen-6-amine;
   2-(3-ethyl-3-azabicyclo[3.1.0]hexan-1-yl)-*N,N*-dimethylbenzo[*b*]thiophen-6-amine;
   2-(3-isopropyl-3-azabicyclo[3.1.0]hexan-1-yl)-*N,N*-dimethylbenzo[*b*]thiophen-6-amine; and
   2-(3-azabicyclo[3.1.0]hexan-1-yl)-benzo[*d*]thiazole;
   or a pharmaceutically acceptable salt thereof.
7. The compound according to clause 6 selected from the group consisting of:
   (1*S*,5*S*)-1-(benzo[*b*]thiophen-2-yl)-3-azabicyclo[3.1.0]hexane;
   (1*R*,5*R*)-1-(benzo[*b*]thiophen-2-yl)-3-azabicyclo[3.1.0]hexane;
   (1*S*,5*S*)-1-(benzo[*b*]thiophen-2-yl)-3-methyl-3-azabicyclo[3.1.0]hexane;
   (1*R*,5*R*)-1-(benzo[*b*]thiophen-2-yl)-3-methyl-azabicyclo[3.1.0]hexane;
   (1*S*,5*S*)-1-(6-fluorobenzo[*b*]thiophen-2-yl)-3-aza-bicyclo[3.1.0]hexane; and
   (1*R*,5*R*)-1-(6-fluorobenzo[*b*]thiophen-2-yl)-3-aza-bicyclo[3.1.0]hexane;
   or a pharmaceutically acceptable salt thereof.
8. The compound according to clause 6, wherein the compound is 1-(benzo[*b*]thiophen-2-yl)-3-methyl-3-aza-bicyclo[3.1.0]hexane or a pharmaceutically acceptable salt thereof.
9. The compound according to clause 6, wherein the compound is 1-(6-fluorobenzo[*b*]thiophen-2-yl)-3-aza-bicyclo[3.1.0]hexane or a pharmaceutically acceptable salt thereof.
10. The compound according to clause 6, wherein the compound is 1-(6-fluorobenzo[*b*]thiophen-2-yl)-3-aza-bicyclo[3.1.0]hexane hydrochloride or 1-(benzo[*b*]thiophen-2-yl)-3-methyl-3-aza-bicyclo[3.1.0]hexane hydrochloride.
11. The compound according to clause 6, wherein the compound is 1-(benzo[*b*]thiophen-2-yl)-3-aza-bicyclo[3.1.0]hexane or a pharmaceutically acceptable salt thereof.
12. The compound according to clause 11, wherein the compound is 1-(benzo[*b*]thiophen-2-yl)-3-aza-bicyclo[3.1.0]hexane hydrochloride.
13. The compound according to clause 11, wherein the compound is (1*S*,5*S*)-(1-benzo[*b*]thiophen-2-yl)-3-azabicyclo[3.1.0]hexane or a pharmaceutically acceptable salt thereof.
14. The compound according to clause 13, wherein the compound is (1*S*,5*S*)-(1-benzo[*b*]thiophen-2-yl)-3-azabicyclo[3.1.0]hexane hydrochloride.
15. A pharmaceutical composition comprising a compound according to clauses 1-14 or a pharmaceutically acceptable salt, enantiomer, polymorph, solvate, or hydrate thereof, and a pharmaceutically acceptable carrier or vehicle therefore.
16. The pharmaceutical composition according to clause 15 comprising a compound selected from the group consisting of:
   1-(6-fluorobenzo[*b*]thiophen-2-yl)-3-aza-bicyclo[3.1.0]hexane;
   1-(benzo[*b*]thiophen-2-yl)-3-aza-bicyclo[3.1.0]hexane; and
   1-(benzo[*b*]thiophen-2-yl)-3-methyl-3-aza-bicyclo[3.1.0]hexane;or a pharmaceutically acceptable salt thereof.
17. The pharmaceutical composition according to clause 16 comprising (1*S*,5*S*)-(1-benzo[b]thiophen-2-yl)-3-azabicyclo[3.1.0]hexane or a pharmaceutically acceptable salt thereof.
18. A compound according to any one of clauses 1-14 or a pharmaceutically acceptable salt, enantiomer, polymorph, solvate, or hydrate thereof, or a composition according to any one of clauses 15, 16, or 17, for use in therapy.
19. A compound according to any one of clauses 1-14 or a pharmaceutically acceptable salt, enantiomer, polymorph, solvate, or hydrate thereof, or a composition according to any one of clauses 15, 16, or 17, for use in treating or preventing a central nervous system (CNS) disorder; optionally, wherein the CNS disorder is: depression, an anxiety disorder, an attention defecit disorder, obesity, substance abuse, PMDD (Premenstrual dysphoric disorder) or a cognitive disorder.
20. The compound for use according to clause 19, wherein the compound is selected from the group consisting of:
   1-(6-fluorobenzo[*b*]thiophen-2-yl)-3-aza-bicyclo[3.1.0]hexane;
   1-(benzo[*b*]thiophen-2-yl)-3-aza-bicyclo[3.1.0]hexane; and
   1-(benzo[*b*]thiophen-2-yl)-3-methyl-3-aza-bicyclo[3.1.0]hexane;
   or a pharmaceutically acceptable salt thereof.
21. The compound for use according to clause 20, wherein the compound is (1*S*,5*S*)-(1-benzo[*b*]thiophen-2-yl)-3-azabicyclo[3.1.0]hexane or a pharmaceutically acceptable salt thereof.
22. A method of making a 1-heteroaryl-3-azabicyclo[3.1.0]hexane of the following Formula III, 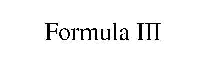 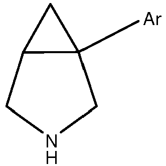 or a pharmaceutically acceptable salt thereof, wherein Ar is a heterocyclic aryl group selected from furan, methylfuran, pyrrole, imidazole, pyrazole, triazole, tetrazole, thiazole, isothiazole, methoxypyridine, pyridazine, pyrazine, triazine, indole, methylindole, benzofuran, benzothiophene, benzothiazole, isoquinoline, cinnoline, phthalazine, quinazoline, chromane and isochromane, and Ar is either unsubstituted or substituted with one or more substituents independently selected from fluoro, chloro, bromo, iodo, -NO₂, -CN, -NH, carboxy, C₂₋₈ alkenyl, C₂₋₈ alkynyl, halo(C₁₋₈)alkyl, hydroxy, trifluoromethyl, C₃₋₈ cycloalkyl, C₁₋₃ alkoxy, C₁₋₃ alkoxy(C₁₋₃)alkyl, carboxy(C₁₋₃)alkyl, C₁₋₃ alkanoyl, halo(C₁₋₃)alkoxyl, C₁₋₈ alkylamino, and di(C₁₋₈)alkylamino, comprising the steps of:
   (a) reacting a compound of the following formula (i), 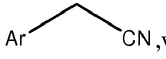 wherein Ar is defined as above, with epichlohydrin or an enantiomer thereof, to produce a compound of the following formula (ii), 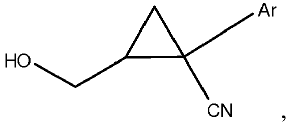 or an enantiomer or diastereomer thereof, wherein Ar is as defined above;
   (b) reducing the compound of formula (ii) to produce a compound of the following formula (iii), 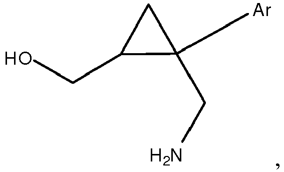 or an enantiomer or diastereomer thereof, wherein Ar is as defined above; and
   (c) causing cyclization of the compound of formula (iii) to produce the 1-heteroaryl-3-azabicyclo[3.1.0]hexane, or an enantiomer or diastereomer thereof.
23. The method according to clause 22 further comprising:
   (d) alkylating the 1-heteroaryl-3-azabicyclo[3.1.0]hexane produced in step (c) to produce a compound of the following Formula I, 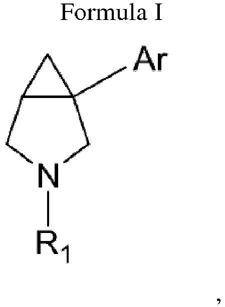 or a pharmaceutically acceptable salt thereof, wherein Ar is as defined in clause 22 and R₁ is selected from hydrogen, unsubstituted C₁₋₁₀ alkyl, C₃₋₈ cycloalkyl, C₂₋₁₀ alkenyl, and C₃₋₁₀ alkynyl, and substituted C₁₋₁₀ alkyl, C₃₋₁₀ alkenyl and C₃₋₁₀ alkynyl wherein the substituent is one or more of hydroxy, cyano, halogen, C₁₋₆ alkoxy, aryl substituted C₁₋₆ alkoxy, aryloxy, aryloxy substituted with one or more halogens, C₁₋₆ alkyl, C₁₋₆ alkyl independently substituted with one or more of cyano and halogen, C₁₋₄ alkoxy, and C₁₋₄ haloalkoxy.
24. A method of making a 1-heteroaryl-3-azabicyclo[3.1.0]hexane of the following Formula I, 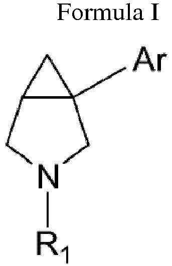 or a pharmaceutically acceptable salt thereof, wherein Ar is a heterocyclic aryl group selected from furan, methylfuran, pyrrole, imidazole, pyrazole, triazole, tetrazole, thiazole, isothiazole, methoxypyridine, pyridazine, pyrazine, triazine, indole, methylindole, benzofuran, benzothiophene, benzothiazole, isoquinoline, cinnoline, phthalazine, quinazoline, chromane and isochromane, and Ar is either unsubstituted or substituted with one or more substituents independently selected from fluoro, chloro, bromo, iodo, -NO₂, -CN, -NH₂, carboxy, C₂₋₈ alkenyl, C₂₋₈ alkynyl, halo(C₁₋₈)alkyl, hydroxy, trifluoromethyl, C₃₋₈ cycloalkyl, C₁₋₃ alkoxy, C₁₋₃ alkoxy(C₁₋₃)alkyl, carboxy(C₁₋₃)alkyl, C₁₋₃ alkanoyl, halo(C₁₋₃)alkoxyl, C₁₋₈ alkylamino, and di(C₁₋₈)alkylamino and R₁ is selected from hydrogen, unsubstituted C₁₋₁₀ alkyl, C₃₋₈ cycloalkyl, C₂₋₁₀ alkenyl, and C₃₋₁₀ alkynyl, and substituted C₁₋₁₀ alkyl, C₃₋₁₀ alkenyl and C₃₋₁₀ alkynyl wherein the substituent is one or more of hydroxy, cyano, halogen, C₁₋₆ alkoxy, aryl substituted C₁₋₆ alkoxy, aryloxy, aryloxy substituted with one or more halogens, C₁₋₆ alkyl, C₁₋₆ alkyl independently substituted with one or more of cyano and halogen, C₁₋₄ alkoxy, and C₁₋₄ haloalkoxy, comprising the steps of:
   (a) reacting a compound of the following formula (iv), 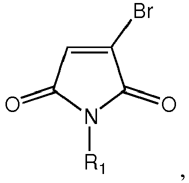 wherein R₁ is as defined above, with 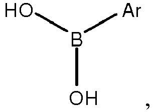 wherein Ar is as defined above, to produce a compound of the following formula (v), 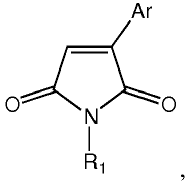 wherein R₁ and Ar are as defined above;
   (b) causing cyclopropanation of the compound of formula (v) to produce a compound of the following formula (vi), 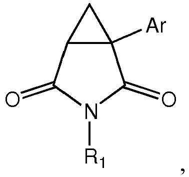 wherein R₁ and Ar are as defined above; and
   (c) reducing the compound of formula (vi) to produce the 1-heteroaryl-3-azabicyclo[3.1.0]hexane.
25. A method of making a 1-heteroaryl-3-azabicyclo[3.1.0]hexane of the following Formula III, 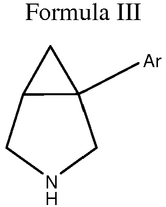 or a pharmaceutically acceptable salt thereof, wherein Ar is a heterocyclic aryl group selected from furan, methylfuran, pyrrole, imidazole, pyrazole, triazole, tetrazole, thiazole, isothiazole, methoxypyridine, pyridazine, pyrazine, triazine, indole, methylindole, benzofuran, benzothiophene, benzothiazole, isoquinoline, cinnoline, phthalazine, quinazoline, chromane and isochromane, and Ar is either unsubstituted or substituted with one or more substituents independently selected from fluoro, chloro, bromo, iodo, -NO₂, -CN, -NH, carboxy, C₂₋₈ alkenyl, C₂₋₈ alkynyl, halo(C₁₋₈)alkyl, hydroxy, trifluoromethyl, C₃₋₈ cycloalkyl, C₁₋₃ alkoxy, C₁₋₃ alkoxy(C₁₋₃)alkyl, carboxy(C₁₋₃)alkyl, C₁₋₃ alkanoyl, halo(C₁₋₃)alkoxyl, C₁₋₈ alkylamino, and di(C₁₋₈)alkylamino, comprising the steps of:
   (a) coupling a compound of the following formula (vii), 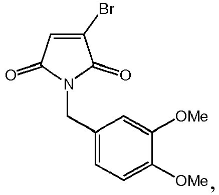 with 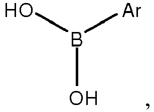 wherein Ar is as defined above, to produce a compound of the following formula (viii), 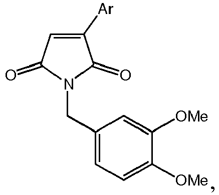 wherein Ar is as defined above;
   (b) causing cyclopropanation of the compound of formula (viii) to produce a compound of the following formula (ix), 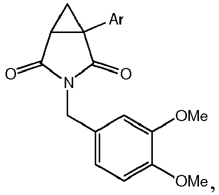 wherein Ar is as defined above;
   (c) reducing the compound of formula (ix) to produce a compound of the following formula (x), 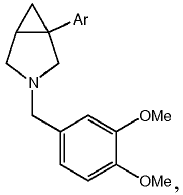 wherein Ar is as defined above; and
   (d) deprotecting the compound of formula (x) to produce the 1-heteroaryl-3-azabicyclo[3.1.0]hexane.
26. The method according to clause 25 further comprising:
   (e) alkylating the 1-heteroaryl-3-azabicyclo[3.1.0]hexane produced in step (d) to produce a compound of the following Formula I, 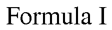 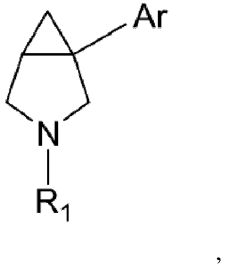
   or a pharmaceutically acceptable salt thereof, wherein Ar is as defined in clause 25 and R₁ is selected from hydrogen, unsubstituted C₁₋₁₀ alkyl, C₃₋₈ cycloalkyl, C₂₋₁₀ alkenyl, and C₃₋₁₀ alkynyl, and substituted C₁₋₁₀ alkyl, C₃₋₁₀ alkenyl and C₃₋₁₀ alkynyl wherein the substituent is one or more of hydroxy, cyano, halogen, C₁₋₆ alkoxy, aryl substituted C₁₋₆ alkoxy, aryloxy, aryloxy substituted with one or more halogens, C₁₋₆ alkyl, C₁₋₆ alkyl independently substituted with one or more of cyano and halogen, C₁₋₄ alkoxy, and C₁₋₄ haloalkoxy.

An illustrative assemblage of 1-heteroaryl-3-azabicyclo-[3.1.0]hexanes according to the present disclosure is provided in Table 1.

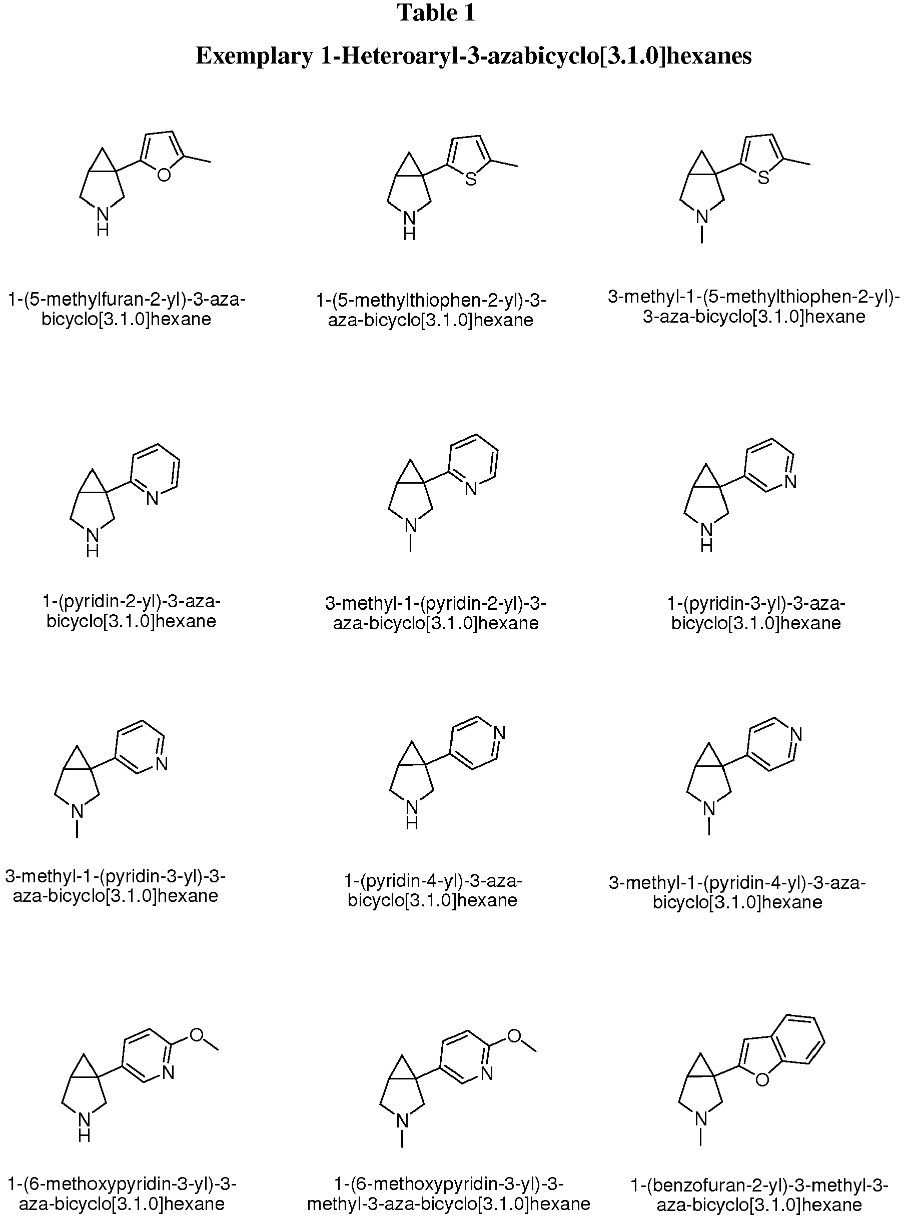

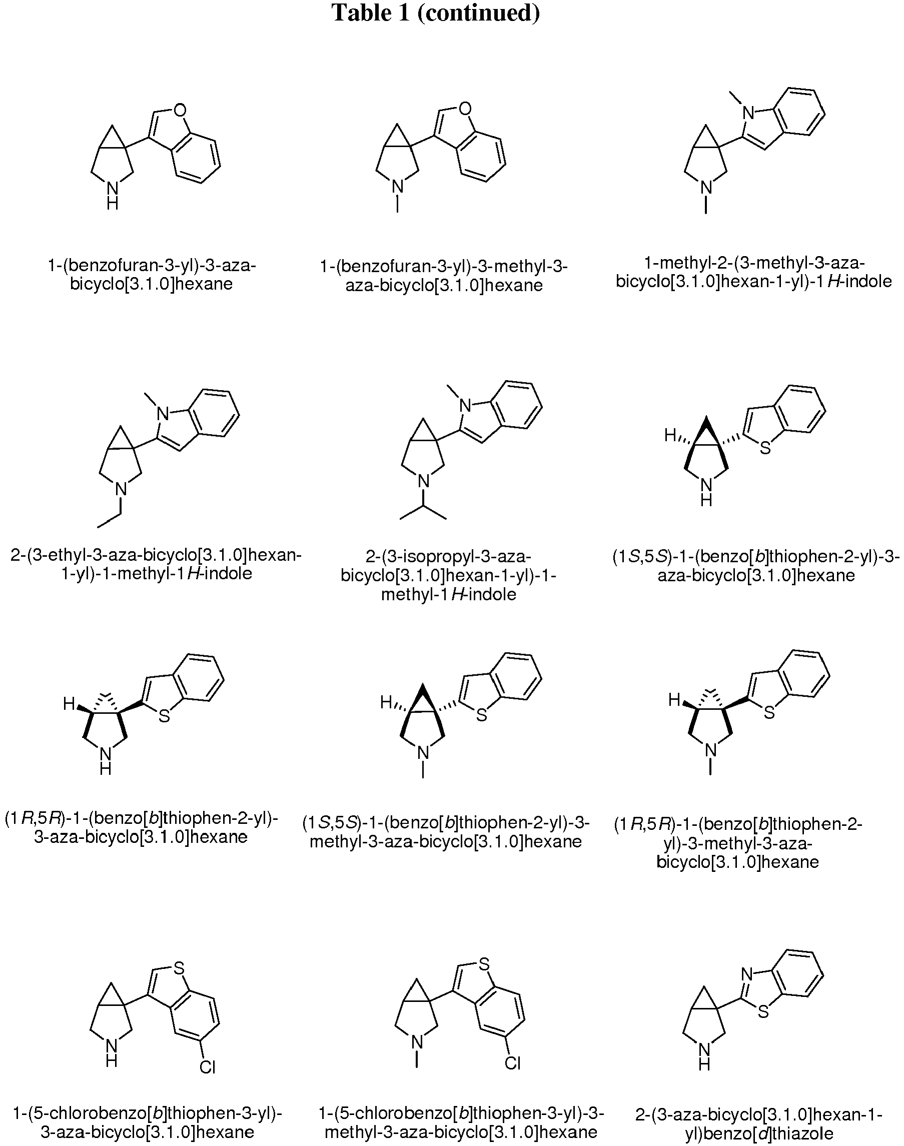

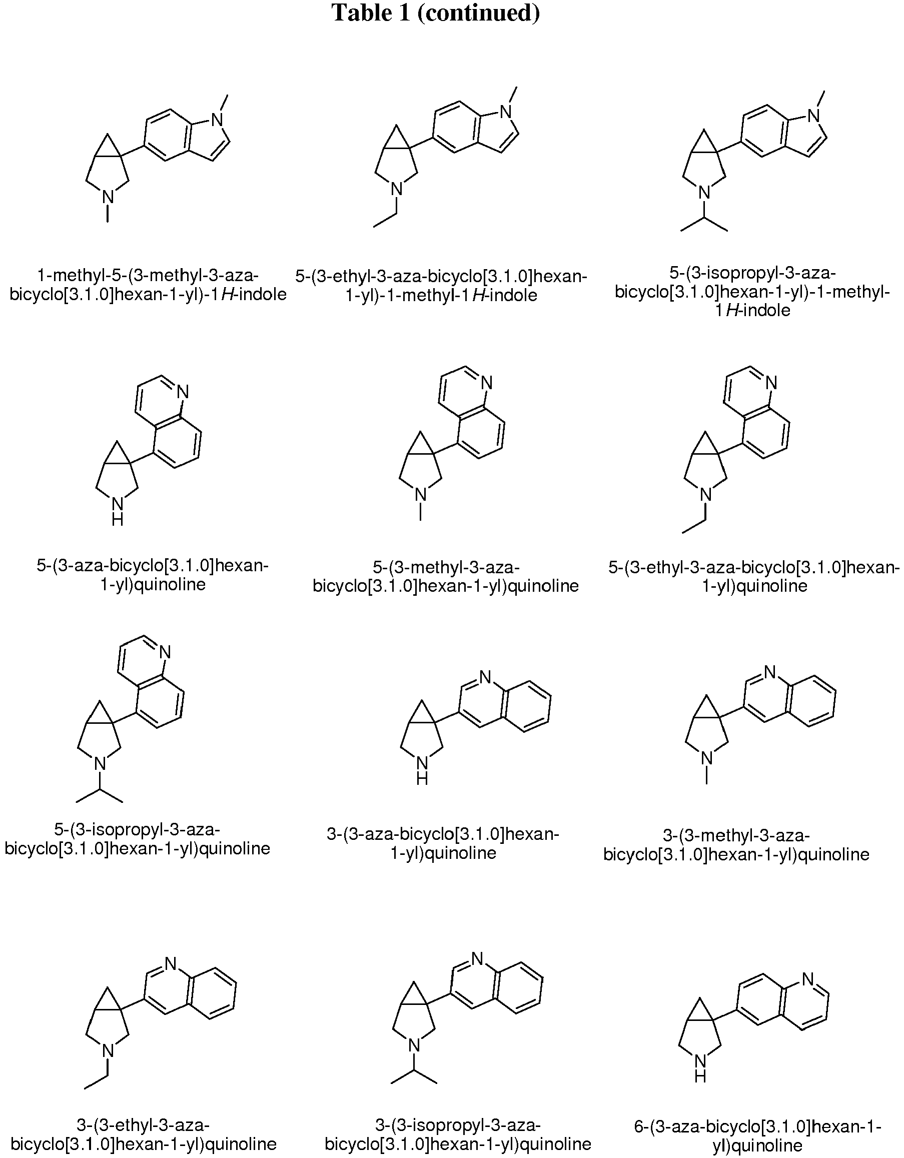

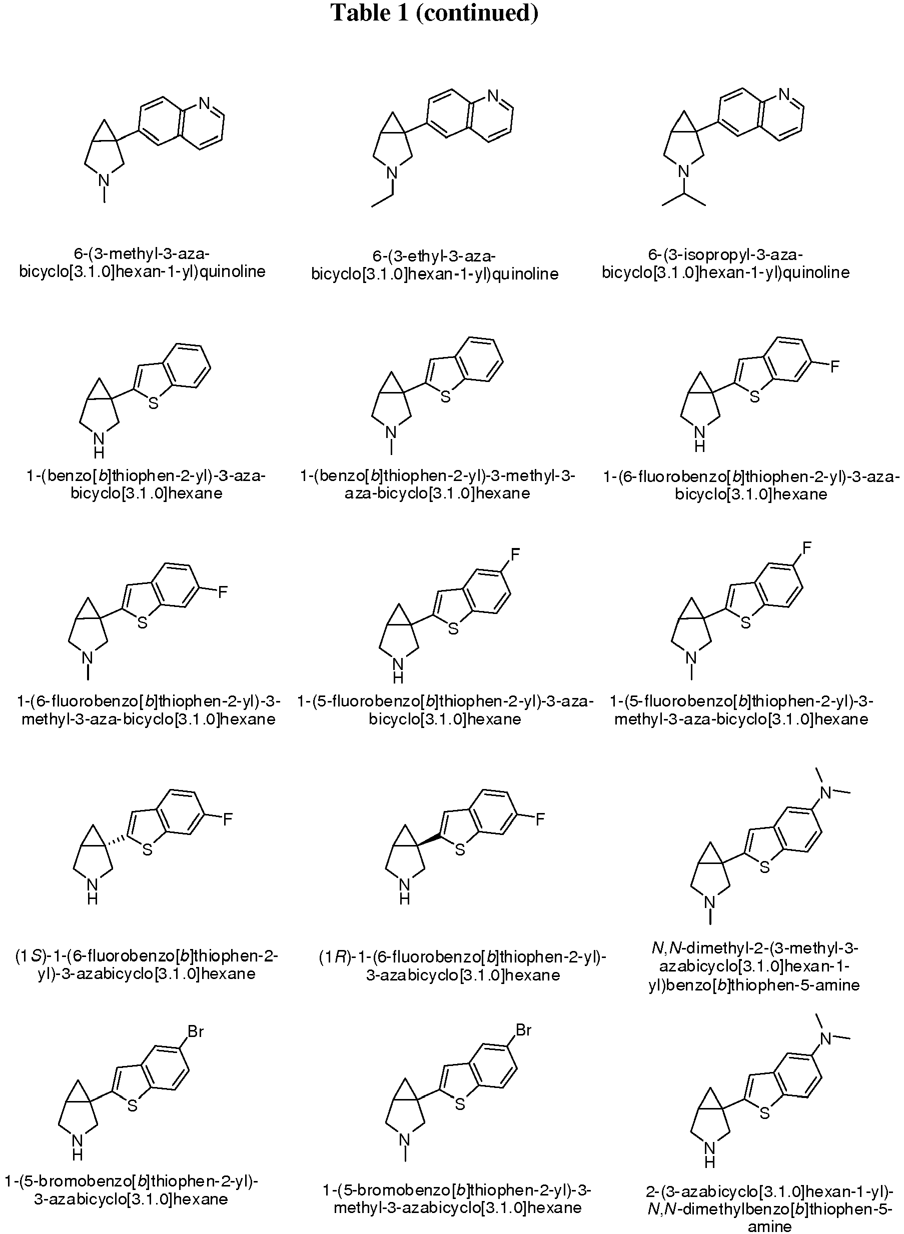

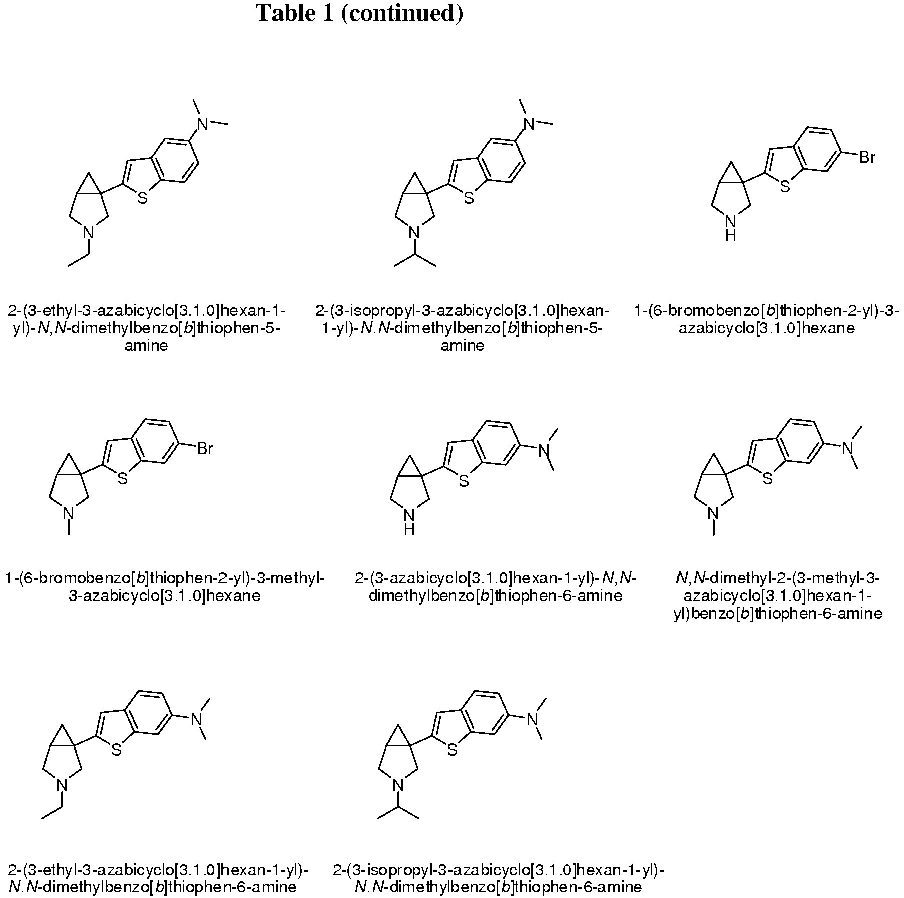

The 1-heteroaryl-3-azabicyclo[3.1.0]hexanes of the present disclosure are provided in any of a variety of forms, including pharmaceutically acceptable, active salts, solvates, hydrates, polymorphs, and/or prodrugs of the compounds disclosed herein, or any combination thereof.

It will be understood that the exemplary compounds identified in Table 1 are illustrative, and that in aspects of the present disclosure the heteroaryl ring can be varied to comprise other substituents, and/or can include yet additional substituents (i.e., three or more substitutions on the heteroaryl ring), combined with one another, or additionally combined with or without substitutions on the nitrogen atom as described herein, to yield yet additional compounds within the present disclosure for treating CNS disorders (including a range of neuropsychiatric disorders, such as depression and anxiety). For example, the present disclosure provides an illustrative assemblage of novel 1-heteroaryl-3-azabicyclo[3.1.0]hexanes amines having multiple substitutions on the aryl ring, combined with or without substitution(s) on the nitrogen atom. Additionally, useful 1-heteroaryl-3-azabicyclo[3.1.0]hexanes of the present disclosure include the substituted 1-heteroaryl-3-azabicyclo[3.1.0]hexanes compounds described herein, as well as their pharmaceutically acceptable salts, enantiomers, polymorphs, solvates, hydrate or prodrugs or combinations thereof.

Within related aspects of the disclosure, enantiomeric forms of the novel compounds described herein, having chiral symmetric structures, are provided, which provide yet additional drug candidates for treating CNS disorders. In certain aspects, the present disclosure provides enantiomers, diastereomers, and other stereoisomeric forms of the disclosed compounds, including racemic and resolved forms and mixtures thereof. The individual enantiomers may be separated according to methods that are well known to those of ordinary skill in the art. In other aspects of the present disclosure, the enantiomers, diastereomers and other stereoisomeric forms of the disclosed compounds contain no more than about 10%, about 5%, about 2% or about 1% of the corresponding enantiomers, diastereomers and stereoisomers. When the compounds described herein contain olefinic double bonds or other centers of geometric asymmetry, and unless specified otherwise, it is intended to include both E and Z geometric isomers. All tautomers are intended to be encompassed by the present disclosure as well.

As noted above, the compounds of the present disclosure can be can be prepared as both acid addition salts formed from an acid and the basic nitrogen group of 1-heteroaryl-3-azabicyclo[3.1.0.]hexanes and base salts. As further noted above, the methods of the present disclosure can be used to prepare compounds as both acid addition salts formed from an acid and the basic nitrogen group of 1-heteroaryl-3-azabicyclo[3.1.0.]hexanes and base salts. Suitable acid addition salts include, for example, hydrochloride, hydrobromide, hydroiodide, sulphate, hydrogen sulphate, nitrate, phosphate, and hydrogen phosphate salts. Other examples of pharmaceutically acceptable acid addition salts include inorganic and organic acid addition salts. Additional pharmaceutically acceptable salts include, but are not limited to, metal salts such as sodium salt, potassium salt, cesium salt and the like; alkaline earth metals such as calcium salt, magnesium salt and the like; organic amine salts such as triethylamine salt, pyridine salt, picoline salt, ethanolamine salt, triethanolamine salt, dicyclohexylamine salt, N,N'-dibenzylethylenediamine salt and the like; organic acid salts such as acetate, citrate, lactate, succinate, tartrate, maleate, fumarate, mandelate, acetate, dichloroacetate, trifluoroacetate, oxalate, formate and the like; sulfonates such as methanesulfonate, benzenesulfonate, p-toluenesulfonate and the like; and amino acid salts such as arginate, asparginate, glutamate, tartrate, gluconate and the like. Suitable base salts are formed from bases, which form nontoxic salts and include, for example, aluminum, calcium, lithium, magnesium, potassium, sodium, zinc and diethanolamine salts. The hydrochloride salt formed with hydrochloric acid is an exemplary useful salt.

In other detailed aspects, the present disclosure provides prodrugs of the disclosed compounds. Prodrugs are considered to be any covalently bonded carriers which release the active parent drug in vivo. Examples of prodrugs include esters or amides of a compound of the present disclosure with hydroxyalkyl or aminoalkyl as a substituent. These may be prepared by reacting such compounds with anhydrides such as succinic anhydride.

The present disclosure will also be understood to disclose in vivo metabolic products of the disclosed compounds. Such products may result for example from the oxidation, reduction, hydrolysis, amidation, esterification and the like of the administered compound, primarily due to enzymatic processes. Accordingly, the present disclosure includes compounds produced by a process comprising contacting a compound of this disclosure with a mammal for a period of time sufficient to yield a metabolic product thereof. Such products typically are identified by preparing a radiolabelled compound of the present disclosure, administering it parenterally in a detectable dose to an animal such as rat, mouse, guinea pig, monkey, or to man, allowing sufficient time for metabolism to occur and isolating its conversion products from the urine, blood or other biological samples.

The present disclosure will also be understood to encompass the disclosed compounds isotopically-labelled by having one or more atoms replaced by an atom having a different atomic mass or mass number. Examples of isotopes that can be incorporated into the disclosed compounds include isotopes of hydrogen, carbon, nitrogen, oxygen, phosphorous, fluorite and chloride, such as ²H, ³H, ¹³C, ¹⁴C, ¹⁵N, ¹⁸O, ¹⁷O, ³¹P, ³²P, ³⁵S, ¹⁸F, and ³⁶Cl, respectively.

The compounds of the present disclosure may be prepared using methods known to those skilled in the art, and in other aspects by employing novel synthetic schemes as provided herein, which, along with the exemplified intermediate compounds, also fall within the present disclosure. Accordingly, the present disclosure also provides novel methods and compositions for producing the compounds of the present disclosure as well as other 1-heteroaryl-3-azabicyclo[3.1.0] hexanes.

In practicing the methods of the present invention for making 1-heteroaryl-3-azabicyclo[3.1.0]hexanes, various reagents may be utilized for the different reaction steps. In general, suitable reagents for the various reaction steps may be selected by one of ordinary skill in the art based on the present disclosure.

Suitable reducing agents and methodologies include, for example, lithium aluminum hydride (LAH), sodium aluminum hydride (SAH), NaBH₄ with ZnCl₂ and catalytic hydrogenation.
Suitable nitrogen protecting groups include, for example, benzyl, allyl, tert-butyl and 3,4-dimethoxy-benzyl groups. In general, nitrogen protecting groups are well known to those skilled in the art, see for example, "Nitrogen Protecting Groups in Organic Synthesis", John Wiley and Sons, New York, N.Y., 1981, Chapter 7; "Nitrogen Protecting Groups in Organic Chemistry", Plenum Press, New York, N.Y., 1973, Chapter 2; T. W. Green and P. G. M. Wuts in "Protective Groups in Organic Chemistry", 3rd edition, John Wiley & Sons, New York, N.Y., 1999.

When the nitrogen protecting group is no longer needed, it may be removed by methods well known in the art. For example, benzyl or 3,4-dimethoxy-benzyl groups may be removed by catalytic hydrogenation. In general, methods of removing nitrogen protecting groups are well known to those skilled in the art, see for example, "Nitrogen Protecting Groups in Organic Synthesis", John Wiley and Sons, New York, N.Y., 1981, Chapter 7; "Nitrogen Protecting Groups in Organic Chemistry", Plenum Press, New York, N.Y., 1973, Chapter 2; T. W. Green and P. G. M. Wuts in "Protective Groups in Organic Chemistry", 3rd edition, John Wiley & Sons, Inc. New York, N.Y., 1999.

Suitable reagents for causing cyclization include, for example, SOCl₂, POCl₃, oxalyl chloride, phosphorous tribromide, triphenylphosphorous dibromide and oxalyl bromide.

Exemplary synthetic methods, starting materials, and intermediates useful in various aspects of the present disclosure for producing compounds of the present disclosure are described below and in the examples.

Reaction Scheme 1 below generally sets forth an exemplary process for preparing 1-heteroaryl-3-azabicyclo[3.1.0]hexanes.

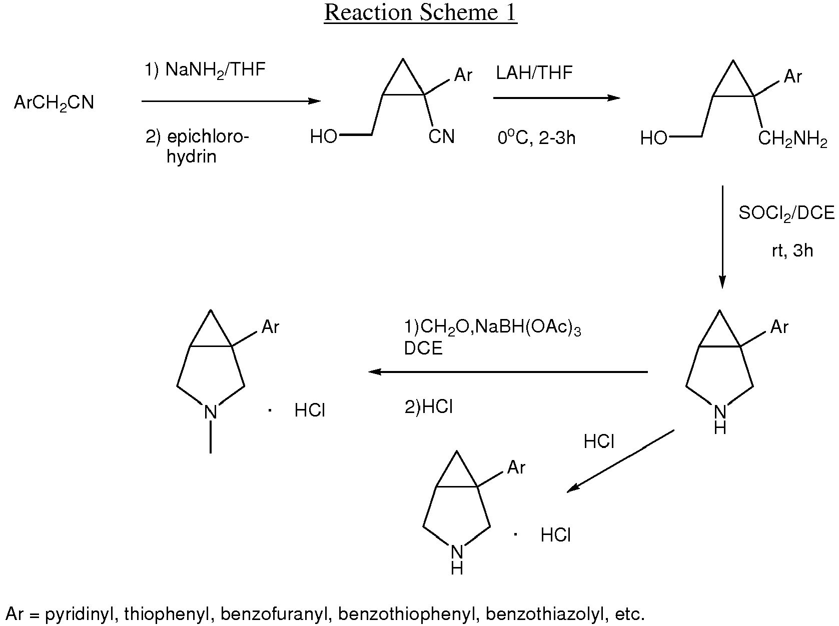

Reaction Scheme 2 below generally sets forth another exemplary process for preparing 1-heteroaryl-3-azabicyclo[3.1.0]hexanes.

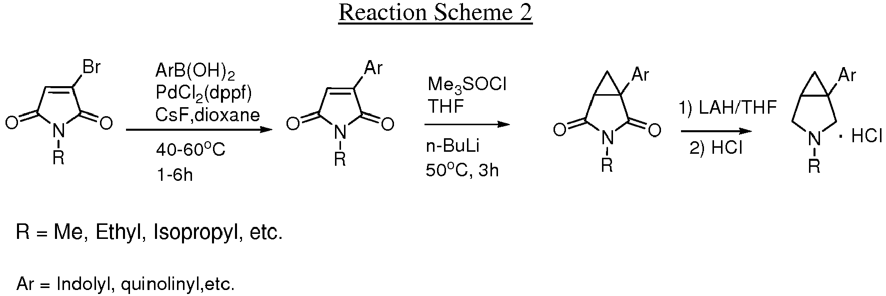

Reaction Scheme 3 below generally sets forth an additional exemplary process for preparing 1-heteroaryl-3-azabicyclo[3.1.0]hexanes.

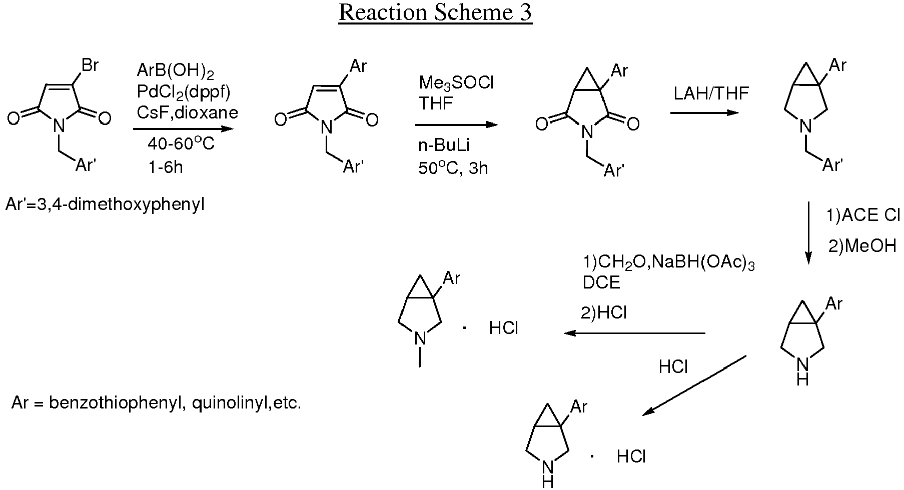

Reaction Scheme 4 below generally sets forth an additional exemplary process for preparing chiral 1-heteroaryl-3-azabicyclo[3.1.0]hexanes.

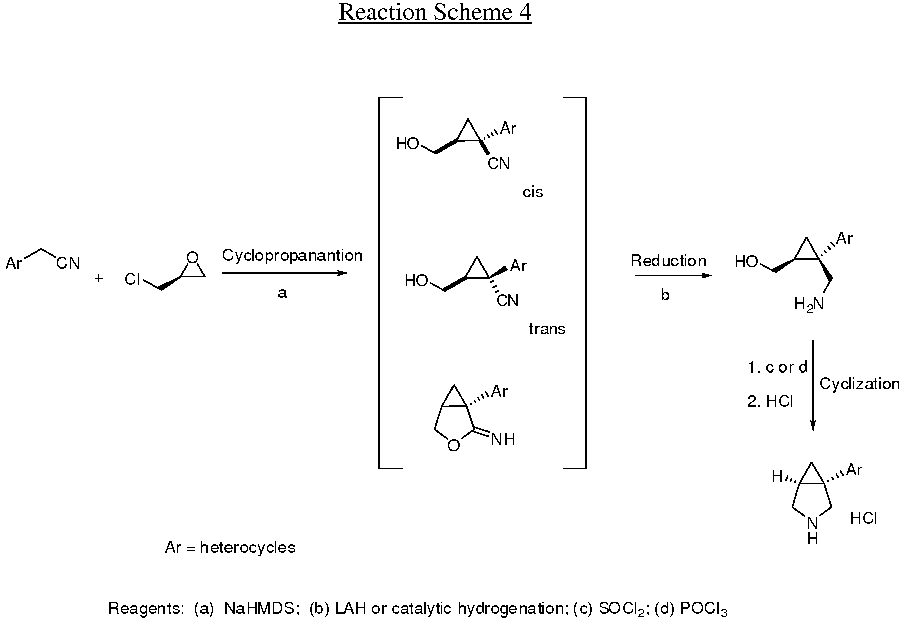

Reaction Scheme 5 below generally sets forth an additional exemplary process for preparing chiral 1-heteroaryl-3-azabicyclo[3.1.0]hexanes.

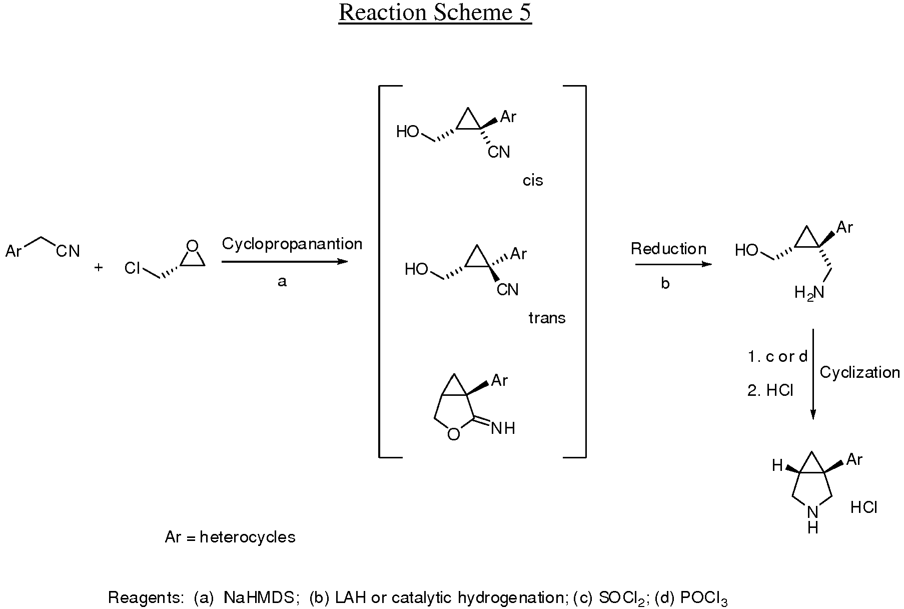

For the purposes of further describing the invention, including the novel compounds and synthetic methods disclosed herein, the following terms and definitions are provided by way of example.

The term "halogen" as used herein refers to bromine, chlorine, fluorine or iodine. In one embodiment, the halogen is chlorine. In another embodiment, the halogen is bromine.

The term "hydroxy" as used herein refers to -OH or --O⁻.

The term "alkyl" as used herein refers to straight- or branched-chain aliphatic groups containing 1-20 carbon atoms, preferably 1-7 carbon atoms and most preferably 1-4 carbon atoms. This definition applies as well to the alkyl portion of alkoxy, alkanoyl and aralkyl groups. In one embodiment, the alkyl is a methyl group.

The term "alkoxy" includes substituted and unsubstituted alkyl, alkenyl, and alkynyl groups covalently linked to an oxygen atom. In one embodiment, the alkoxy group contains 1 to 4 carbon atoms. Embodiments of alkoxy groups include, but are not limited to, methoxy, ethoxy, isopropyloxy, propoxy, butoxy, and pentoxy groups. Embodiments of substituted alkoxy groups include halogenated alkoxy groups. In a further embodiment, the alkoxy groups can be substituted with groups such as alkenyl, alkynyl, halogen, hydroxyl, alkylcarbonyloxy, phenylcarbonyloxy, alkoxycarbonyloxy, phenyloxycarbonyloxy, carboxylate, alkylcarbonyl, phenylcarbonyl, alkoxycarbonyl, aminocarbonyl, alkylaminocarbonyl, dialkylaminocarbonyl, alkylthiocarbonyl, alkoxyl, phosphate, phosphonato, phosphinato, cyano, amino (including alkylamino, dialkylamino, phenylamino, diphenylamino, and alkylphenylamino), acylamino (including alkylcarbonylamino, phenylcarbonylamino, carbamoyl and ureido), amidino, imino, sulfhydryl, alkylthio, phenylthio, thiocarboxylate, sulfates, alkylsulfinyl, sulfonato, sulfamoyl, sulfonamido, nitro, trifluoromethyl, cyano, azido, heterocyclyl, alkylphenyl, or aromatic or heteroaromatic moieties. Exemplary halogen substituted alkoxy groups include, but are not limited to, fluoromethoxy, difluoromethoxy, trifluoromethoxy, chloromethoxy, dichloromethoxy, and trichloromethoxy.

The term "aryl" as used herein refers to monocyclic or bicyclic aromatic hydrocarbon groups having from 6 to 12 carbon atoms in the ring portion, for example, phenyl, naphthyl, biphenyl and diphenyl groups, each of which may be substituted with, for example, one to four substituents such as alkyl, substituted alkyl as defined above, halogen, trifluoromethyl, trifluoromethoxy, hydroxy, alkoxy, cycloalkyloxy, alkanoyl, alkanoyloxy, amino, alkylamino, dialkylamino, nitro, cyano, carboxy, carboxyalkyl, carbamyl, carbamoyl and aryloxy. Specific embodiments of aryl groups in accordance with the present disclosure include phenyl, substituted phenyl, naphthyl, biphenyl, and diphenyl.

The term "nitro", as used herein alone or in combination refers to a --NO₂ group.

The term "amino" as used herein refers to the group --NRR', where R and R' may independently be hydrogen, alkyl, phenyl, alkoxy, or heterophenyl. The term "aminoalkyl" as used herein represents a more detailed selection as compared to "amino" and refers to the group --NRR', where R and R' may independently be hydrogen or (C₁-C₄)alkyl.

The term "trifluoromethyl" as used herein refers to --CF_{3.}

The term "trifluoromethoxy" as used herein refers to --OCF₃.

The term "cycloalkyl" as used herein refers to a saturated cyclic hydrocarbon ring system containing from 3 to 7 carbon atoms that may be optionally substituted. Exemplary embodiments include, but are not limited to, cyclopropyl, cyclobutyl, cyclopentyl and cyclohexyl. In certain embodiments, the cycloalkyl group is cyclopropyl.

In another embodiment, the (cycloalkyl)alkyl groups contain from 3 to 7 carbon atoms in the cyclic portion and 1 to 4 carbon atoms in the alkyl portion. In certain embodiments, the (cycloalkyl)alkyl group is cyclopropylmethyl. The alkyl groups are optionally substituted with from one to three substituents selected from the group consisting of halogen, hydroxy and amino.

The terms "alkanoyl" and "alkanoyloxy" as used herein refer, respectively, to-C(O)-alkyl groups and -O-C(O)-alkyl groups, each optionally containing 2-5 carbon atoms. Specific embodiments of alkanoyl and alkanoyloxy groups are acetyl and acetoxy, respectively.

The term "aroyl," as used alone or in combination herein, refers to a phenyl radical derived from an aromatic carboxylic acid, such as optionally substituted benzoic or naphthoic acids.

The term "aralkyl" as used herein refers to a phenyl group bonded to an alkyl group, preferably one containing 1-4 carbon atoms. A preferred aralkyl group is benzyl.

The term "nitrile" or "cyano" as used herein refers to the group -CN.

The term "pyrrolidine-1-yl" as used herein refers to the structure:

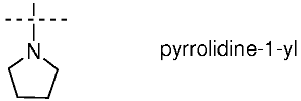

The term "morpholino" as used herein refers to the structure:

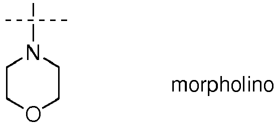

The term "dialkylamino" refers to an amino group having two attached alkyl groups that can be the same or different.

The term "alkenyl" refers to a straight or branched alkenyl group of 2 to 10 carbon atoms having 1 to 3 double bonds. Preferred embodiments include ethenyl, 1-propenyl, 2-propenyl, 1-methylethenyl, 1-butenyl, 2-butenyl, 3-butenyl, 2-methyl-2-propenyl, 1-pentenyl, 2-pentenyl, 4-pentenyl, 3-methyl-2-butenyl, 1-hexenyl, 2-hexenyl, 1-heptenyl, 2-heptenyl, 1-octenyl, 2-octenyl, 1,3-octadienyl, 2-nonenyl, 1,3-nonadienyl, 2-decenyl, etc.

The term "alkynyl" as used herein refers to a straight or branched alkynyl group of 2 to 10 carbon atoms having 1 to 3 triple bonds. Exemplary alkynyls include, but are not limited to, ethynyl, 1-propynyl, 2-propynyl, 1-butynyl, 2-butynyl, 3-butynyl, 1-pentynyl, 2-pentynyl, 4-pentynyl, 1-octynyl, 6-methyl-1-heptynyl, and 2-decynyl.

The term "hydroxyalkyl" alone or in combination, refers to an alkyl group as previously defined, wherein one or several hydrogen atoms, preferably one hydrogen atom has been replaced by a hydroxyl group. Examples include hydroxymethyl, hydroxyethyl and 2-hydroxyethyl.

The term "aminoalkyl" and "alkylamino" as used herein refers to the group-NRR', where R and R' may independently be hydrogen or (C₁-C₄)alkyl.
The term "alkylaminoalkyl" refers to an alkylamino group linked via an alkyl group (i.e., a group having the general structure --alkyl-NH-alkyl or --alkyl-N(alkyl)(alkyl)). Such groups include, but are not limited to, mono- and di-(C₁-C₈ alkyl)aminoC₁-C₈ alkyl, in which each alkyl may be the same or different.

The term "dialkylaminoalkyl" refers to alkylamino groups attached to an alkyl group. Examples include, but are not limited to, N,N-dimethylaminomethyl, N,N-dimethylaminoethyl, N,N-dimethylaminopropyl, and the like. The term dialkylaminoalkyl also includes groups where the bridging alkyl moiety is optionally substituted.

The term "haloalkyl" refers to an alkyl group substituted with one or more halo groups, for example chloromethyl, 2-bromoethyl, 3-iodopropyl, trifluoromethyl, perfluoropropyl, 8-chlorononyl and the like.

The term "carboxyalkyl" as used herein refers to the substituent --R'--COOH wherein R' is alkylene; and carbalkoxyalkyl refers to --R'--COOR wherein R' and R are alkylene and alkyl respectively. In certain embodiments, alkyl refers to a saturated straight- or branched-chain hydrocarbyl radical of 1-6 carbon atoms such as methyl, ethyl, n-propyl, isopropyl, n-butyl, t-butyl, n-pentyl, 2-methylpentyl, n-hexyl, and so forth. Alkylene is the same as alkyl except that the group is divalent.

The term "alkoxyalkyl" refers to an alkylene group substituted with an alkoxy group. For example, methoxyethyl [CH₃OCH₂CH₂--] and ethoxymethyl (CH₃CH₂OCH₂--] are both C₃ alkoxyalkyl groups.

The term "carboxy", as used herein, represents a group of the formula-COOH.

The term "alkanoylamino" refers to alkyl, alkenyl or alkynyl groups containing the group --C(O)-- followed by --N(H)--, for example acetylamino, propanoylamino and butanoylamino and the like.

The term "carbonylamino" refers to the group --NR--CO--CH₂--R', where R and R' may be independently selected from hydrogen or (C₁-C₄)alkyl.

The term "carbamoyl" as used herein refers to --O--C(O)NH₂.

The term "carbamyl" as used herein refers to a functional group in which a nitrogen atom is directly bonded to a carbonyl, i.e., as in --NRC(=O)R' or-C(=O)NRR', wherein R and R' can be hydrogen, alkyl, substituted alkyl, alkenyl, substituted alkenyl, alkoxy, cycloalkyl, phenyl, heterocyclo, or heterophenyl.

The terms "heterocyclo" or "heterocyclic" refers to an optionally substituted, unsaturated, partially saturated, or fully saturated, aromatic or nonaromatic cyclic group that is a 4 to 7 membered monocyclic, or 7 to 11 membered bicyclic ring system that has at least one heteroatom in at least one carbon atom-containing ring. The substituents on the heterocyclo rings may be selected from those given above for the aryl groups. Each ring of the heterocyclo group containing a heteroatom may have 1, 2 or 3 heteroatoms selected from nitrogen atoms, oxygen atoms and sulfur atoms. Plural heteroatoms in a given heterocyclo ring may be the same or different.

The term "heteroaryl" refers to an optionally substituted monocyclic or bicyclic heterocyclic aryl group (ie., an aromatic heterocyclic group) that is a 4 to 7 membered monocyclic, or 7 to 11 membered bicyclic ring system that has at least one heteroatom in at least one carbon atom-containing ring. Each ring of the heteroaryl group containing a heteroatom may have 1,2 or 3 heteroatoms selected from nitrogen atoms, oxygen atoms and sulfur atoms. Plural heteroatoms in a given heteroaryl group may be the same or different. Specific embodiments of heteroaryl groups in accordance with the present disclosure include furan, thiophene, pyrrole, imidazole, pyrazole, triazole, tetrazole, thiazole, isothiazole, pyridine, pyridizine, pyrimidine, pyrazine, triazine, indole, benzofuran, benzothiophene, benzothiazole, quinoline, isoquinoline, cinnoline, phthalazine, quinazoline, quinoxaline, chromane and isochromane groups.

The term "alkylsulfonylamino" refers to refers to the group --NHS(O)₂Rₐ wherein Rₐ is an alkyl as defined above.

As used herein, the term "stereoisomers" is a general term for all isomers of individual molecules that differ only in the orientation of their atoms in space. It includes enantiomers and isomers of compounds with more than one chiral center that are not mirror images of one another (diastereomers).

The term "chiral center" refers to a carbon atom to which four different groups are attached.

The term "enantiomer" or "enantiomeric" refers to a molecule that is nonsuperimposeable on its mirror image and hence optically active wherein the enantiomer rotates the plane of polarized light in one direction and its mirror image rotates the plane of polarized light in the opposite direction.

The term "racemic" refers to a mixture of equal parts of enantiomers and which is optically inactive.

The term "resolution" refers to the separation or concentration or depletion of one of the two enantiomeric forms of a molecule.

The compositions and methods of the present disclosure comprising a 1-heteroaryl-3-azabicyclo[3.1.0] hexane are effective for treating or preventing a variety of central nervous system (CNS) disorders in mammals. In certain aspects of the present disclosure, pharmaceutical compositions and methods are provided for treating a CNS disorder in a mammalian subject. Mammalian subjects amenable for treatment using these compositions and methods include, but are not limited to, human and other mammalian subjects suffering from a CNS disorder that responds positively to intervention by inhibition of biogenic amine transport. In related aspects of the present disclosure, therapeutic compositions and methods are provided which employ an effective amount of one or more 1-heteroaryl-3-azabicyclo[3.1.0] hexane(s) described herein to treat or prevent a selected CNS disorder in a subject, wherein administration of the composition to the subject effectively inhibits the function of one or more, and in certain aspects of the present disclosure all three, norepinephrine, serotonin, and/or dopamine transport proteins in the subject, thereby preventing, or reducing the occurrence or severity of symptoms of, the targeted CNS disorder. Suitable forms of the compounds of the present disclosure for use in biologically active compositions and methods of the present disclosure include the compounds exemplified herein, as well as their pharmaceutically acceptable salts, polymorphs, solvates, hydrates and prodrugs.

In related aspects of the present disclosure, a biogenic amine transport inhibitory-effective amount of a 1-heteroaryl-3-azabicyclo[3.1.0.]hexane of the present disclosure is administered to treat or prevent a CNS disorder, including neurological or psychiatric conditions, in a mammalian subject responsive to inhibition of biogenic amine transport. In more detailed aspects, administration of an active compound of the present disclosure provides a therapeutic or prophylactic benefit by inhibiting or blocking reuptake of one or more, including any combination of two, or all three, biogenic amines selected from norepinephrine, serotonin, and dopamine.

Within more detailed treatment methods of the present disclosure, administration of the active 1-heteroaryl-3-azabicyclo[3.1.0.]hexane(s) mediates a therapeutic effect via the active compound inhibiting reuptake of norepinephrine, serotonin, and/or dopamine. Biogenic amine reuptake inhibition in this context can optionally be determined and selected by using one or more 1-heteroaryl-3-azabicyclo[3.1.0.]hexane(s) of the present disclosure to achieve variable selectivity and potency of transporter inhibition, wherein one or any combination of norepinephrine, serotonin and/or dopamine transporters can be inhibited, at predetermined levels or ratios among or between different transporters. In this context, the various 1-heteroaryl-3-azabicyclo[3.1.0.]hexanes of the present disclosure exhibit a wide range of potencies as inhibitors of one, two, or all three of the norepinephrine, serotonin and dopamine transporters--rendering them useful in a broad array of therapeutic applications.

In accordance with the present disclosure, compounds disclosed herein, optionally formulated with additional ingredients in a pharmaceutically acceptable composition, are administered to mammalian subjects, for example a human patient, to treat or prevent one or more symptom(s) of a CNS disorder alleviated by inhibiting dopamine reuptake, and/or norepinephrine reuptake, and/or serotonin reuptake. In certain aspects of the present disclosure, "treatment" or "treating" refers to amelioration of one or more symptom(s) of a CNS disorder, whereby the symptom(s) is/are alleviated by inhibiting dopamine and/or norepinephrine and/or serotonin reuptake. In other aspects of the present disclosure, "treatment" or "treating" refers to an amelioration of at least one measurable physical parameter associated with a CNS disorder. In yet another aspect of the present disclosure, "treatment" or "treating" refers to inhibiting or reducing the progression or severity of a CNS disorder (or one or more symptom(s) thereof) alleviated by inhibiting dopamine and/or norepinephrine and/or serotonin reuptake, e.g., as discerned based on physical, physiological, and/or psychological parameters. In additional aspects of the present disclosure, "treatment" or "treating" refers to delaying the onset of a CNS disorder (or one or more symptom(s) thereof) alleviated by inhibiting dopamine and/or norepinephrine and/or serotonin reuptake.

In certain aspects of the present disclosure, a compound of the present disclosure or a pharmaceutically acceptable salt thereof is administered to a mammalian subject, for example a human patient, as a preventative or prophylactic treatment against a CNS disorder (or one or more symptom(s) thereof) alleviated by inhibiting dopamine and/or norepinephrine and/or serotonin reuptake. As used herein, "prevention", "preventing", and prophylaxis refers to a reduction in the risk or likelihood that the subject will acquire a CNS disorder or one or more symptom(s) thereof, which risk or likelihood is reduced in the subject by inhibiting dopamine and/or norepinephrine and/or serotonin reuptake. Alternatively, prevention and prophylaxis may correlate with a reduced risk of recurrence of the CNS disorder or symptom(s) thereof in the subject once the subject has been cured, restored to a normal state, or placed in remission from the subject CNS disorder. In related aspects of the present disclosure, a compound or pharmaceutical composition of the present disclosure is administered as a preventative measure to the subject. Exemplary subjects amenable to prophylactic treatment in this context may have a genetic predisposition to a CNS disorder amenable to treatment by inhibiting dopamine, and/or serotonin, and/or norepinephrine reuptake, such as a family history of a biochemical imbalance in the brain, or a non-genetic predisposition to a disorder alleviated by inhibiting dopamine and/or norepinephrine and/or serotonin reuptake.

A compound of the present disclosure and pharmaceutically acceptable salts thereof are useful for treating or preventing endogenous disorders alleviated by inhibiting dopamine and/or norepinephrine and/or serotonin reuptake. Such disorders include, but are not limited to, attention-deficit disorder, depression, anxiety, obesity, Parkinson's disease, tic disorders, and addictive and substance abuse disorders.

Disorders alleviated by inhibiting dopamine and/or norepinephrine and/or serotonin reuptake are not limited to the specific disorders described herein, and the compositions and methods of the present disclosure will be understood or readily ascertained to provide effective treatment agents for treating and/or preventing a wide range of additional CNS disorders and associated symptoms. For example, the compounds of the present disclosure will provide promising candidates for treatment and/or prevention of attention deficit hyperactivity disorder and related symtoms, as well as forms and symptoms of alcohol abuse, drug abuse, cognitive distorders, obsessive compulsive behaviors, learning disorders, reading problems, gambling addiction, manic symptoms, phobias, panic attacks, oppositional defiant behavior, conduct disorder, academic problems in school, smoking, abnormal sexual behaviors, schizoid behaviors, somatization, depression, sleep disorders, general anxiety, stuttering, and tic disorders (See, for example, U.S. Pat. No. 6,132,724). Other disorders for which the compounds of the present disclosure may be useful include irritable bowel syndrome; inflammatory bowel disease; urinary tract disorders, such as stress urinary incontinence; PMDD (Premenstrual dysphoric disorder), degenerative diseases, including Alzheimers disease, and amyotrophic lateral sclerosis; and pyretic conditions (including fevers, and post-and peri-menopausal hot flashes). These and other symptoms, regardless of the underlying CNS disorder, are each targets for the novel compositions and methods of the present disclosure that mediate therapeutic benefits by inhibiting dopamine and/or norepinephrine and/or serotonin reuptake. Additional CNS disorders contemplated for treatment employing the compositions and methods of the present disclosure are described, for example, in the Quick Reference to the Diagnostic Criteria From DSM-IV (Diagnostic and Statistical Manual of Mental Disorders, Fourth Edition), The American Psychiatric Association, Washington, D.C., 1994, 358 pages. Cognitive disorders for treament and/or prevention according to the present disclosure, include, but are not limited to, Attention-Deficit/Hyperactivity Disorder, Predominately Inattentive Type; Attention-Deficit/Hyperactivity Disorder, Predominately Hyperactivity-Impulsive Type; Attention-Deficit/Hyperactivity Disorder, Combined Type; Attention-Deficit/Hyperactivity Disorder not otherwise specified (NOS); Conduct Disorder; Oppositional Defiant Disorder; and Disruptive Behavior Disorder not otherwise specified (NOS).

Depressive disorders amenable for treatment and/or prevention according to the present disclosure include, but are not limited to, Major Depressive Disorder, Recurrent; Dysthymic Disorder; Depressive Disorder not otherwise specified (NOS); and Major Depressive Disorder, Single Episode.

Addictive disorders amenable for treatment and/or prevention employing the methods and compositions of the present disclosure include, but are not limited to, eating disorders, impulse control disorders, alcohol-related disorders, nicotine-related disorders, amphetamine-related disorders, cannabis-related disorders, cocaine-related disorders, hallucinogen use disorders, inhalant-related disorders, and opioid-related disorders, all of which are further sub-classified as listed below.

Eating disorders include, but are not limited to, Bulimia Nervosa, Nonpurging Type; Bulimia Nervosa, Purging Type; and Eating Disorder not otherwise specified (NOS).

Impulse control disorders include, but are not limited to, Intermittent Explosive Disorder, Kleptomania, Pyromania, Pathological Gambling, Trichotillomania, and Impulse Control Disorder not otherwise specified (NOS).

Alcohol-related disorders include, but are not limited to, Alcohol-Induced Psychotic Disorder, with delusions; Alcohol Abuse; Alcohol Intoxication; Alcohol Withdrawal; Alcohol Intoxication Delirium; Alcohol Withdrawal Delirium; Alcohol-Induced Persisting Dementia; Alcohol-Induced Persisting Amnestic Disorder; Alcohol Dependence; Alcohol-Induced Psychotic Disorder, with hallucinations; Alcohol-Induced Mood Disorder; Alcohol-Induced Anxiety Disorder; Acohol-Induced Sexual Dysfunction; Alcohol-Induced Sleep Disorders; Alcohol-Related Disorders not otherwise specified (NOS); Alcohol Intoxication; and Alcohol Withdrawal.

Nicotine-related disorders include, but are not limited to, Nicotine Dependence, Nicotine Withdrawal, and Nicotine-Related Disorder not otherwise specified (NOS). Amphetamine-related disorders include, but are not limited to, Amphetamine Dependence, Amphetamine Abuse, Amphetamine Intoxication, Amphetamine Withdrawal, Amphetamine Intoxication Delirium, Amphetamine-Induced Psychotic Disorder with delusions, Amphetamine-Induced Psychotic Disorders with hallucinations, Amphetamine-Induced Mood Disorder, Amphetamine-Induced Anxiety Disorder, Amphetamine-Induced Sexual Dysfunction, Amphetamine-Induced Sleep Disorder, Amphetamine Related Disorder not otherwise specified (NOS), Amphetamine Intoxication, and Amphetamine Withdrawal.

Cannabis-related disorders include, but are not limited to, Cannabis Dependence; Cannabis Abuse; Cannabis Intoxication; Cannabis Intoxication Delirium; Cannabis-Induced Psychotic Disorder, with delusions; Cannabis-Induced Psychotic Disorder with hallucinations; Cannabis-Induced Anxiety Disorder; Cannabis Related Disorder not otherwise specified (NOS); and Cannabis Intoxication.

Cocaine-related disorders include, but are not limited to, Cocaine Dependence, Cocaine Abuse, Cocaine Intoxication, Cocaine Withdrawal, Cocaine Intoxication Delirium, Cocaine-Induced Psychotic Disorder with delusions, Cocaine-Induced Psychotic Disorders with hallucinations, Cocaine-Induced Mood Disorder, Cocaine-Induced Anxiety Disorder, Cocaine-Induced Sexual Dysfunction, Cocaine-Induced

Sleep Disorder, Cocaine Related Disorder not otherwise specified (NOS), Cocaine Intoxication, and Cocaine Withdrawal.

Hallucinogen-use disorders include, but are not limited to, Hallucinogen Dependence, Hallucinogen Abuse, Hallucinogen Intoxication, Hallucinogen Withdrawal, Hallucinogen Intoxication Delirium, Hallucinogen-Induced Psychotic Disorder with delusions, Hallucinogen-Induced Psychotic Disorders with hallucinations, Hallucinogen- Induced Mood Disorder, Hallucinogen-Induced Anxiety Disorder, Hallucinogen-Induced Sexual Dysfunction, Hallucinogen-Induced Sleep Disorder, Hallucinogen Related Disorder not otherwise specified (NOS), Hallucinogen Intoxication, and Hallucinogen Persisting Perception Disorder (Flashbacks).

Inhalant-related disorders include, but are not limited to, Inhalant Dependence; Inhalant Abuse; Inhalant Intoxication; Inhalant Intoxication Delirium; Inhalant-Induced Psychotic Disorder, with delusions; Inhalant-Induced Psychotic Disorder with hallucinations; Inhalant-Induced Anxiety Disorder; Inhalant Related Disorder not otherwise specified (NOS); and Inhalant Intoxication.

Opioid-related disorders include, but are not limited to, Opioid Dependence, Opioid Abuse, Opioid Intoxication, Opioid Intoxication Delirium, Opioid-Induced Psychotic Disorder, with delusions, Opioid-Induced Psychotic Disorder with hallucinations, Opioid-Induced Anxiety Disorder, Opioid Related Disorder not otherwise specified (NOS), Opioid Intoxication, and Opioid Withdrawal.

Tic disorders include, but are not limited to, Tourette's Disorder, Chronic Motor or Vocal Tic Disorder, Transient Tic Disorder, Tic Disorder not otherwise specified (NOS), Stuttering, Autistic Disorder, and Somatization Disorder.

By virtue of their multiple reuptake inhibitory activity, the novel compounds of the present invention are thus useful in a wide range of veterinary and human medical applications, in particular for treating and/or preventing a wide array of CNS disorders and/or associated symptom(s) alleviated by by inhibiting dopamine and/or norepinephrine and/or serotonin reuptake.

Within additional aspects of the present disclosure, combinatorial formulations and coordinate administration methods are provided which employ an effective amount of a compound of the invention (or a pharmaceutically effective enantiomer, salt, solvate, hydrate, polymorph, or prodrug thereof), and one or more additional active agent(s) that is/are combinatorially formulated or coordinately administered with the compound of the invention-yielding a combinatorial formulation or coordinate administration method that is effective to modulate, alleviate, treat or prevent a targeted CNS disorder, or one or more symptom(s) thereof, in a mammalian subject. Exemplary combinatorial formulations and coordinate treatment methods in this context comprise a therapeutic compound of the present disclosure in combination with one or more additional or adjunctive treatment agents or methods for treating the targeted CNS disorder or symptom(s), for example one or more antidepressant or anxiolytic agent(s) and/or therapeutic method(s).

In related aspects of the present disclosure, the compounds disclosed herein can be used in combination therapy with at least one other therapeutic agent or method. In this context, compounds of the present disclosure can be administered concurrently or sequentially with administration of a second therapeutic agent, for example a second agent that acts to treat or prevent the same, or different, CNS disorder or symptom(s) for which the compound of the present disclosure is administered. The compound of the present disclosure and the second therapeutic agent can be combined in a single composition or adminstered in different compositions. The second therapeutic agent may also be effective for treating and/or preventing a CNS disorder or associated symptom(s) by inhibiting dopamine and/or norepinephrine and/or serotonin reuptake. The coordinate administration may be done simultaneously or sequentially in either order, and there may be a time period while only one or both (or all) active therapeutic agents, individually and/or collectively, exert their biological activities and therapeutic effects. A distinguishing aspect of all such coordinate treatment methods is that the compound of the present disclosure exerts at least some detectable therapeutic activity toward alleviating or preventing the targeted CNS disorder or symptom(s), as described herein, and/or elicit a favorable clinical response, which may or may not be in conjunction with a secondary clinical response provided by the secondary therapeutic agent. Often, the coordinate administration of a compound of the present disclosure with a secondary therapeutic agent as contemplated herein will yield an enhanced therapeutic response beyond the therapeutic response elicited by either or both the compound of the present disclosure and/or secondary therapeutic agent alone.

As many of the CNS disorders and symptoms treatable or preventable using compounds of the present present disclosure are chronic, in one aspect of the present disclosure combination therapy involves alternating between administering a compound of the present disclosure and a second therapeutic agent (i.e., alternating therapy regimens between the two drugs, e.g., at one week, one month, three month, six month, or one year intervals). Alternating drug regimens in this context will often reduce or even eliminate adverse side effects, such as toxicity, that may attend long-term administration of one or both drugs alone.

In certain aspects of the present disclosure of combinatorial formulations and coordinate treatment methods of the present disclosure, the secondary therapeutic is a norepinephrine reuptake inhibitor. Examples of norepinephrine reuptake inhibitors useful in this context include tertiary amine tricyclics such as amitriptyline, clomipramine, doxepin, imipramine, (+)-trimipramine, and secondary amine tricyclics including amoxapine, atomoxetine, desipramine, maprotiline, nortriptyline, and protriptyline.

In certain aspects of the present disclosure of combinatorial formulations and coordinate treatment methods of the present disclosure, the secondary therapeutic is a serotonin reuptake inhibitor. Examples of other serotonin reuptake inhibitors useful in this context include citalopram, fluoxetine, fluvoxamine, (-)-paroxetine, sertraline, and venlafaxine.

In other aspects of the present disclosure of combinatorial formulations and coordinate treatment methods provided herein, the secondary therapeutic agent is an anti-attention-deficit-disorder treatment agent. Examples of useful anti-attention-deficit-disorder agents for use in these aspects include, but are not limited to, methylphenidate; dextroamphetamine; tricyclic antidepressants, such as imipramine, desipramine, and nortriptyline; and psychostimulants, such as pemoline and deanol.

In additional aspects of the present disclosure of combinatorial formulations and coordinate treatment methods provided herein, the secondary therapeutic agent is an anti-addictive-disorder agent. Examples of useful anti-addictive-disorder agents include, but are not limited to, tricyclic antidepressants; glutamate antagonists, such as ketamine HCl, dextromethorphan, dextrorphan tartrate and dizocilpine (MK801); degrading enzymes, such as anesthetics and aspartate antagonists; GABA agonists, such as baclofen and muscimol HBr; reuptake blockers; degrading enzyme blockers; glutamate agonists, such as D-cycloserine, carboxyphenylglycine, L-glutamic acid, and cis-piperidine-2,3-dicarboxylic acid; aspartate agonists; GABA antagonists such as gabazine (SR-95531), saclofen, bicuculline, picrotoxin, and (+) apomorphine HCl; and dopamine antagonists, such as spiperone HCl, haloperidol, and (-) sulpiride.

In other aspects of the present disclosure of combinatorial formulations and coordinate treatment methods provided herein, the secondary therapeutic agent is an anti-alcohol agent. Examples of useful anti-alcohol agents include, but are not limited to, disulfiram and naltrexone.

In other aspects of the present disclosure of combinatorial formulations and coordinate treatment methods provided herein, the secondary therapeutic agent is an anti-nicotine agent. Examples of useful anti-nicotine agents include, but are not limited to, clonidine.

In other aspects of the present disclosure of combinatorial formulations and coordinate treatment methods provided herein, the secondary therapeutic agent is an anti-opiate agent. Examples of useful anti-opiate agents include, but are not limited to, methadone, clonidine, lofexidine, levomethadyl acetate HCl, naltrexone, and buprenorphine.

In other aspects of the present disclosure of combinatorial formulations and coordinate treatment methods provided herein, the secondary therapeutic agent is anti-cocaine agent. Examples of useful anti-cocaine agents include, but are not limited to, desipramine, amantadine, fluoxidine, and buprenorphine.

In other aspects of the present disclosure of combinatorial formulations and coordinate treatment methods provided herein, the secondary therapeutic agent is an anti-lysergic acid diethylamide ("anti-LSD") agent. Examples of useful anti-LSD agents include, but are not limited to, diazepam.

In other aspects of the present disclosure of combinatorial formulations and coordinate treatment methods provided herein, the secondary therapeutic agent is an anti-phencyclidine ("anti-PCP") agent. Examples of useful anti-PCP agents include, but are not limited to, haloperidol.

In other aspects of the present disclosure of combinatorial formulations and coordinate treatment methods provided herein, the secondary therapeutic agent is an appetite suppressant. Examples of useful appetite suppressants include, but are not limited to, fenfluramine, phenylpropanolamine, and mazindol.

In yet additional aspects of the present disclosure of combinatorial formulations and coordinate treatment methods provided herein, the secondary therapeutic agent is an anti-Parkinson's-disease agent. Examples of useful anti-Parkinson's-disease agents include, but are not limited to dopamine precursors, such as levodopa, L-phenylalanine, and L-tyrosine; neuroprotective agents; dopamine agonists; dopamine reuptake inhibitors; anticholinergics such as amantadine and memantine; and 1,3,5-trisubstituted adamantanes, such as 1-amino-3,5-dimethyl-adamantane. (See, U.S. Patent No. 4,122,193)

Administration of an effective amount of a 1-heteroaryl-3-azabicyclo[3.1.0.]hexane of the present disclosure to a mammalian subject presenting with one or more symptoms of a CNS disorder or other neurological or psychiatric condition will detectably decrease, eliminate, or prevent the targeted CNS disorder and/or associated symptom(s). In exemplary aspects of the present disclosure, administration of a 1-heteroaryl-3-azabicyclo[3.1.0.]hexane composition to a suitable test subject will yield a reduction in one or more target symptom(s) associated with a selected CNS disorder, such as pain, by at least 10%, 20%, 30%, 50% or greater, up to a 75-90%, or 95% or greater, reduction in the targeted CNS disorder or one or more target symptom(s), compared to placebo-treated or other suitable control subjects. Comparable levels of efficacy are contemplated for the entire range of CNS disorders, including all contemplated neurological and psychiatric disorders, and related conditions and symptoms, for treatment or prevention using the compositions and methods of the present disclosure.

The active compounds of the present disclosure may be optionally formulated with a pharmaceutically acceptable carrier and/or various excipients, vehicles, stabilizers, buffers, preservatives, etc. An "effective amount," "therapeutic amount," "therapeutically effective amount," or "effective dose" is an effective amount or dose of an active compound as described herein sufficient to elicit a desired pharmacological or therapeutic effect in a mammalian subject--typically resulting in a measurable reduction in an occurrence, frequency, or severity of one or more symptom(s) of a CNS disorder, including any combination of neurological and/or psychological symptoms, diseases, or conditions, associated with or caused by the targeted CNS disorder, in the subject. In certain aspects of the present disclosure, when a compound of the present disclosure is administered to treat a CNS disorder, for example a pain disorder, an effective amount of the compound will be an amount sufficient *in vivo* to delay or eliminate onset of symptoms of the targeted condition or disorder. Therapeutic efficacy can alternatively be demonstrated by a decrease in the frequency or severity of symptoms associated with the treated condition or disorder, or by altering the nature, recurrence, or duration of symptoms associated with the treated condition or disorder. Therapeutically effective amounts, and dosage regimens, of the 1-heteroaryl-3-azabicyclo[3.1.0.]hexane compositions of the present disclosure, including pharmaceutically effective salts, solvates, hydrates, polymorphs or prodrugs thereof, will be readily determinable by those of ordinary skill in the art, often based on routine clinical or patient-specific factors.

Suitable routes of administration for a 1-heteroaryl-3-azabicyclo[3.1.0.]hexane of the present disclosure include, but are not limited to, oral, buccal, nasal, aerosol, topical, transdermal, mucosal, injectable, slow release, controlled release, iontophoresis, sonophoresis, and other conventional delivery routes, devices and methods. Injectable delivery methods are also contemplated, including but not limited to, intravenous, intramuscular, intraperitoneal, intraspinal, intrathecal, intracerebroventricular, intraarterial, and subcutaneous injection.

Suitable effective unit dosage amounts of a 1-heteroaryl-3-azabicyclo[3.1.0.]hexane of the present disclosure for mammalian subjects may range from about 25 to 1800 mg, 50 to 1000mg, 75 to 900 mg, 100 to 750 mg, or 150 to 500 mg. In certain aspects of the present disclosure, the effective dosage will be selected within narrower ranges of, for example, 10 to 25 mg, 30-50 mg, 75 to 100mg, 100 to 250 mg, or 250 to 500 mg. These and other effective unit dosage amounts may be administered in a single dose, or in the form of multiple daily, weekly or monthly doses, for example in a dosing regimen comprising from 1 to 5, or 2-3, doses administered per day, per week, or per month. In exemplary aspects of the present disclosure, dosages of 10 to 25 mg, 30-50 mg, 75 to 100 mg, 100 to 250 mg, or 250 to 500 mg, are administered one, two, three, or four times per day. In more detailed aspects of the present disclosure, dosages of 50-75 mg, 100-200 mg, 250-400 mg, or 400-600 mg are administered once or twice daily. In alternate aspects of the present disclosure, dosages are calculated based on body weight, and may be administered, for example, in amounts from about 0.5mg/kg to about 20mg/kg per day, 1mg/kg to about 15mg/kg per day, 1mg/kg to about 10mg/kg per day, 2mg/kg to about 20mg/kg per day, 2mg/kg to about 10mg/kg per day or 3mg/kg to about 15mg/kg per day.

The amount, timing and mode of delivery of compositions of the present disclosure comprising an effective amount of a 1-heteroaryl-3-azabicyclo[3.1.0.]hexane of the present disclosure will be routinely adjusted on an individual basis, depending on such factors as weight, age, gender, and condition of the individual, the acuteness of the condition to be treated and/or related symptoms, whether the administration is prophylactic or therapeutic, and on the basis of other factors known to effect drug delivery, absorption, pharmacokinetics, including half-life, and efficacy. An effective dose or multi-dose treatment regimen for the compounds of the present disclosure will ordinarily be selected to approximate a minimal dosing regimen that is necessary and sufficient to substantially prevent or alleviate one or more symptom(s) of a neurological or psychiatric condition in the subject, as described herein. Thus, following administration of a 1-heteroaryl-3-azabicyclo[3.1.0.]hexane of the present disclosure according to the formulations and methods herein, test subjects will exhibit a 10%, 20%, 30%, 50% or greater reduction, up to a 75-90%, or 95% or greater, reduction, in one or more symptoms associated with a targeted CNS disorder or other neurological or psychiatric condition, compared to placebo-treated or other suitable control subjects.

Pharmaceutical dosage forms of the 1-heteroaryl-3-azabicyclo[3.1.0.]hexanes of the present disclosure may optionally include excipients recognized in the art of pharmaceutical compounding as being suitable for the preparation of dosage units as discussed above. Such excipients include, without limitation, binders, fillers, lubricants, emulsifiers, suspending agents, sweeteners, flavorings, preservatives, buffers, wetting agents, disintegrants, effervescent agents and other conventional excipients and additives.

The compositions of the present disclosure for treating CNS disorders, including depression, anxiety, and/or pain, can thus include any one or combination of the following: a pharmaceutically acceptable carrier or excipient; other medicinal agent(s); pharmaceutical agent(s); adjuvants; buffers; preservatives; diluents; and various other pharmaceutical additives and agents known to those skilled in the art. These additional formulation additives and agents will often be biologically inactive and can be administered to patients without causing unacceptable deleterious side effects or serious adverse interactions with the active agent.

If desired, the substituted 1-heteroaryl-3-azabicyclo[3.1.0.]hexanes of the present disclosure can be administered in a controlled release form, for example by use of a slow release carrier such as a hydrophilic, slow release polymer. Exemplary controlled release agents in this context include, but are not limited to, hydroxypropyl methyl cellulose, having a viscosity in the range of about 100 cps to about 100,000 cps.

1-heteroaryl-3-azabicyclo[3.1.0.]hexane compositions of the present disclosure will often be formulated and administered in an oral dosage form, optionally in combination with a carrier or other additive(s). Suitable carriers common to pharmaceutical formulation technology include, but are not limited to, microcrystalline cellulose, lactose, sucrose, fructose, glucose, dextrose, or other sugars, di-basic calcium phosphate, calcium sulfate, cellulose, methylcellulose, cellulose derivatives, kaolin, mannitol, lactitol, maltitol, xylitol, sorbitol, or other sugar alcohols, dry starch, dextrin, maltodextrin or other polysaccharides, inositol, or mixtures thereof. Exemplary unit oral dosage forms for use in this dislcosure include tablets, which may be prepared by any conventional method of preparing pharmaceutical oral unit dosage form. Oral unit dosage forms, such as tablets, may contain one or more conventional additional formulation ingredients, including, but not limited to, release modifying agents, glidants, compression aides, disintegrants, lubricants, binders, flavors, flavor enhancers, sweeteners and/or preservatives. Suitable lubricants include stearic acid, magnesium stearate, talc, calcium stearate, hydrogenated vegetable oils, sodium benzoate, leucine carbowax, magnesium lauryl sulfate, colloidal silicon dioxide and glyceryl monostearate. Suitable glidants include colloidal silica, fumed silicon dioxide, silica, talc, fumed silica, gypsum and glyceryl monostearate. Substances which may be used for coating include hydroxypropyl cellulose, titanium oxide, talc, sweeteners and colorants. The aforementioned effervescent agents and disintegrants are useful in the formulation of rapidly disintegrating tablets known to those skilled in the art. These typically disintegrate in the mouth in less than one minute, and preferably in less than thirty seconds. By effervescent agent is meant a couple, typically an organic acid and a carbonate or bicarbonate. Such rapidly acting dosage forms would be useful, for example, in the prevention or treatment of acute attacks of panic disorder.

Additional 1-heteroaryl-3-azabicyclo[3.1.0.]hexane compositions of the present disclosure can be prepared and administered in any of a variety of inhalation or nasal delivery forms known in the art. Devices capable of depositing aerosolized substituted 1-heteroaryl-3-azabicyclo[3.1.0.]hexane formulations in the sinus cavity or pulmonary alveoli of a patient include metered dose inhalers, nebulizers, dry powder generators, sprayers, and the like. Pulmonary delivery to the lungs for rapid transit across the alveolar epithelium into the blood stream may be particularly useful in treating impending episodes of seizures or panic disorder. Methods and compositions suitable for pulmonary delivery of drugs for systemic effect are well known in the art. Suitable formulations, wherein the carrier is a liquid, for administration, as for example, a nasal spray or as nasal drops, may include aqueous or oily solutions of a 1-heteroaryl-3-azabicyclo[3.1.0.]hexane, and any additional active or inactive ingredient(s).

Intranasal and pulmonary delivery permits the passage of active compounds of the present disclosure into the blood stream directly after administering an effective amount of the compound to the nose or lung. In the case of intranasal delivery, this mode of administration can achieve direct, or enhanced, delivery of the active compound to the CNS. For intranasal and pulmonary administration, a liquid aerosol formulation will often contain an active compound of the present disclosure combined with a dispersing agent and/or a physiologically acceptable diluent. Alternatively, dry powder aerosol formulations may contain a finely divided solid form of the subject compound and a dispersing agent allowing for the ready dispersal of the dry powder particles. With either liquid or dry powder aerosol formulations, the formulation must be aerosolized into small, liquid or solid particles in order to ensure that the aerosolized dose reaches the mucous membranes of the nasal passages or the lung. The term "aerosol particle" is used herein to describe a suitable liquid or solid particle of a sufficiently small particle diameter, e.g., in a range of from about 2-5 microns, for nasal or pulmonary distribution to targeted mucous or alveolar membranes. Other considerations include the construction of the delivery device, additional components in the formulation, and particle characteristics. These aspects of nasal or pulmonary administration of drugs are well known in the art, and manipulation of formulations, aerosolization means, and construction of delivery devices, is within the level of ordinary skill in the art.

Yet additional compositions and methods of the present disclosure are provided for topical administration of 1-heteroaryl-3-azabicyclo[3.1.0.]hexanes for treating CNS disorders, including pain. Topical compositions may comprise a 1-heteroaryl-3-azabicyclo[3.1.0.]hexane and any other active or inactive component(s) incorporated in a dermatological or mucosal acceptable carrier, including in the form of aerosol sprays, powders, dermal patches, sticks, granules, creams, pastes, gels, lotions, syrups, ointments, impregnated sponges, cotton applicators, or as a solution or suspension in an aqueous liquid, non-aqueous liquid, oil-in-water emulsion, or water-in-oil liquid emulsion. These topical compositions may comprise a 1-heteroaryl-3-azabicyclo[3.1.0.]hexane dissolved or dispersed in a portion of a water or other solvent or liquid to be incorporated in the topical composition or delivery device. Transdermal administration may be enhanced by the use of dermal penetration enhancers known to those skilled in the art.

Yet additional 1-heteroaryl-3-azabicyclo[3.1.0.]hexane formulations are provided for parenteral administration, including aqueous and non-aqueous sterile injection solutions which may optionally contain anti-oxidants, buffers, bacteriostats and/or solutes which render the formulation isotonic with the blood of the mammalian subject; and aqueous and non-aqueous sterile suspensions which may include suspending agents and/or thickening agents. The formulations may be presented in unit-dose or multi-dose containers.

1-heteroaryl-3-azabicyclo[3.1.0.]hexane formulations of the present disclosure may also include polymers for extended release following parenteral administration. Extemporaneous injection solutions, emulsions and suspensions may be prepared from sterile powders, granules and tablets of the kind previously described. Preferred unit dosage formulations are those containing a daily dose or unit, daily sub-dose, as described herein above, or an appropriate fraction thereof, of the active ingredient(s).

In more detailed aspects of the present disclosure, 1-heteroaryl-3-azabicyclo[3.1.0.]hexanes may be encapsulated for delivery in microcapsules, microparticles, or microspheres, prepared, for example, by coacervation techniques or by interfacial polymerization, for example, hydroxymethylcellulose or gelatin-microcapsules and poly(methylmethacylate) microcapsules, respectively, in colloidal drug delivery systems (for example, liposomes, albumin microspheres, microemulsions, nano-particles and nanocapsules) or in macroemulsions.

The present disclosure also provides pharmaceutical packs or kits comprising one or more containers holding a 1-heteroaryl-3-azabicyclo[3.1.0.]hexane, or any composition comprising a 1-heteroaryl-3-azabicyclo[3.1.0.]hexane as described herein, including pharmaceutically acceptable salts and other forms of 1-heteroaryl-3-azabicyclo[3.1.0.]hexanes as described, in a pharmaceutically acceptable, stable form. Optionally packaged with these packs and kits can be a notice, e.g., in a form prescribed by a governmental agency regulating pharmaceuticals or biological products, reflecting approval by the agency of the manufacture, use and/or sale of the product contained in the pack or kit for human administration (optionally specifying one or more approved treatment indications as described herein).

The pharmaceutical agents of the present disclosure may be administered parenterally, e.g. intravenously, intramuscularly, subcutaneously or intraperitoneally. The parenteral preparations may be solutions, dispersions or emulsions suitable for such administration. The subject agents may also be formulated into polymers for extended release following parenteral administration. Pharmaceutically acceptable formulations and ingredients will typically be sterile or readily sterilizable, biologically inert, and easily administered. Such polymeric materials are well known to those of ordinary skill in the pharmaceutical compounding arts. Parenteral preparations typically contain buffering agents and preservatives, and may be lyophilized to be re-constituted at the time of administration.

Compounds and compositions of the present disclosure are also useful in a variety of in vitro applications, including a range of diagnostic uses. In certain in vitro assays, compounds and compositions of the present disclosure can be used as CNS imaging agents. In other aspects of the present disclosure, the compounds of the present disclosure can be used in a variety of conventional, clinical assays to determine whether it is desired to administer a compound of the present disclosure, or a particular dosage form or quantity of the compound, to a particular patient as a therapeutic agent. For example, assays employing cell cultures, tissue cultures, or animal model systems can be used to demonstrate safety and efficacy of the compounds and pharmaceutical formulations described herein. Additional uses of the compounds of the present disclosure, e.g., in radiolabeled or other labeled form, can be used to study biochemical mechanisms, metabolic processes, pharmacokinetics, etc. of the subject compounds and/or their targets in a diverse array of in vitro, ex vivo, and in vivo assays. Each of the foregoing general applications of the subject compounds will be understood by those skilled in the art to have many corresponding aspects and modified formats following conventional methods and procedures widely known in the art.

The following examples illustrate certain embodiments of the present invention, and are not to be construed as limiting the present disclosure.

### Example I

### Preparation of 1-heteroaryl-3-azabicyclo[3.1.0]hexanes using Reaction Scheme 1

### A. Synthesis of 1-(pyridine-2-yl)-3-azabicyclo[3.1.0]hexane, dihydrochloride

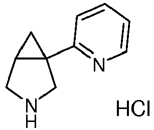

A cooled (-25°C) stirred suspension of sodium amide (0.43g, 11 mmol) in anhydrous THF (20mL) under nitrogen was treated with a solution of pyridine-2-acetonitrile (591mg, 5.0 mmol) in anhydrous THF (5mL) and stirred at room temperature for 3h. The mixture was cooled (-25°C) and treated with a solution of epichlorohydrin (0.52mL, 6.0 mmol) in anhydrous THF (2mL) in one portion, then stirred at room temperature for 1.5h. Reaction was incomplete, so the solution was heated to 35°C for 1.5h, then cooled on an ice bath. Saturated aqueous ammonium chloride (5mL) was added, followed by ethyl acetate (70mL). The organic layer was separated, the aqueous extracted with more ethyl acetate (30mL), and the combined organic layers dried (MgSO₄) and concentrated *in vacuo*. The residue was dissolved in methylene chloride and loaded onto a silica gel column (~150cc) and eluted with 1:1 methylene chloride/ethyl acetate to afford the intermediate cyanocyclopropanemethanol (447mg, 51%) as a 4:1 E/Z mixture of isomers (by NMR).

An ice-cooled (3°C) stirred solution of lithium aluminum hydride/THF (3.0mL, 3.0 mmol) under nitrogen was treated dropwise with a solution of the above cyanocyclopropanemethanol (348mg, 2.0 mmol) in THF (3mL), and the mixture stirred on an ice bath for 2h and carefully quenched dropwise by successive addition of water (0.115mL), 15% aqueous sodium hydroxide (0.115mL), and water (0.35mL). The suspension was diluted with THF to loosen it up, stirred a few minutes, filtered, and the filtrate concentrated *in vacuo* (toluene added to ensure water removal). The residual oil was dissolved in anhydrous 1,2-dichloroethane (DCE, 12mL), cooled (3°C), and treated with thionyl chloride (0.175mL, 2.4 mmol). The mixture was warmed to room temperature, stirred 3h, concentrated *in vacuo*, and the residue taken up in water (10mL). The aqueous solution was made basic with 5N NaOH (~2mL), extracted with methylene chloride containing a little 2-propanol (5X15mL), and the combined extracts dried (Na₂SO₄) and concentrated *in vacuo*. The residue was dissolved in methylene chloride and loaded onto a silica gel column (~50cc) and eluted with 80:18:2 methylene chloride/ethanol/ammonium hydroxide to afford 1-(pyridine-2-yl)-3-azabicyclo[3.1.0]hexane (225mg, 70%) as a pale amber oil. MS (M+1) 161.0. ¹H NMR (CDCl₃) δ 8.48 (m, 1H), 7.56 (m, 1H), 7.03-7.12 (m, 2H), 3.40 (m, 1H), 3.31 (d, 1H, *J*=11Hz), 3.11 (dd, 1H, *J*=11Hz,3Hz), 3.04 (d, 1H, *J*=11Hz), 1.98 (m, 1H), 1.31 (m, 1H), 0.97 (t, 1H, *J*=5Hz).

The above free base (190mg, 1.18 mmol) was dissolved in methanol (5mL) and treated with 2N HCl/ether (1.5mL, 3 mmol) and the resulting solution was concentrated *in vacuo.* The residue was triturated from a mixture of ethanol and acetonitrile to afford 1-(pyridine-2-yl)-3-azabicyclo[3.1.0]-hexane, dihydrochloride (184mg, 67%) as a pale tan solid. MS (M+1) 161.0. ¹H NMR (DMSO-d₆) δ 10.21 (br s, 1H), 9.98 (br s, 1H), 8.64 (m, 1H), 8.24 (t, 1H, *J*=8Hz), 7.75 (d, 1H, *J*=8Hz), 7.67 (t, 1H, *J*=7Hz), 3.73 (m, 2H), 3.45-3.57 (m, 1H), 3.38 (m, 1H), 2.51 (m, 1H), 1.74 (t, 1H, *J*=6Hz), 1.50 (dd, 1H, *J*=6Hz,9Hz). ¹³C NMR (DMSO-d₆) δ 155.19, 144.08, 142.81, 124.12, 123.87, 47.84, 46.34, 30.39, 25.75, 16.55.

### B. Synthesis of 3-methyl-1-(pyridine-2-yl)-3-azabicyclo[3.1.0]hexane, dihydrochloride

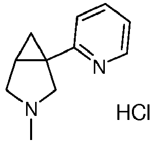

A solution of 1-(pyridine-2-yl)-3-azabicyclo[3.1.0]hexane (240mg, 1.5 mmol) in anhydrous methylene chloride (10mL) was treated with di-tert-butyl dicarbonate (382mg, 1.75 mmol), stirred for 2h, and concentrated *in vacuo* (with toluene added late). An ice-cooled stirred solution of 1N LAH/THF (4.5mL, 4.5 mmol) under nitrogen was treated dropwise with a solution of the above residue in anhydrous THF (3.5mL), then the mixture was stirred for 1h at room temperature and for 5h at reflux, cooled on an ice bath, and carefully quenched dropwise successively with water (0.17mL), 15% aqueous sodium hydroxide (0.17mL), and water (0.50mL). The suspension was diluted with THF to loosen it up, stirred a few minutes, filtered, and the filtrate concentrated *in vacuo*. The residue was dissolved in methylene chloride, loaded onto a silica gel column (~75cc), and eluted with 95:4.5:0.5, then 90:9:1 methylene chloride/ethanol/ammonium hydroxide to afford a colorless oil. This was dissolved in methanol (5mL), treated with 2N HCl/ether (1.5mL, 3.0mL), and concentrated *in vacuo* to a solid. Trituration from ether afforded 3-methyl-1-(pyridine-2-yl)-3-azabicyclo[3.1.0]hexane, dihydrochloride (214mg, 58%) as a white solid. MS (M+1) 175.1. ¹H NMR (DMSO-d₆) δ 11.63 (br s, 1H), 8.64 (m, 1H), 8.23 (t, 1H, *J*=8Hz) 7.73 (d, 1H, *J*=8Hz), 7.66 (t, 1H, *J*=7Hz), 3.82-3.97 (m, 2H), 3.61 (m, 2H), 2.81 (br s, 3H), 2.45 (m, 1H), 2.03 (t, 1H, *J*=6Hz), 1.52 (m, 1H). ¹³C NMR (DMSO-d₆) δ 154.90, 144.42, 142.52, 123.94, 57.18, 55.65, 30.25, 25.69, 15.93.

### C. Synthesis of 1-(pyridine-3-yl)-3-azabicyclo[3.1.0]hexane, dihydrochloride

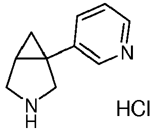

A cooled (-25°C) stirred solution of pyridine-3-acetonitrile (1.18g, 10 mmol) in anhydrous THF (25mL) under nitrogen was treated with 1.0N sodium bis(trimethylsilyl)amide (11mL, 11 mmol), warmed to room temperature, stirred 10 min, then recooled (-25°C). More base (12mL, 12 mmol) was added, followed by epichlorohydrin (1.04mL, 12 mmol) in one portion, and the mixture was stirred at room temperature for 2h, cooled on an ice bath, and quenched (10mL saturated aqueous ammonium chloride), followed by dilution with ethyl acetate (100mL). The organic layer was separated and the aqueous extracted with ethyl acetate (25mL). The combined organic solution was dried (MgSO₄) and concentrated *in vacuo*, and the residual brown solid dissolved in methylene chloride and loaded onto a silica gel column (~250cc). This was eluted with ethyl acetate, then 5% ethanol/ethyl acetate, then 10% ethanol/ethyl acetate to afford an orange solid, which was recrystallized from ethyl acetate/heptane to afford the intermediate cyanocyclopropanemethanol (625mg, 36%) as a 3.5:1 E/Z mixture of isomers (by NMR). An ice-cooled (3°C) stirred solution of lithium aluminum hydride/THF (4.1mL, 4.1 mmol) under nitrogen was treated dropwise with a solution of the above cyanocyclopropanemethanol (470mg, 2.7 mmol) in THF (4mL), and the mixture stirred on an ice bath for 2h and carefully quenched dropwise successively with water (0.16mL), 15% aqueous sodium hydroxide (0.16mL), and water (0.48mL). The suspension was diluted with THF/isopropanol to loosen it up, stirred a few minutes, filtered, and the filtrate concentrated *in vacuo* (toluene added to ensure water removal). The residual oil was dissolved in anhydrous 1,2-dichloroethane (DCE, 12mL), cooled (3°C), and treated with thionyl chloride (0.22mL, 3.0 mmol). The mixture was warmed to room temperature, stirred 3h, concentrated *in vacuo*, and the residue taken up in water (10mL). The aqueous solution was basified with 5N NaOH (~3mL), extracted with methylene chloride containing a little 2-propanol (4X25mL), and the combined extracts dried (MgSO₄) and concentrated *in vacuo*. The residue was dissolved in methylene chloride and loaded onto a silica gel column (~50cc) and eluted with 80:18:2 methylene chloride/ethanol/ammonium hydroxide to afford 1-(pyridine-3-yl)-3-azabicyclo[3.1.0]hexane (156mg, 36%) as a pale yellow oil. The above free base (68mg, 0.424 mmol) was dissolved in methanol (2mL) and treated with 2N HCl/ether (1.0mL, 2.0 mmol), then the solution was concentrated *in vacuo*. The residue was rinsed with ether and triturated from acetonitrile to afford 1-(pyridine-3-yl)-3-azabicyclo[3.1.0]- hexane, dihydrochloride (67mg, 68%) as a pale tan solid. MS (M+1) 161.0. ¹H NMR (DMSO-d₆) δ 10.20 (br s, 1H), 9.95 (br s, 1H), 8.87 (m, 1H), 8.74 (d, 1H, *J*=5Hz), 8.41 (m, 1H), 7.94 (dd, 1H, *J*=6Hz,9Hz), 3.74 (m, 1H), 3.42-3.60 (m, 2H), 3.37 (m, 1H), 2.37 (m, 1H), 1.64 (t, 1H, *J*=6Hz), 1.30 (m, 1H). ¹³C NMR (DMSO-d₆) δ 143.15, 141.00, 140.31, 139.35, 126.38, 48.27, 46.41, 28.42, 24.30, 16.15.

### D. Synthesis of 3-Methyl-1-(pyridine-3-yl)-3-azabicyclo[3.1.0]hexane, dihydrochloride

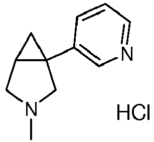

A solution of 1-(pyridine-3-yl)-3-azabicyclo[3.1.0]hexane (81.7mg, 0.51 mmol) in anhydrous methylene chloride (3mL) was treated with di-tert-butyl dicarbonate (120mg, 0.55 mmol), stirred for 2h, and concentrated *in vacuo* (with toluene added late). An ice-cooled stirred solution of 1N LAH/THF (1.5mL, 1.5 mmol) under nitrogen was treated dropwise with a solution of the above residue in anhydrous THF (1.5mL), then the mixture was stirred for 1h at room temperature and for 5h at reflux, cooled on an ice bath, and carefully quenched dropwise successively with water (0.07mL), 15% aqueous sodium hydroxide (0.07mL), and water (0.22mL). The suspension was diluted with THF/isopropanol to loosen it up, stirred a few minutes, filtered, and the filtrate concentrated *in vacuo*. The residue was dissolved in methylene chloride, loaded onto a silica gel column (~70cc), and eluted with 95:4.5:0.5, then 90:9:1 methylene chloride/ethanol/ammonium hydroxide to afford a pale tan oil. This was dissolved in methanol (5mL), treated with 2N HCl/ether (0.75mL, 1.5mL), and concentrated *in vacuo* to a solid. Trituration from ether afforded 3-methyl-1-(pyridine-3-yl)-3-azabicyclo[3.1.0]hexane, dihydrochloride (73mg, 58%) as a pale tan solid. MS (M+1) 175.0. ¹H NMR (DMSO-d₆) δ 11.59 (br s, 1H), 8.91 (m, 1H), 8.76 (d, 1H, *J*=5Hz), 8.43 (m, 1H), 7.94 (dd, 1H, J=6Hz,9Hz), 3.93 (m, 1H), 3.58-3.70 (m, 2H), 3.50 (m, 1H), 2.79 (br s, 3H), 2.33 (m, 1H), 1.97 (t, 1H, *J*=6Hz), 1.34 (m, 3H). ¹³C NMR (DMSO-d₆) δ 143.13, 141.39, 140.63, 138.89, 126.34, 57.54, 55.71, 28.27, 24.37, 15.39.

### E. Synthesis of 1-(pyridine-4-yl)-3-azabicyclo[3.1.0]hexane, dihydrochloride

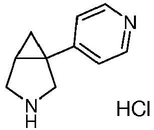

A cooled (-25°C) stirred solution of pyridine-4-acetonitrile (1.83g, 15.5 mmol) in anhydrous THF (40mL) under nitrogen was treated with 1.0N sodium bis(trimethylsilyl)amide (16mL, 16mmol), warmed to room temperature, stirred 15min, then recooled (-25°C). More base (17mL, 17 mmol) was added, followed by epichlorohydrin (1.56mL, 18 mmol) in one portion, and the mixture was stirred at room temperature for 18h, cooled on an ice bath, and quenched (15mL saturated aqueous ammonium chloride), followed by dilution with ethyl acetate (200mL). The organic layer was separated and the aqueous extracted with ethyl acetate (50mL). The combined organic solution was dried (MgSO₄) and concentrated *in vacuo*, and the residual brown oil dissolved in methylene chloride and loaded onto a silica gel column (~300cc). This was eluted with 10% ethanol/methylene chloride to afford the intermediate cyanocyclopropanemethanol (750mg, 28%) as a viscous amber oil, a 5:1 E/Z mixture of isomers (by NMR).

An ice-cooled (3°C) stirred solution of lithium aluminum hydride/THF (6.5mL, 6.5 mmol) under nitrogen was treated dropwise with a solution of the above cyanocyclopropanemethanol (740mg, 4.25 mmol) in THF (6mL), and the mixture stirred on an ice bath for 2h and carefully quenched dropwise successively with water (0.25mL), 15% aqueous sodium hydroxide (0.25mL), and water (0.75mL). The suspension was diluted with THF/isopropanol to loosen it up, stirred a few minutes, filtered, and the filtrate concentrated *in vacuo* (toluene added to ensure water removal). The residual oil was dissolved in anhydrous 1,2-dichloroethane (DCE, 20mL), cooled (3°C), and treated with thionyl chloride (0.33mL, 4.5 mmol). The mixture was warmed to room temperature, stirred 3h, concentrated *in vacuo*, and the residue taken up in water (10mL). The aqueous solution was basified with 5N NaOH (~4mL), extracted with methylene chloride containing a little 2-propanol (5X25mL), and the combined extracts dried (Na₂SO₄) and concentrated *in vacuo*. The residue was dissolved in methylene chloride and loaded onto a silica gel column (~100cc) and eluted with 90:9:1, then 85:13.5:1.5 methylene chloride/ethanol/ammonium hydroxide to afford 1-(pyridine-4-yl)-3-azabicyclo[3.1.0]hexane (175mg, 26%) as an amber oil.

The above free base (101mg, 0.63 mmol) was dissolved in methanol (5mL) and treated with 2N HCl/ether (1.0mL, 2.0 mmol), then the solution was concentrated *in vacuo.* The residue was triturated from ether to afford 1-(pyridine-4-yl)-3-azabicyclo[3.1.0]hexane, dihydrochloride (130mg, 88%) as a white solid. MS (M+1) 161.0. ¹H NMR (DMSO-d₆) δ 10.33 (br s, 1H), 10.10 (br s, 1H), 8.79 (d, 2H, *J*=7Hz), 7.82 (d, 2H, *J*=7Hz), 3.78 (m, 1H), 3.64 (m, 1H), 3.41 (m, 2H), 2.58 (m, 1H), 1.94 (t, 1H, *J*=6Hz), 1.49 (m, 1H). ¹³C NMR (DMSO-d₆) δ 161.21, 141.84, 124.18, 47.35, 46.78, 31.72, 28.81, 21.28.

### F. Synthesis of 3-methyl-1-(pyridine-4-yl)-3-azabicyclo[3.1.0]hexane, dihydrochloride

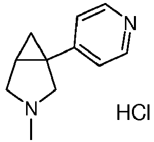

A stirred solution of 1-(pyridine-4-yl)-3-azabicyclo[3.1.0]hexane (272mg, 1.7 mmol) in DCE (50mL) was treated with 37% aqueous formaldehyde (1.02mL, 13.6 mmol), then with sodium triacetoxyborohydride (1.44g, 6.8 mmol) and stirred at room temperature for 3h. 1N sodium hydroxide (25mL) was added, stirring continued for 15min, the organic layer was separated, and the aqueous solution extracted with methylene chloride (2X50mL). The combined organic extracts were dried (MgSO₄), concentrated *in vacuo,* dissolved in methylene chloride, and loaded onto a silica gel column (∼100cc). This was eluted with 95:4.5:0.5, then 90:10:1 methylene chloride/ethanol/ammonium hydroxide to afford product free base as a colorless oil. This was dissolved in methanol, treated with 2N HCl/ether (2mL, 4mmol), concentrated *in vacuo,* and the residual solid triturated from ether to afford 3-methyl-1-(pyridine-4-yl)-3-azabicyclo[3.1.0]hexane, dihydrochloride (285mg, 68%) as a white solid. MS (M+1) 175.0. ¹H NMR (DMSO-d₆) δ 11.76 (br s, 1H), 8.80 (d, 2H, *J*=7Hz), 7.78 (d, 2H, *J*=7Hz), 3.99 (m, 1H), 3.76 (m, 1H), 3.64 (m, 1H), 3.46 (m, 1H), 2.79 (br s, 3H), 2.57 (m, 1H), 2.25 (t, 1H, *J*=6Hz), 1.49 (m, 1H). ¹³C NMR (DMSO-d₆) δ 159.52, 141.58, 123.31, 55.82, 55.29, 30.63, 27.94, 19.99.

### G. Synthesis of 1-(6-methoxypyridine-3-yl)-3-azabicyclo[3.1.0]hexane, dihydrochloride

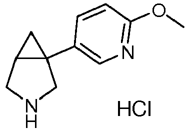

A cooled (-25°C) stirred solution of 6-methoxypyridine-3-acetonitrile (1.48g, 10.0 mmol) in anhydrous THF (25mL) under nitrogen was treated with 1.0N sodium bis(trimethylsilyl)amide (11mL, 11 mmol), warmed to room temperature, stirred 15min, then recooled (-25°C). More base (12mL, 12 mmol) was added, followed by epichlorohydrin (1.04mL, 12 mmol) in one portion, and the mixture was stirred at room temperature for 2h, cooled on an ice bath, and quenched (10mL saturated aqueous ammonium chloride), followed by dilution with ethyl acetate (100mL). The organic layer was separated and the aqueous extracted with ethyl acetate (25mL). The combined organic solution was dried (MgSO₄) and concentrated *in vacuo,* and the residue dissolved in ethyl acetate and loaded onto a silica gel column (∼150cc). This was eluted with ethyl acetate to afford the intermediate cyanocyclopropanemethanol (970mg, 47%) as a viscous pale yellow oil, a 5:2 E/Z mixture of isomers (by NMR).

An ice-cooled (3°C) stirred solution of the above cyanocyclopropanemethanol (960mg, 4.7 mmol) in anhydrous THF (8mL) under nitrogen was treated dropwise with 1N LAH/THF (7.1mL, 7.1mmol) at a rate to keep pot temp<8°C, then stirred at 3°C for 2h and carefully dropwise quenched with water (0.27mL), 15% aqueous sodium hydroxide (0.27mL), and water (0.80mL). The thick suspension was diluted with THF to facilitate stirring, stirred for 15min, then filtered through Celite^{®} (filter cake rinsed with THF). The filtrate was concentrated *in vacuo* and the residue was dissolved in methylene chloride and loaded onto a column of silica gel (∼130cc), then eluted with 80:18:2 methylene chloride/ethanol/ammonium hydroxide to afford the intermediate aminomethylcyclopropanemethanol (496mg, 51%) as a pale yellow oil.

An ice-cooled (3°C) stirred solution of the above aminomethylcyclopropanemethanol (489mg, 2.35 mmol) in anhydrous DCE (12mL) under nitrogen was treated dropwise with thionyl chloride (0.205mL, 2.8 mmol), then stirred for 3h at room temperature and concentrated *in vacuo.* The residue was taken up in water (10mL), stirred for 15min, basified with 5N NaOH (3mL), and extracted with methylene chloride containing a little isopropanol (4X25mL). The combined organic solution was rinsed with water (30mL), dried (MgSO₄), and concentrated *in vacuo.* The residue was dissolved in methylene chloride, loaded onto a silica gel column (∼75cc), and eluted with 90:9:1, then 80:18:2 methylene chloride/ethanol/ammonium hydroxide to afford bicyclic amine free base (192mg, 43%) as a pale yellow viscous oil.

A solution of the above bicyclic amine free base (70mg, 0.368 mmol) in methanol (5mL) was treated with 2N HCl/ether (0.5mL, 1.0 mmol), the solution concentrated *in vacuo,* and the residual solid triturated successively with ether and acetonitrile to afford 1-(6-methoxypyridine-3-yl)-3-azabicyclo[3.1.0]hexane, dihydrochloride (82mg, 85%) as a white solid. MS (M+1) 190.9. ¹H NMR (DMSO-d₆) δ 9.96 (br s, 1H), 9.71 (br s, 1H), 8.11 (m, 1H), 7.67 (m, 1H), 6.81 (m, 1H), 3.82 (s, 3H), 3.61 (m, 1H), 3.45 (m, 1H), 3.28-3.40 (m, 2H), 2.05 (m, 1H), 1.39 (m, 1H), 1.04 (m, 1H). ¹³C NMR (DMSO-d₆) δ 162.27, 144.91, 138.90, 128.04, 110.23, 53.49, 49.40, 46.68, 27.93, 22.48, 14.45.

### H. Synthesis of 1-(6-methoxypyridine-3-yl)-3-methyl-3-azabicyclo[3.1.0]hexane, dihydrochloride

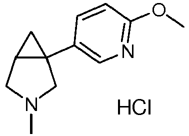

A stirred solution of 1-(6-methoxypyridine-3-yl)-3-azabicyclo[3.1.0]hexane (90mg, 0.473 mmol) in DCE (15mL) was treated with 37% aqueous formaldehyde (0.29mL, 3.8 mmol), followed by sodium triacetoxyborohydride (0.403g, 1.9 mmol), and stirred at room temperature for 3h. 1N sodium hydroxide (6mL) was added, stirring continued for 15min, and the organic layer was separated and the aqueous solution extracted with methylene chloride (3X15mL). The combined organic extracts were dried (Na₂SO₄), concentrated *in vacuo,* dissolved in methylene chloride, and loaded onto silica gel (∼50cc). This was eluted with 95:4.5:0.5, then 85:13.5:1.5 methylene chloride/ethanol/ammonium hydroxide to afford the above free base as a colorless oil, which was dissolved in methanol (5mL). The stirred solution was treated with 2N HCl/ether (0.5mL, 1.0mmol), concentrated *in vacuo,* and the residual solid triturated with ether to afford 1-(6-methoxypyridine-3-yl)-3-methyl-3-azabicyclo[3.1.0]hexane, dihydrochloride (101mg, 77%) as a white solid. MS (M+1) 204.9. ¹H NMR (DMSO-d₆) δ 11.36 (br s, 1H), 8.12 (m, 1H), 7.71 (m, 1H), 6.82 (m, 1H), 3.82 (s, 3H), 3.77-3.87 (m, 1H), 3.57 (m, 1H), 3.45 (m, 2H), 2.76 (br s, 3H), 2.04 (m, 1H), 1.76 (m, 1H), 1.05 (m, 1H). ¹³C NMR (DMSO-d₆) δ 162.36, 145.07, 138.91, 127.78, 110.21, 58.50, 55.93, 53.47, 27.78, 22.54, 14.13.

### I. Synthesis of 1-(5-methylfuran-2-yl)-3-azabicyclo[3.1.0]hexane, hydrochloride

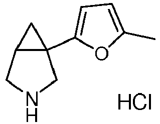

A cooled (3°C) stirred suspension of sodium amide (1.37g, 35 mmol) in anhydrous THF (40mL) under nitrogen was treated with a solution of 5-methylfuran-2-acetonitrile (1.82g, 15 mmol) in anhydrous THF (20mL), stirred 1.5h at room temperature, and recooled (3°C). Epichlorohydrin (1.53mL, 19.5 mmol) was added, and the mixture stirred at room temperature for 2h, then cooled on an ice bath and quenched with saturated aqueous ammonium chloride (15mL). After stirring for 15min, the mixture was taken up in ethyl acetate (150mL) and the organic layer separated. The aqueous solution was extracted with ethyl acetate (30mL) and the combined organic solution was dried (MgSO₄) and concentrated *in vacuo.* The residue was dissolved in methylene chloride and loaded onto a silica gel column (∼150cc) and eluted with methylene chloride, 5% ethyl acetate/methylene chloride, and 10% ethyl acetate/methylene chloride to afford the intermediate cyanocyclopropanemethanol (1.25g, 47%) as an amber oil, a 2:1 E/Z mixture of isomers (by NMR).

A cooled (-25°C) stirred solution of the intermediate cyanocyclopropanemethanol (1.24g, 7.00 mmol) in anhydrous THF (15mL) was carefully treated dropwise with 1.0N LAH/THF (14mL, 14 mmol), then stirred for 3h on an ice bath. The solution was cooled (-25°C) and carefully treated dropwise with water (0.53mL), 15% NaOH (0.53mL), and water (1.6mL), diluted with THF to facilitate stirring, stirred 15min, and filtered through Celite^{®} (filter cake rinsed with THF). The filtrate was concentrated *in vacuo* and the residue dissolved in methylene chloride and loaded onto a silica gel column (∼150cc). This was eluted with 9:1 methylene chloride/ethanol, 95:4.5:0.5 methylene chloride/ethanol/ammonium hydroxide, and 90:9:1 methylene chloride/ethanol/ammonium hydroxide to afford the intermediate aminomethylcyclopro panemethanol (453mg, 38%) as a pale amber oil. MS (M+1) 181.9. ¹H NMR (CDCl₃) δ 5.91 (m, 1H), 5.83 (m, 1H), 4.08 (dd, 1H, *J*=12Hz,5Hz), 3.67 (d, 1H, *J*=13Hz), 3.28 (dd, 1H, *J*=13Hz,11Hz), 2.48 (d, 1H, *J*=12Hz), 2.23 (s, 3H), 1.78 (m, 1H), 1.08 (dd, 1H, *J*=9Hz,5Hz), 0.68 (t, 1H, *J*=5Hz).

A cooled (3°C) stirred solution of the intermediate aminomethylcyclopropanemethanol (435mg, 2.4 mmol) in anhydrous DCE (15mL) was treated dropwise with thionyl chloride (0.22mL, 3.0 mmol). The solution was stirred 3h at room temperature, then concentrated *in vacuo,* taken up in water (15mL), stirred 15min, and basified (3mL of 5N NaOH). The dark aqueous mixture was extracted with methylene chloride (3X40mL) and the combined extracts washed with brine (50mL), dried (Na₂SO₄) and concentrated *in vacuo.* The residue was dissolved in methylene chloride, loaded onto a silica gel column (∼75cc), and eluted with 90:9:1 methylene chloride/ethanol/ammonium hydroxide to afford the bicyclic amine free base (158mg, 40%) as an amber glass. MS (M+1) 163.9.

A solution of the bicyclic amine free base (70mg, 0.429 mmol) in methylene chloride (5mL) was treated with 2N HCl/ether (0.25mL, 0.50 mmol), stirred a few minutes, then concentrated *in vacuo.* The residue was triturated from ether to afford 1-(5-methylfuran-2-yl)-3-azabicyclo[3.1.0]hexane, hydrochloride (65mg, 76%) as a brown solid. MS (M+1) 163.9. ¹H NMR (CDCl₃) δ 10.26 (br s, 1H), 9.75 (br s, 1H), 5.90 (m, 2H), 3.45-3.75 (m, 4H), 2.23 (s, 3H), 1.89 (m, 1H), 1.50 (m, 1H), 1.36 (m, 1H). ¹³C NMR (CDCl₃) δ 151.59, 149.26, 106.89, 106.28, 48.37, 47.26, 25.71, 23.46, 13.45, 13.35.

### J. Synthesis of 1-(benzofuran-3-yl)-3-azabicyclo[3.1.0]hexane, hydrochloride

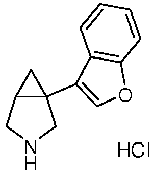

A cooled (3°C) stirred suspension of sodium amide (1.37g, 35 mmol) in anhydrous THF (45mL) under nitrogen was treated with a solution of benzofuran-3-acetonitrile (2.36g, 15 mmol) in anhydrous THF (15mL), stirred 1h at room temperature, and recooled (3°C). Epichlorohydrin (1.53mL, 19.5 mmol) was added, and the mixture stirred at room temperature for 1h, then cooled on an ice bath and quenched with saturated aqueous ammonium chloride (15mL). After stirring for 15min, the mixture was taken up in ethyl acetate (150mL) and the organic layer separated. The aqueous solution was extracted with ethyl acetate (2X20mL) and the combined organic solution was dried (MgSO₄) and concentrated *in vacuo.* The residue was dissolved in methylene chloride and loaded onto a silica gel column (∼150cc) and eluted with 5% ethyl acetate/methylene chloride, then 10% ethyl acetate/methylene chloride to afford the intermediate cyanocyclopropanemethanol (1.83g, 57%) as a pale amber oil, a 2:1 E/Z mixture of isomers (by NMR).

A cooled (-25°C) stirred solution of the intermediate cyanocyclopropanemethanol (1.80g, 8.44 mmol) in anhydrous THF (15mL) was carefully treated dropwise with 1.0N LAH/THF (14.8mL, 14.8 mmol), then stirred for 3h on an ice bath. The solution was carefully treated dropwise with water (0.60mL), 15% NaOH (0.60mL), and water (1.7mL), diluted with THF to facilitate stirring, stirred 15min, and filtered through Celite^{®} (filter cake rinsed with THF). The filtrate was concentrated *in vacuo* and the residue dissolved in methylene chloride and loaded onto a silica gel column (∼125cc). This was eluted with 90:9:1, then 85:13.5:1.5 methylene chloride/ethanol/ammonium hydroxide to afford the intermediate aminomethylcyclopropanemethanol (1.06g, 58%) as a viscous colorless oil. MS (M+1) 217.9. ¹H NMR (CDCl₃) δ 7.76 (m, 1H), 7.59 (s, 1H), 7.47 (m, 1H), 7.23-7.33 (m, 2H), 4.16 (dd, 1H, *J*=13Hz,5Hz), 3.57 (d, 1H, *J*=12Hz), 3.38 (dd, 1H, *J*=13Hz,11Hz), 2.59 (d, 1H, *J*=12Hz), 1.76 (m, 1H), 0.99 (dd, 1H, *J*=9Hz,5Hz), 0.80 (t, 1H, *J*=5Hz).

A cooled (3°C) stirred solution of the intermediate aminomethylcyclopropanemethanol (1.03g, 4.75 mmol) in anhydrous DCE (25mL) was treated dropwise with thionyl chloride (0.44mL, 6.0 mmol). The solution was stirred 3h at room temperature, then concentrated *in vacuo,* taken up in water (15mL), stirred 25min, and basified (6mL of 5N NaOH). The aqueous mixture was extracted with methylene chloride (4X50mL) and the combined extracts washed with brine (50mL), dried (Na₂SO₄) and concentrated *in vacuo.* The residue was dissolved in methylene chloride, loaded onto a silica gel column (∼150cc), and eluted with 95:4.5:0.5, then 90:9:1, then 85:13.5:1.5 methylene chloride/ethanol/ammonium hydroxide to afford the bicyclic amine free base (709mg, 75%) as a pale tan solid. MS (M+1) 180.0. ¹H NMR (CDCl₃) δ 7.55 (m, 1H), 7.46 (m, 1H), 7.44 (s, 1H), 7.20-7.32 (m, 2H), 3.15-3.26 (m, 3H), 3.09 (d, 1H, *J*=11 Hz), 1.76 (m, 1H), 1.05 (dd, 1H, *J*=8Hz,5Hz), 0.83 (t, 1H, *J*=5Hz).

A stirred solution of the above bicyclic amine free base (240mg, 1.205 mmol) in anhydrous ether (5mL) was treated with 2.0N HCl/ether (1.0mL, 2.0 mmol), stirred until a solid formed and for a few minutes more, filtered, and the solid rinsed with ether, collected, and dried *in vacuo* to afford 1-(benzofuran-3-yl)-3-azabicyclo[3.1.0]hexane, hydrochloride (266mg, 94%) as a white solid. MS (M+1) 199.9. ¹H NMR (CDCl₃) δ 10.35 (br s, 1H), 9.89 (br s, 1H), 7.53 (d, 1H, *J*=8Hz), 7.50 (s, 1H), 7.48 (d, 1H, *J*=8Hz), 7.23-7.35 (m, 2H), 3.60-3.80 (m, 4H), 2.01 (m, 1H), 1.62 (m, 1H), 1.32 (d, 1H, *J*=8Hz). ¹³C NMR (CDCl₃) δ 155.49, 142.93, 126.52, 124.93, 123.04, 119.43, 117.80, 111.98, 49.98, 47.56, 22.74, 21.54, 13.44.

### K. Synthesis of 1-(benzofuran-3-yl)-3-methyl-3-azabicyclo[3.1.0]hexane, hydrochloride

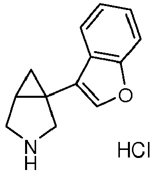

A stirred solution of 1-(benzofuran-3-yl)-3-azabicyclo[3.1.0]hexane (199mg, 1.00 mmol) in DCE (25mL) was treated with 37% aqueous formaldehyde (0.61mL, 8.0 mmol), then with sodium triacetoxyborohydride (850mg, 4.0 mmol), and stirred at room temperature for 3h. 1N Sodium hydroxide (15mL) was added, stirring was continued for a few minutes, and the organic layer was separated. The aqueous layer was extracted with methylene chloride (2X25mL) and the combined organic solution was washed with brine (50mL), dried (Na₂SO₄) and concentrated *in vacuo.* The residue was dissolved in methylene chloride, loaded onto a silica gel column (∼60cc), and eluted with 95:4.5:0.5 methylene chloride/ethanol/ammonium hydroxide to afford the bicyclic amine free base. This was dissolved in anhydrous ether (5mL) and treated with 2N HCl/ether (0.8mL, 1.6 mmol) and stirred until several minutes after a solid formed, then filtered, rinsed with ether, and the solid collected and dried *in vacuo* to afford 1-(benzofuran-3-yl)-3-methyl-3-azabicyclo[3.1.0]hexane, hydrochloride (226mg, 94%) as a white solid. MS (M+1) 213.9. ¹H NMR (CDCl₃) δ 12.64 (br s, 1H), 7.45-7.55 (m, 3H), 7.23-7.25 (m, 2H), 4.08 (m, 1H), 3.96 (m, 1H), 3.41 (m, 1H), 3.29 (m, 1H), 2.92 (br s, 3H), 2.23 (m, 1H), 2.06 (m, 1H), 1.27 (t, 1H, J=8Hz). ¹³C NMR (CDCl₃) δ 155.45, 143.01, 126.44, 125.03, 123.05, 119.11, 117.61, 112.09, 59.57, 57.09, 41.24, 22.65, 21.45, 13.87.

### L. Synthesis of 1-(5-methylthiophen-2-yl)-3-azabicyclo[3.1.0]hexane, hydrochloride

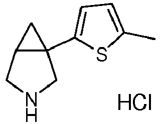

A cooled (3°C) stirred suspension of sodium amide (1.25g, 32 mmol) in anhydrous THF (35mL) under nitrogen was treated with a solution of 5-methylthiophen-2-acetonitrile^{1,2} (1.85g, 13.5 mmol) in anhydrous THF (15mL), stirred 1.5h at room temperature, and recooled (3°C). Epichlorohydrin (1.40mL, 17.8 mmol) was added, and the mixture stirred at room temperature for 2h, then cooled on an ice bath and quenched with saturated aqueous ammonium chloride (15mL). After stirring for 15min, the mixture was taken up in ethyl acetate (125mL) and the organic layer separated. The aqueous solution was extracted with ethyl acetate (25mL) and the combined organic solution was dried (MgSO₄) and concentrated *in vacuo.* The residue was dissolved in methylene chloride and loaded onto a silica gel column (∼150cc) and eluted with 10% ethyl acetate/methylene chloride, then 20% ethyl acetate/methylene chloride to afford the intermediate cyanocyclopropanemethanol (1.74g, 67%) as an amber oil, a 2:1 E/Z mixture of isomers (by NMR).

A cooled (-20°C) stirred solution of the intermediate cyanocyclopropanemethanol (1.64g, 8.50 mmol) in anhydrous THF (15mL) was carefully treated dropwise with 1.0N LAH/THF (17mL, 17 mmol), then stirred for 3h on an ice bath. The solution was cooled (-20°C) and carefully treated dropwise with water (0.65mL), 15% NaOH (0.65mL), and water (2.0mL), diluted with THF to facilitate stirring, stirred 15min, and filtered through Celite^{®} (filter cake rinsed with THF). The filtrate was concentrated *in vacuo* and the residue dissolved in methylene chloride and loaded onto a silica gel column (∼125cc). This was eluted with 90:9:1 methylene chloride/ethanol/- ammonium hydroxide to afford the intermediate aminomethylcyclopropanemethanol (584mg, 35%) as a pale yellow oil. MS (M+1) 198.1. ¹H NMR (CDCl₃) δ 6.73 (m, 1H), 6.54 (m, 1H), 4.09 (dd, 1H, *J*=12Hz,5Hz), 3.52 (d, 1H, *J*=13Hz), 3.29 (dd, 1H, *J*=13Hz,11Hz), 2.56 (d, 1H, *J*=12Hz), 2.42 (br s, 3H), 1.84 (m, 1H), 1.03 (dd, 1H, *J*=9Hz,5Hz), 0.77 (t, 1H, *J*=5Hz).

A cooled (3°C) stirred solution of the intermediate aminomethylcyclopropanemethanol (572mg, 2.90 mmol) in anhydrous DCE (15mL) was treated dropwise with thionyl chloride (0.27mL, 3.7 mmol). The solution was stirred 3h at room temperature, then concentrated *in vacuo,* taken up in water (15mL), stirred 15min, and basified (4mL of 5N NaOH). The aqueous mixture was extracted with methylene chloride (4X30mL) and the combined extracts washed with brine (50mL), dried (Na₂SO₄) and concentrated *in vacuo.* The residue was dissolved in methylene chloride, loaded onto a silica gel column (∼150cc), and eluted with 95:4.5:0.5, then 90:9:1 methylene chloride/ethanol/ammonium hydroxide to afford the bicyclic amine free base (308mg, 59%) as a brown oil. MS (M+1) 180.0. ¹H NMR (CDCl₃) δ 6.59 (m, 1H), 6.54 (m, 1H), 3.10-3.22 (m, 3H), 2.99 (d, 1H, *J*=12Hz), 2.41 (s, 3H), 1.61 (m, 1H), 1.04 (m, 1H), 0.88 (t, 1H, *J*=5 Hz).

A solution of the bicyclic amine free base (163mg, 0.909 mmol) in anhydrous ether (5mL) was treated with 2N HCl/ether (0.75mL, 1.50 mmol), stirred a few hours, the solid filtered, rinsed with ether, collected, and dried *in vacuo* to afford 1-(5-methylthiophen-2-yl)-3-azabicyclo[3.1.0]hexane, hydrochloride (174mg, 89%) as a pale gray solid. MS (M+1) 179.9. ¹H NMR (CDCl₃) δ 10.22 (br s, 1H), 9.74 (br s, 1H), 6.65 (m, 1H), 6.55 (m, 1H), 3.50-3.75 (m, 4H), 2.41 (s, 3H), 1.87 (m, 1H), 1.59 (m, 1H), 1.30 (m, 1H). ¹³C NMR (CDCl₃) δ 139.00, 138.98, 125.15, 125.04, 50.83, 47.40, 27.39, 24.89, 15.97, 15.26.

### M. Synthesis of 1-(5-methylthiophen-2-yl)-3-methyl-3-azabicyclo[3.1.0]hexane, hydrochloride

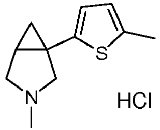

A stirred solution of 1-(5-methylthiophen-2-yl)-3-azabicyclo[3.1.0]hexane (126mg, 0.70 mmol) in DCE (15mL) was treated with 37% aqueous formaldehyde (0.43mL, 5.0 mmol), then with sodium triacetoxyborohydride (594mg, 2.8 mmol), and stirred at room temperature for 3h. 1N Sodium hydroxide (12mL) was added, stirring was continued for a few minutes, and the organic layer was separated. The aqueous layer was extracted with methylene chloride (2X15mL) and the combined organic solution was washed with brine (20mL), dried (Na₂SO₄) and concentrated *in vacuo.* The residue was dissolved in methylene chloride, loaded onto a silica gel column (∼50cc), and eluted with 95:4.5:0.5 methylene chloride/ethanol/ammonium hydroxide to afford the bicyclic amine free base. This was dissolved in anhydrous ether (5mL) and treated with 2N HCl/ether (0.6mL, 1.2 mmol) and stirred for 2h, then filtered, rinsed with ether, and the solid collected and dried *in vacuo* to afford 1-(5-methylthiophen-2-yl)-3-methyl-3-azabicyclo[3.1.0]hexane, hydrochloride (133mg, 82%) as a pale tan solid. MS(M+1)193.9. ¹H NMR (CDCl₃) δ 12.51 (br s, 1H), 6.65 (m, 1H), 6.55 (m, 1H), 4.02 (m, 1H), 3.85 (m, 1H), 3.18-3.36 (m, 2H), 2.87 (br s, 3H), 2.40 (s, 3H), 2.21 (m, 1H), 1.91 (m, 1H), 1.28 (m, 1H). ¹³C NMR (CDCl₃) δ 139.22, 138.64, 125.35, 125.09, 60.54, 57.06, 41.31, 27.43, 24.91, 16.54, 15.25.

### N. Synthesis of 1-(benzothiazol-2-yl)-3-azabicyclo[3.1.0]hexane, hydrochloride

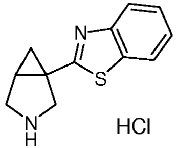

An ice-cooled (3°C) stirred suspension of sodium amide (1.80g, 46 mmol) in anhydrous THF (50mL) under nitrogen was treated with a solution of benzothiazole-2-acetonitrile (3.49g, 20 mmol) in anhydrous THF (20mL), then stirred at room temperature for 2.5h. The mixture was recooled (3°C), treated in one portion with epichlorohydrin (2.1mL, 26.9 mmol), and stirred at room temperature for 20h, then cooled on an ice bath and quenched with saturated aqueous ammonium chloride (20mL). The mixture was extracted with ethyl acetate (200mL, then 50mL), and the combined extracts were dried (MgSO₄) and concentrated *in vacuo.* The residue was dissolved in methylene chloride, loaded onto a silica gel column (∼250cm), and eluted with 4:1 methylene chloride/ethyl acetate to afford the intermediate cyanocyclopropanemethanol (1.00g, 22%) as a pale tan foam, a somewhat still crude mixture of isomers (by NMR). MS (M+1) 231.7.

A cooled (-25°C) stirred solution of the intermediate cyanocyclopropanemethanol (1.00g, 4.34 mmol) in anhydrous THF (7mL) under nitrogen was treated dropwise with 1N LAH/THF (6.5mL, 6.5 mmol), stirred at 3°C for 2h, recooled (-25°C), and carefully quenched dropwise with water (0.25mL), 15% NaOH ().25mL), and water (0.75mL). Extra THF was added to facilitate stirring, and after a few minutes the mixture was filtered through Celite^{®} (filter cake rinsed with THF). The filtrate was concentrated *in vacuo,* and the residue dissolved in methylene chloride and loaded onto a silica gel column (∼125cc) and eluted with 90:9:1, then 85:13.5:1.5 methylene chloride/ethanol/ammonium hydroxide to afford the intermediate aminomethylcyclopropanemethanol (137mg, 13%) as an orange viscous oil. MS (M+1) 234.7. ¹H NMR (CDCl₃) δ 7.88 (d, 1H, *J*=8Hz), 7.77 (d, 1H, *J*=8Hz), 7.41 (t, 1H, *J*=8Hz), 7.30 (t, 1H, *J*=8Hz), 4.10-4.18 (m, 2H), 3.37 (t, 1H, *J*=12Hz), 2.58 (d, 1H, *J*=12Hz), 2.02 (m, 1H), 1.43 (dd, 1H, *J*=9Hz,5Hz), 1.12 (t, 1H, *J*=6Hz).

An ice-cooled (3°C) stirred solution of the intermediate aminomethylcyclopropanemethanol (137mg, 0.585 mmol) in anhydrous DCE (3mL) under nitrogen was treated with thionyl chloride (87mg, 0.73 mmol) and stirred at room temperature for 3h, then concentrated *in vacuo.* Water (2mL) was added, stirring continued for 15min, then the solution was basified with 5N sodium hydroxide (1mL) and extracted with 5:1 methylene chloride/isopropanol (3X15mL). The combined extracts were dried (MgSO₄), concentrated *in vacuo,* and the residue dissolved in methylene chloride and loaded onto a silica gel column (∼50cc). This was eluted with 90:9:1, then 85:13.5:1.5 methylene chloride/ethanol/ammonium hydroxide to afford 1-(benzothiazol-2-yl)-3-azabicyclo[3.1.0]hexane (88mg, 70%) as an amber solid. This was dissolved in ether containing a little methanol (5mL), treated with 2.0N HCl/ether (0.40mL, 0.80 mmol), the solvent evacuated from the flask, and the residual solid triturated from ether to afford 1-(benzothiazol-2-yl)-3-azabicyclo[3.1.0]hexane, hydrochloride (99mg, 96% from free base) as a pale brick-colored powder. MS (M+1) 216.8. ¹H NMR (DMSO-d₆) δ 10.20 (br s, 1H), 9.95 (br s, 1H), 8.05 (d, 1H, *J*=8Hz), 7.91 (d, 1H, *J*=8Hz), 7.48 (t, 1H, *J*=8Hz), 7.39 (t, 1H, *J*=8Hz), 3.88 (m, 1H), 3.73 (m, 1H), 3.50 (m, 1H), 3.38 (m, 1H), 2.35 (m, 1H), 1.88 (t, 1H, *J*=6Hz), 1.59 (m, 1H). ¹³C NMR (DMSO-d₆) δ 170.19, 152.52, 133.73, 126.46, 125.09, 122.20, 121.98, 47.52, 46.39, 31.07, 28.61, 18.23.

### O. Synthesis of 1-(5-chlorothiouhen-3-yl)-3-azabicyclo[3.1.0]hexane, hydrochloride

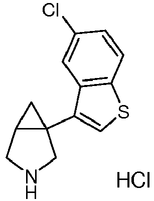

A cooled (3°C) stirred suspension of sodium amide (0.90g, 23 mmol) in anhydrous THF (30mL) under nitrogen was treated with a solution/suspension of 5-chlorobenzothiophen-3-acetonitrile (2.08g, 10 mmol) in anhydrous THF (10mL), stirred 1h at room temperature, and recooled (3°C). Epichlorohydrin (1.04mL, 12 mmol) was added, and the mixture stirred at room temperature for 4h, then cooled on an ice bath and quenched with saturated aqueous ammonium chloride (10mL). After stirring for 15min, the mixture was taken up in ethyl acetate (100mL) and the organic layer separated. The aqueous solution was extracted with ethyl acetate (50mL) and the combined organic solution was dried (MgSO₄) and concentrated *in vacuo.* The residue was dissolved in methylene chloride and loaded onto a silica gel column (∼150cc) and eluted with 10% ethyl acetate/methylene chloride to afford the intermediate cyanocyclopropane- methanol (1.51g, 57%) as a tan solid, a 4:1 E/Z mixture of isomers (by NMR).

A ice-cooled (3°C) stirred solution of 1.0N LAH/THF (5.25mL, 5.25 mmol) under nitrogen was treated dropwise with a solution of the intermediate cyanocyclopropanemethanol (932mg, 3.5 mmol) in anhydrous THF (5mL), and the mixture was stirred 2h at 3°C. Water (0.2mL), 15% NaOH (0.2mL), and water (0.6mL) were carefully added dropwise, and the mixture was diluted with THF and stirred 15 min at room temperature, filtered through Celite^{®} (filter cake rinsed with THF), and the filtrate concentrated *in vacuo.* The residue was dissolved in methylene chloride, loaded onto a silica gel column (∼60cc), and eluted with 10% EtOH/CH₂Cl₂, then 90:9:1 methylene chloride/ethanol/ammonium hydroxide to afford the intermediate aminomethylcyclopropanemethanol (650mg, 69%) as a white solid. MS (M+1) 267.7. ¹H NMR (CDCl₃) δ 7.94 (d, 1H, *J*=2Hz), 7.76 (d, 1H, *J*=9Hz), 7.46 (s, 1H), 7.32 (dd, 1H, *J*=9Hz,2Hz), 4.18 (dd, 1H, *J*=12Hz,5.5Hz), 3.54 (d, 1H, *J*=12Hz), 3.39 (t, 1H, *J*=11Hz), 2.60 (d, 1H, *J*=12Hz), 1.80 (m, 1H), 1.01 (dd, 1H, *J*=9Hz,5Hz), 0.89 (t, 1H, *J*=5Hz).

A cooled (3°C) stirred solution of the intermediate aminomethylcyclopropanemethanol (0.65g, 2.43 mmol) in anhydrous DCE (12mL) was treated dropwise with thionyl chloride (0.20mL, 2.70 mmol). The solution was stirred 3h at room temperature, then concentrated *in vacuo,* taken up in water (10mL), stirred 15min, and basified (3mL of 5N NaOH). The aqueous mixture was extracted with methylene chloride (4X25mL) and the combined extracts washed with brine (30mL), dried (Na₂SO₄) and concentrated *in vacuo.* The residue was dissolved in methylene chloride, loaded onto a silica gel column (∼75cc), and eluted with 95:4.5:0.5, then 90:9:1 methylene chloride/ethanol/ammonium hydroxide to afford the bicyclic amine free base (435mg, 72%) as a white solid. MS (M+1) 249.7. ¹H NMR (CDCl₃) δ 7.79 (d, 1H, *J*=2Hz), 7.75 (d, 1H, *J*=9Hz), 7.30 (dd, 1H, *J*=9Hz,2Hz), 7.27 (s, 1H), 3.33 (dd, 1H, *J*=12Hz,2.5Hz), 3.24 (d, 1H, *J*=11Hz), 3.14 (d, 1H, *J*=12Hz), 3.09 (d, 1H, *J*=12Hz), 1.71 (m, 1H), 0.96 (m, 1H), 0.89 (t, 1H, *J*=5Hz).

A stirred solution of the above bicyclic amine free base (150mg, 0.60 mmol) in anhydrous ether (6mL) was treated with 2.0N HCl/ether (0.5mL, 1.0 mmol), stirred for 2h, filtered, and the solid rinsed with ether, collected, and dried *in vacuo* to afford 1-(5-chlorothiophen-3-yl)-3-azabicyclo[3.1.0]hexane, hydrochloride (166mg, 97%) as a white solid. MS (M+1) 249.7. ¹H NMR (CDCl₃) δ 10.23 (br s, 1H), 9.83 (br s, 1H), 7.69 (d, 1H, *J*=9Hz), 7.67 (d, 1H, *J*=2Hz), 7.33 (s, 1H), 7.27 (dd, 1H, *J*=9Hz,2Hz), 3.74 (m, 2H), 3.65 (m, 1H), 3.45 (m, 1H), 1.97 (m, 1H), 1.61 (m, 1H), 1.17 (m, 1H). ¹³C NMR (CDCl₃) δ 139.41, 138.71, 132.01, 131.09, 127.97, 125.36, 124.22, 121.37, 50.52, 47.64, 26.29, 21.50, 13.68.

### P. Synthesis of 1-(5-chlorothiophen-3-yl)-3-methyl-3-azabicyclo[3.1.0]hexane, hydrochloride

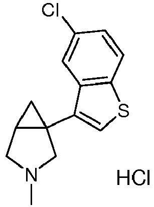

A stirred solution of 1-(5-chlorothiophen-3-yl)-3-azabicyclo[3.1.0]hexane (150mg, 0.60 mmol) in DCE (20mL) was treated with 37% aqueous formaldehyde (0.37mL, 4.8 mmol), then with sodium triacetoxyborohydride (510mg, 2.4 mmol), and stirred at room temperature for 3h. 1N Sodium hydroxide (8mL) was added, stirring was continued for 15min, and the organic layer was separated. The aqueous layer was extracted with methylene chloride (2X25mL) and the combined organic solution was washed with brine (50mL), dried (Na₂SO₄) and concentrated *in vacuo.* The residue was dissolved in methylene chloride, loaded onto a silica gel column (∼75cc), and eluted with 95:4.5:0.5 methylene chloride/ethanol/ammonium hydroxide to afford the bicyclic amine free base. This was dissolved in anhydrous ether (5mL) and treated with 2N HCl/ether (0.5mL, 1.0 mmol) and stirred for 30min, then filtered, rinsed with ether, and the solid collected and dried *in vacuo* to afford 1-(5-chlorothiophen-3-yl)-3-methyl-3-azabicyclo[3.1.0]hexane, hydrochloride (153mg, 85%) as a white solid. MS (M+1) 263.8. ¹H NMR (CDCl₃) δ 12.68 (br s, 1H), 7.77 (d, 1H, *J*=9Hz), 7.70 (d, 1H, *J*=2Hz), 7.44 (s, 1H), 7.34 (dd, 1H, *J*=9Hz,2Hz), 4.10 (dd, 1H, *J*=11Hz,5Hz), 4.02 (dd, 1H, *J*=11Hz,5Hz), 3.50 (m, 1H), 3.20 (t, 1H, *J*=10Hz), 2.94 (br s, 3H), 2.30 (m, 1H), 2.08 (m, 1H), 1.21 (t, 1H, *J*=8Hz). ¹³C NMR (CDCl₃) δ 139.22, 138.75, 131.64, 131.12, 128.30, 125.41, 124.38, 121.05, 59.88, 57.15, 41.26, 26.24, 21.49, 13.96.

### Example II

### Preparation of 1-heteroaryl-3-methyl-3-azabicyclo[3.1.0]hexanes using Reaction Scheme 2

### A. Synthesis of 3-methyl-1-(1-methylindol-2-yl)-3-azabicyclo[3.1.0]bicyclohexane, hydrochloride

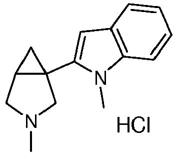

A stirred solution of N-methylbromomaleimide (1.9g 10 mmol) and 1-methyl-2-(tributhyltin)indole (5.25g, 12.5 mmol) in anhydrous dioxane (60mL) was degassed under a stream of nitrogen for 10 minutes, then treated with cesium fluoride (3.6g, 23.6 mmol) and Cl₂Pd(PPh₃)₂ (0.4g 0.6 mmol), stirred at room temperature for 0.5h, then at 40°C for 1h and at 50°C for 2h. Then the reaction mixture was cooled to room temperature, methylene chloride (100ml) added, and the mixture was filtered through celite, concentrated and purified by silica gel chromatography (2% ethyl acetate/methylene chloride) to afford maleimide intermediate (1.2g, 98%). ¹H NMR (CDCl₃) δ 7.72-7.68 (m, 1H), 7.62-7.61 (m, 1H), 7.36-7.33 (m, 2H), 7.17-7.12 (m, 1H) 6.59-6.57 (m, 1H), 3.89 (s, 3H) 3.11 (s, 3H).

A cooled (-20°C) stirred solution of trimethylsulfoxonium chloride (0. 892g, 6.96 mmol) in anhydrous tetrahydrofuran (30mL) under nitrogen was treated with n-butyllithium/hexane (2.5N, 2.2mL, 5.5 mmol) and warmed to 50°C over 30 minutes. A solution of the above maleimide intermediate (1.2g, 5 mmol) in anhydrous tetrahydrofuran (25mL) was warmed to 50°C and added all at once. After 2h at 50°C, the mixture was cooled on ice and quenched with saturated aqueous ammonium chloride (2mL). The mixture was stirred at room temperature for 15 minutes, then it was added to methylene chloride (100mL) and the organic solution was dried (Na₂SO₄) and concentrated *in vacuo.* The residue was dissolved in methylene chloride, loaded onto a silica gel column and eluted with 2% ethyl acetate/methylene chloride to afford diimide intermediate (140mg, 12%). ¹H NMR (CDCl₃ δ 7.59-7.54 (m, 1H), 7.35-7.30 (m, 1H), 7.28-7.23 (m, 1H), 7.14-7.08 (m, 1H), 6.46-6.43 (m, 1H), 3.82 (s, 3H), 2.96 (s, 3H), 2.83-2.78 (m, 1H), 1.99-1.96 (m, 1H) 1.92-1.87 (m, 1H).

A stirred, ice-cooled (2°C) solution of 1N lithium aluminum hydride /THF (3.43mL, 3.43 mmol) under nitrogen was treated dropwise with a solution of the above diimide intermediate (140mg, 0.55 mmol) in anhydrous tetrahydrofuran (20mL), stirred for 1h at room temperature and 7h at reflux, then cooled on an ice bath. Water (0.30mL), 15% NaOH (0.30mL) and water (0.90mL) were added dropwise together with more tetrahydrofuran to facilitate stirring. The mixture was stirred at room temperature for 15 minutes, filtered through celite, and the filtrate concentrated *in vacuo.* The residue was dissolved in methylene chloride, loaded onto a silica gel column, and eluted with 3:1 methylene chloride/ethyl acetate to afford 3-methyl-1-(1-methylindol-2-yl)-3-azabicyclo[3.1.0]bicyclohexane (100mg, 80%), which was then converted to the hydrochloride salt by treating with 2N HCl/ether(82mg, 80%). MS (M+1) 226.8 ¹H NMR (DMSO-*d*₆) δ 11.35- 11.12 (br s, 1H), 7.52-7.34 (m, 2H), 7.15-6.93 (m, 2H), 6.45-6.34 (m, 1H), 3.92-3.84 (m, 1H), 3.79-3.75 (s, 3H), 3.71-3.64 (m, 1H), 3.63-3.56 (m, 1H), 3.39-3.26 (m, 1H), 2.81 (d, 3H), 2.24- 2.16 (m, 1H), 1.91-1.83 (m, 1H), 1.11-1.01 (m, 1H). ¹³C NMR (DMSO-*d*₆) δ 138.06, 137.98, 127.38, 122.00, 14.21, 120.67, 119.98, 110.28, 102.01, 59.38, 56.47, 40.36, 30.96, 24.26, 22.51.

### B. Synthesis of 3-methyl-1-(1-methylindol-5-yl)-3-azabicyclo[3.1.0]bicyclohexane, hydrochloride

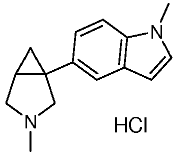

A stirred solution of N-methylbromomaleimide (1.8g, 9.5 mmol) and 1-methylindole-5-boronic acid (2g, 11.4 mmol) in dioxane (50mL) was degassed under a stream of nitrogen for 10 minutes, then treated with cesium fluoride (3.8g, 24.7 mmol) and Cl₂Pd(dppf)•CH₂Cl₂ (0.475g, 0.58 mmol), then stirred at room temperature for 1 hour and at 40°C for 1h. The mixture was cooled and diluted with methylene chloride, stirred a few minutes and filtered through celite. The filtrate was concentrated *in vacuo* and purified by silica gel chromatography using 2% ethyl acetate/ methylene chloride as an eluting solvent to afford maleimide intermediate (2.17g, 95%). ¹H NMR (CDCl₃) δ 8.43 (d, 1H, *J*=1.5Hz), 7.74 (dd, 1H, *J*=8.5Hz,1.5 Hz), 7.37 (d, 1H, *J*=9Hz), 6.66 (s, 1 H), 6.60 (d, 1H, *J*=3Hz), 3.83 (s, 3H), 3.10 (s, 3H).

A cooled (-20°C) stirred solution of trimethylsulfoxonium chloride (1.4g, 11.25 mmol) in anhydrous tetrahydrofuran (40 mL) under nitrogen was treated with n-butyllithium/hexane (2.5N, 3.6mL, 9 mmol) and warmed to 50°C over 30 minutes. A solution of the above maleimide intermediate (2g, 8.3 mmol) in anhydrous tetrahydrofuran (40mL) was warmed to 50°C and added all at once. After 2h at 50°C, the mixture was cooled on ice and quenched with saturated aqueous ammonium chloride. The mixture was stirred at room temperature for 15 minutes, then it was added to methylene chloride (150mL) and the organic solution was dried (Na₂SO₄) and concentrated *in vacuo.* The residue was purified by silica gel chromatography, using ethyl acetate/methylene chloride to afford bicyclic diimide intermediate (500mg, 25%). ¹H NMR (CDCl₃) δ 7.61-7.65 (m, 1 H), 7.30-7.34 (m, 1H), 7.23-7.26 (m, 1H), 7.07 (d, 1H, *J*=3Hz), 6.46 (dd, 1H, *J*=3Hz,1Hz,), 3.79 (s, 1H), 2.94 (s, 3H), 1.90 (dd, 1H, *J*=8Hz,4.5Hz), 1.84-1.87 (m, 1H).

A stirred, ice-cooled (2°C) solution of 1N lithium aluminum hydride /THF (12mL, 12mmol) under nitrogen was treated dropwise with a solution of the above bicyclic diimide intermediate (0.5g, 1.96mmol) in anhydrous tetrahydrofuran (10mL), stirred for 1h at room temperature and 2h at reflux, then cooled on an ice bath. Water (0.4mL), 15% NaOH (0.4mL), and water (1.2mL) were added dropwise together with more tetrahydrofuran to facilitate stirring. The mixture was stirred at room temperature for 15 minutes, filtered through celite, and the filtrate concentrated *in vacuo.* The residue was purified by silica gel chromatography, using methylene chloride/methanol as an eluting solvent to afford 3-methyl-1-(1-methylindol-5-yl)-3-azabicyclo[3.1.0]bicyclo-hexane, hydrochloride (0.262g, 60%), which was converted to the hydrochloride salt by treating with 2N HCl/ether (270mg, 90%). MS (M+1) 227. ¹H NMR (CDCl₃) δ 12.21 (br s, 1H), 7.45 (s, 1H), 7.22- 7.28 (m, 1H), 7.02-7.07 (m, 2H), 6.40 (d, 1H, *J*=2.5Hz), 4.05 (dd, 1H, *J*=10.5Hz,5Hz), 3.88 (dd, 1H, *J*=10Hz,4.5 Hz), 3.74 (s, 3H), 3.62-3.71 (m, 1H), 3.32-3.40 (m, 1H), 3.27 (t, 1H, *J*=9.5Hz), 2.87 (d, 3H, *J*=4Hz), 2.06-2.11 (m, 1H), 1.93-1.99 (m, 1H). ¹³C NMR (CDCl₃) δ 136.19, 130.17, 128.82 , 128.50 , 121.34 , 120.10 , 109.91 , 100.99 , 61.73 , 57.75, 41.43, 33.17, 32.18, 22.95, 15.68.

### C. Synthesis of 1-(quinolin-5-yl)-3-methyl-3-azabicyclo[3.1.0]hexane, hydrochloride

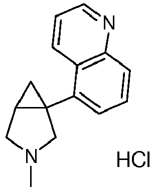

A stirred solution/suspension of N-methylbromomaleimide (0.78 g, 4.1 mmol) and quinoline-5-boronic acid (1.1g, 2.6 mmol) in dioxane (15mL) under nitrogen was degassed with a stream of nitrogen for 10 min, treated with cesium fluoride (1.6g, 10.8 mmol) and Cl₂Pd(dppf).CH₂Cl₂ (0.25g, 0.3 mmol), then stirred at room temperature for 0.5h and at 45°C for 30min then at 65°C for 45min. The mixture was cooled and diluted with methylene chloride (50mL). The mixture was filtered through Celite^{®} (rinse filter cake with methylene chloride) and the brown filtrate concentrated *in vacuo.* The residue was dissolved in methylene chloride and filtered through a column of silica gel (eluted with methylene chloride 60% and ethyl acetate 40%) to afford a yellowish solid, which was triturated from cold petroleum ethers to afford the arylmaleimide intermediate (0.48 g, 50%) as a pale yellow solid.

A cooled (-20°C) stirred solution of trimethylsulfoxonium chloride (290mg, 2.2 mmol) in anhydrous tetrahydrofuran (15mL) under nitrogen was treated dropwise with n-butyllithium/hexane (2.4N, 1.1mL, 2.03 mmol) and gradually warmed to 50°C over 30 minutes. Meanwhile, a solution of the intermediate arylmaleimide (0.690g, 2.6 mmol) in anhydrous THF (10mL) was heated to 50°C, then added quickly in one portion to the above heated suspension, and the mixture was stirred at 50°C for 2h, then cooled on an ice bath. Saturated aqueous ammonium chloride (1mL) was added to quench, and the mixture was diluted with methylene chloride (75mL), dried (MgSO₄), filtered through Celite^{®} (rinse with methylene chloride), and concentrated *in vacuo.* The residue was dissolved in methylene chloride, loaded onto a silica gel column, and the product eluted with 3% ethyl acetate/methylene chloride to afford the intermediate bicyclic diimide (120mg, 24%) as a very pale yellow viscous oil.

A stirred ice-cooled solution of 1.0N borane/THF (10.5mL, 10.5 mmol) under nitrogen was treated dropwise with a solution of the above bicyclic diimide intermediate (120mg, 0.48 mmol) in anhydrous THF (10mL). The solution was stirred at room temperature for 15min, refluxed for 4h, cooled on an ice bath, and carefully treated dropwise with 6N HCl (10mL, vigorous evolution of gas). The solution was concentrated to a white solid, which was partitioned between 5N sodium hydroxide (25mL) and ether (50mL). The organic layer was separated and the aqueous extracted with ether (50mL). The combined organic solution was washed with water (25mL), dried (Mg₂SO₄), and concentrated *in vacuo.* The residue was dissolved in methanol (23mL), treated with 4N HCl/dioxane (7mL), then stirred at room temperature for 16h and at 55°C for 4h. The solution was concentrated *in vacuo.* and the residue triturated from ether to afford 1-(quinolin-5-yl)-3-methyl-3-azabicyclo[3.1.0]hexane, hydrochloride (50mg, 20%) as a white solid. MS (M+1) 225.1. ¹H NMR (CDCl₃) δ 7.23 (dd, 1H, *J*=6.5Hz,2Hz), 7.01-7.15 (m, 2H), 4.10 (dd, 1H, *J*=11Hz,5Hz), 3.92 (dd, 1H, *J*=11Hz,5Hz), 2.95-3.30 (m, 4H), 2.36 (dd, 1H, *J*=6.5Hz,4.5Hz), 1.93-2.02 (m, 1H), 1.51 (t, 3H, *J*=7Hz), 1.13-1.20 (m, 1H). ¹³C NMR (DMSO-*d*₆) δ 146.98, 141.45, 140.98, 137.01, 133.53, 131.50, 128.73, 127.46, 123.07, 61.39, 60.37, 56.60, 29.59, 22.58, 14.35.

### D. Synthesis of 1-(quinolin-3-yl)-3-methyl-3-azabicyclo[3.1.0]hexane, hydrochloride

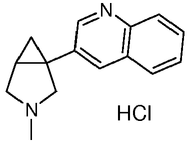

A stirred solution/suspension of 3-bromo-1-methylmaleimide (2.0g, 10.5 mmol) and quinoline-3-boronic acid (2.2g, 12.6 mmol) in dioxane (50mL) under nitrogen was degassed with a stream of nitrogen for 10 min, treated with cesium fluoride (3.5g, 22.0 mmol) and Cl₂Pd(dppf).CH₂Cl₂ (0.5g, 0.6 mmol), then stirred at room temperature for 0.5h, at 45°C for 30min, and at 65°C for 45min. The mixture was cooled and diluted with methylene chloride (50mL). The mixture was filtered through Celite^{®} (rinse filter cake with methylene chloride) and the brown filtrate concentrated *in vacuo.* The residue was dissolved in methylene chloride and filtered through a column of silica gel (eluted with methylene chloride 60% and ethyl acetate 40%) to afford a tan solid, which was triturated from cold petroleum ethers to afford arylmaleimide intermediate (880 mg, 15%) as a tan solid.

A cooled (-20°C) stirred solution of trimethylsulfoxonium chloride (261mg, 2.03 mmol) in anhydrous tetrahydrofuran (15mL) under nitrogen was treated dropwise with n-butyllithium/hexane (2.4N, 1.1mL, 2.03 mmol) and gradually warmed to 50°C over 30 minutes. Meanwhile, a solution of the intermediate arylmaleimide (0.350g, 1.5 mmol) in anhydrous THF (10mL) was heated to 50°C, then added quickly in one portion to the above heated suspension, and the mixture was stirred at 50°C for 2h, then cooled on an ice bath. Saturated aqueous ammonium chloride (1mL) was added to quench, and the mixture was diluted with methylene chloride (75mL), dried (MgSO₄), filtered through Celite^{®} (rinse with methylene chloride), and concentrated *in vacuo.* The residue was dissolved in methylene chloride, loaded onto a silica gel column, and the product eluted with 3% ethyl acetate/methylene chloride to afford the intermediate bicyclic diimide (148mg, 50%) as a very pale yellow viscous oil. ¹H NMR (CDCl₃ δ 7.74- 7.90 (m, 3H), 7.38-7.55 (m, 3H), 4.54 (dd, 2H), 3.86 (d, 6H, *J*=4.5 Hz), 2.81 (dd, 1H, *J*=8Hz, 3.5Hz), 1.91 (dd, 1H, *J*=8Hz,4.5Hz), 1.78 (dd, 1H, *J*=4.5, 3.5Hz).

A stirred ice-cooled solution of 1.0N borane/THF (10.5mL, 10.5 mmol) under nitrogen was treated dropwise with a solution of the above bicyclic diimide intermediate (560mg, 2.1mmol) in anhydrous THF (10mL). The solution was stirred at room temperature for 15min, refluxed for 4h, cooled on an ice bath, and carefully treated dropwise with 6N HCl (10mL, vigorous evolution of gas). The solution was concentrated to a white solid, which was partitioned between 5N sodium hydroxide (25mL) and ether (50mL). The organic layer was separated and the aqueous extracted with ether (50mL). The combined organic solution was washed with water (25mL), dried (Mg₂SO₄), and concentrated *in vacuo.* The residue was dissolved in methanol (23mL), treated with 4N HCl/dioxane (7mL), then stirred at room temperature for 16h and at 55°C for 4h. The solution was concentrated *in vacuo* and the residue triturated from ether to afford 1-(quinolin-3-yl)-3-methyl-3-azabicyclo[3.1.0]hexane, hydrochloride (100mg, 20%) as a white solid. MS (M+1) 240.1. ¹H NMR (CDCl₃) δ 7.23 (dd, 1H, *J*=6.5Hz,2Hz), 7.01-7.15 (m, 2H), 4.10 (dd, 1H, *J*=11Hz,5Hz), 3.92 (dd, 1H, *J*=11Hz,5Hz), 2.95-3.30 (m, 4H), 2.36 (dd, 1H, *J*=6.5Hz,4.5Hz), 1.93-2.02 (m, 1H), 1.51 (t, 3H, *J*=7Hz,3Hz), 1.13-1.20 (m, 1H). ¹³C NMR (DMSO-*d*₆) δ 146.98, 141.45, 140.98, 137.01, 133.53, 131.50, 128.73, 127.46, 123.07, 61.39, 60.37, 56.60, 29.59, 22.58, 14.35.

### Example III

### Preparation of 1-heteroaryl-3-ethyl-3-azabicyclo[3.1.0]hexanes using Reaction Scheme 2

### A. Synthesis of 3-bromo-1-ethylmaleimide

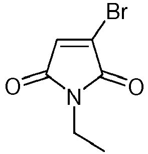

A cooled (5°C) solution of N-ethylmaleimide (20g, 0.16mole) in carbon tetrachloride (20mL) under nitrogen was treated dropwise over 45 min with bromine (23g, 0.14mole) at a rate to keep the pot temp <10°C. The mixture was stirred at 5°C for 2 hours. Dichloromethane (20mL) was added to the reaction and N₂ was bubbled through the reaction for 15min to remove excess bromine. The reaction was blown dry with a steady stream of N₂ and then brought up in ethanol. Anhydrous sodium acetate (12.3g, 0.15mole) was added and the reaction was refluxed for 4 hours. The mixture was concentrated *in vacuo* and the residue taken up in methylene chloride (300mL), filtered and concentrated *in vacuo* to yield an orange oil. Pure 3-bromo-1-ethylmaleimide was obtained from recrystallization in chloroform to yield a yellowish solid (26g, 82%). ¹H NMR (CDCl₃) δ 1.20 (t, *J*=7.22 Hz, 3 H) 3.62 (q, *J*=7.22 Hz, 2 H) 6.85 (s, 1 H).

### B. Synthesis of 1-(quinolin-3-yl)-3-ethyl-3-azabicyclo[3.1.0]hexane, hydrochloride

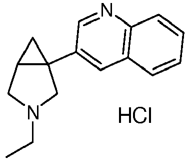

A stirred solution/suspension of 3-bromo-1-ethylmaleimide (2.4 g, 5.9mmol) and 3-quinolineboronic acid (2.2g, 6.5mmol) in dioxane (15mL) under nitrogen was degassed with a stream of nitrogen for 10 min, treated with cesium fluoride (3.2g, 10.8mmol) and POPd (Combiphos, cat# AC1000) (0.500g, 0.5mmol), then stirred at room temperature for 0.5h and at 45°C for 30min then at 65°C for 45min. The mixture was cooled and diluted with methylene chloride (50mL). The mixture was filtered through Celite^{®} (rinse filter cake with methylene chloride) and the brown filtrate concentrated *in vacuo.* The residue was dissolved in methylene chloride and filtered through a column of silica gel (eluted with methylene chloride 60% and ethyl acetate 40%) to afford a light gray solid, which was triturated from cold petroleum ethers to afford the arylmaleimide intermediate (300 mg, 10%) as a gray solid.

A stirred solution of sodium hydride oil dispersion (60%, 140mg, 3.5mmol) in anhydrous tetrahydrofuran (30mL) under nitrogen was treated with trimethyl-sulfoxonium chloride (0.55g, 4.25mmol), then refluxed for 2.5h and cooled (50°C). The above arylmaleimide (937mg, 3.5mmol) was added in one portion and the mixture stirred at 50°C for 3h, cooled on an ice bath, and quenched with saturated ammonium chloride (10mL). The product mixture was extracted with ether (2X50mL), and the combined extracts washed with water (30mL), dried (MgSO₄), and concentrated *in vacuo.* The residual solid was dissolved in 1:1 methylene chloude/heptane and loaded onto a silica gel column and eluted with 1:1, 2:1, then 3:1 methylene chloride/heptane to afford the bicyclic diimide intermediate (250mg, 64%) as a very pale yellow oil. ¹H NMR (CDCl₃) δ 1.18 (t, 3 H) 1.89 - 1.96 (m, 2 H) 2.91 (dd, *J*=6.44, 5.47 Hz, 1 H) 3.46 - 3.59 (m, 2 H) 7.55 - 7.65 (m, 1 H) 7.69 - 7.81 (m, 1 H) 7.84 (d, *J*=8.20 Hz, 1 H) 8.16 (d, *J*=8.40 Hz, 1 H) 8.30 (d, *J*=1.76 Hz, 1 H) 8.92 (d, *J*=2.34 Hz, 1 H).

A stirred ice-cooled solution of 2.0N LAH/THF (1.9mL, 3.76mmol) under nitrogen was treated dropwise with a solution of the above bicyclic diimide intermediate (250mg, 0.94mmol) in anhydrous THF (10mL). The solution was stirred at room temperature for 15min, refluxed for 4h, cooled on an ice bath, and carefully treated dropwise with 30% NaOH (1.3mL, vigorous evolution of gas). The solution was filtered and the filtrate was reduced to a clear oil. The residue was dissolved in methanol (23mL), treated with 4N HCl/dioxane (7mL), then stirred at room temperature for 3h. The solution was concentrated *in vacuo* and the residue triturated from ether to afford 1-(quinolin-3-yl)-3-ethyl-3-azabicyclo[3.1.0]hexane, hydrochloride (105mg, 43%) as a white solid. MS (M+1) 239. ¹H NMR (CDCl₃) δ 7.25 (m, 1H), 7.08 (m, 2H), 4.04 (m, 1H), 3.85 (m, 1H), 3.35 (m, 2H), 3.21 (m, 1H), 2.39 (m, 1H), 1.97 (m, 1H), 1.50 (d, 6H, J=7Hz), 1.10 (m, 1H). ¹³C NMR (CDCl₃) δ 158.83, 156.34, 135.62, 129.93, 127.57, 121.54, 117.17, 59.78, 57.35, 53.99, 30.68, 23.06, 19.05, 16.29.

### C. Synthesis of 1-(1-methyl-1H-indol-5-yl)-3-ethyl-3-azabicyclo[3.1.0]hexane, hydrochloride

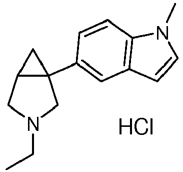

A stirred solution/suspension of 3-bromo-1-ethylmaleimide (0.7 g, 3.43mmol) and 1-methyl-1H-indol-5-ylboronic acid (0.65g, 3.7mmol) in dioxane (15mL) under nitrogen was degassed with a stream of nitrogen for 10 min, treated with cesium fluoride (1.6g, 10.8mmol) and Cl₂Pd(dppf).CH₂Cl₂ (0.25g, 0.3mmol), then stirred at room temperature for 0.5h and at 45°C for 30min then at 65°C for 45min. The mixture was cooled and diluted with methylene chloride (50mL). The mixture was filtered through Celite^{®} (rinse filter cake with methylene chloride) and the brown filtrate concentrated in vacuo. The residue was dissolved in methylene chloride and filtered through a column of silica gel (eluted with methylene chloride 60% and ethyl acetate 40%) to afford an orange solid, which was triturated from cold petroleum ethers to afford arylmaleimide intermediate (585 mg, 67%) as an orange solid.

A stirred suspension of sodium hydride oil dispersion (60%, 140mg, 3.5mmol) in anhydrous tetrahydrofuran (30mL) under nitrogen was treated with trimethylsulfoxonium chloride (0.55g, 4.25mmol), then refluxed for 2.5h and cooled (50°C). The above arylmaleimide (937mg, 3.5mmol) was added in one portion and the mixture stirred at 50°C for 3h, cooled on an ice bath, and quenched with saturated ammonium chloride (10mL). The product mixture was extracted with ether (2X50mL), and the combined extracts washed with water (30mL), dried (MgSO₄), and concentrated in vacuo. The residual solid was dissolved in 1:1 methylene chloride/heptane and loaded onto a silica gel column and eluted with 1:1, 2:1, then 3:1 methylene chloride/heptane to afford the bicyclic diimide intermediate (628mg, 64%) as a very pale yellow oil. ¹H NMR (CDCl₃) δ 1.14 (t, 3 H) 1.79 (dd, J=4.39, 3.61 Hz, 1 H) 1.89 (dd, J=8.10, 4.39 Hz, 1 H) 2.70 (dd, J=8.10, 3.61 Hz, 1 H) 3.42 - 3.56 (m, 2 H) 3.78 - 3.81 (m, 1 H) 6.46 (dd, J=3.12, 0.78 Hz, 1 H) 7.07 (d, J=2.93 Hz, 1 H) 7.26 (d, J=1.76 Hz, 1 H) 7.29 - 7.37 (m, 1 H) 7.63 (d, J=0.98 Hz, 1 H).

A stirred ice-cooled solution of 2.0N LAH/THF (2.2mL, 2.23mmol) under nitrogen was treated dropwise with a solution of the above bicyclic diimide intermediate (150mg, 0.6mmol) in anhydrous THF (10mL). The solution was stirred at room temperature for 15min, refluxed for 4h, cooled on an ice bath, and carefully treated dropwise with 30% NaOH (1.3mL, vigorous evolution of gas). The solution was filtered and the filtrate was reduced to a clear oil. The residue was dissolved in methanol (23mL), treated with 4N HCl/dioxane (7mL), then stirred at room temperature for 3h. The solution was concentrated in vacuo and the residue triturated from ether to afford 1-(1-methyl-1H-indol-5-yl)-3-ethyl-3-azabicyclo[3.1.0]hexane, hydrochloride (76mg, 53%) as a white solid. MS (M+1) 241. ¹H NMR (CDCl₃) δ 1.10 (d, J=6.44 Hz, 1 H) 1.48 (t, 3 H) 1.92 - 2.02 (m, 1 H) 2.19 (dd, J=6.54, 4.59 Hz, 1 H) 3.13 - 3.25 (m, 3 H) 3.25 - 3.36 (m, 1 H) 3.74 - 3.79 (m, 3 H) 3.91 (dd, J=11.03, 5.37 Hz, 1 H) 4.10 (dd, J=10.84, 5.37 Hz, 1 H) 6.38 - 6.47 (m, 1 H) 6.98 - 7.12 (m, 2 H) 7.26 - 7.33 (m, 1 H) 7.40 - 7.54 (m, 1 H). ¹³C NMR (CDCl₃) δ 11.12, 22.47, 31.79, 33.16, 51.48, 55.69, 59.78, 66.05, 100.44, 109.10, 120.10, 121.37, 122.56, 123.34, 129.62.

### D. Synthesis of 2-(3-ethyl-3-azabicyclo[3.1.0]hex-1-yl)-1-methyl-1H-indole, hydrochloride

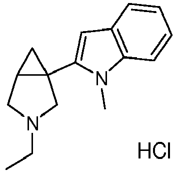

A stirred solution/suspension of 3-bromo-1-ethylmaleimide (1.0 g, 4.9mmol) and 1-methyl-2-tributylstannylindole (2.3g, 5.4mmol) in dioxane (15mL) under nitrogen was degassed with a stream of nitrogen for 10 min, treated with cesium fluoride (1.6g, 10.8mmol) and Cl₂Pd(dppf).CH₂Cl₂ (0.25g, 0.3mmol), then stirred at room temperature for 0.5h and at 45°C for 30min then at 65°C for 45min. The mixture was cooled and diluted with methylene chloride (50mL). The mixture was filtered through Celite^{®} (rinse filter cake with methylene chloride) and the brown filtrate concentrated *in vacuo.* The residue was dissolved in methylene chloride and filtered through a column of silica gel (eluted with methylene chloride 60% and ethyl acetate 40%) to afford a yellowish solid, which was triturated from cold petroleum ethers to afford the arylmaleimide intermediate (1.32 g, 87%) as a yellowish solid.

A stirred suspension of sodium hydride oil dispersion (60%, 140mg, 3.5mmol) in anhydrous tetrahydrofuran (30mL) under nitrogen was treated with trimethyl-sulfoxonium chloride (0.495g, 3.85mmol), then refluxed for 2.5h and cooled (50°C). The above arylmaleimide (900mg, 3.5mmol) was added in one portion and the mixture stirred at 50°C for 3h, cooled on an ice bath, and quenched with saturated ammonium chloride (10mL). The product mixture was extracted with ether (2X50mL), and the combined extracts washed with water (30mL), dried (MgSO₄), and concentrated *in vacuo.* The residual solid was dissolved in *1:1 methylene chloude/heptane and loaded onto a silica gel column and eluted with 1:1, 2:1, then 3:1 methylene chloride/heptane to afford the bicyclic diimide intermediate (370mg, 40%) as a very pale yellow oil. ¹H NMR (CDCl₃) δ 1.15 (t, 3 H) 1.86 (dd, *J*=4.88, 3.71 Hz, 1 H) 1.93 (dd, *J*=8.20, 4.88 Hz, 1 H) 2.57 (dd, *J*=8.30, 3.81 Hz, 1 H) 3.44 - 3.53 (m, 2 H) 7.29 (d, *J*=1.17 Hz, 2 H) 7.45 (t, *J*=1.17 Hz, 1 H).

A stirred ice-cooled solution of 2.0N LAH/THF (6.0mL, 5.8mmol) under nitrogen was treated dropwise with a solution of the above bicyclic diimide intermediate (370mg, 1.45mmol) in anhydrous THF (10mL). The solution was stirred at room temperature for 15min, refluxed for 4h, cooled on an ice bath, and carefully treated dropwise with 30% NaOH (1.3mL, vigorous evolution of gas). The solution was filtered and the filtrate was reduced to a clear oil. The residue was dissolved in methanol (23mL), treated with 4N HCl/dioxane (7mL), then stirred at room temperature for 3h. The solution was concentrated *in vacuo* and the residue triturated from ether to afford 2-(3-ethyl-3-aza-bicyclo[3.1.0]hex-1-yl)-1-methyl-1H-indole, hydrochloride (262mg, 75%) as a white solid. MS (M+1) 241. ¹H NMR (CDCl₃) δ 1.12 - 1.19 (m, 1 H) 1.48 (t, *J*=7.13 Hz, 3 H) 2.10 - 2.20 (m, 1 H) 2.39 (dd, *J*=6.25, 4.69 Hz, 1 H) 3.04 - 3.26 (m, 3 H) 3.28 - 3.42 (m, 1 H) 3.78 (s, 3 H) 3.93 (dd, *J*=10.93, 5.27 Hz, 1 H) 4.10 (dd, *J*=10.93, 5.27 Hz, 1 H) 6.36 (s, 1 H) 7.02 - 7.15 (m, 1 H) 7.15 - 7.31 (m, 2 H) 7.52 (d, *J*=8.00 Hz, 1 H). ¹³C NMR (CDCl₃) δ 11.11, 15.28, 22.42, 24.24, 30.74, 51.55, 54.91, 58.62, 101.39, 109.43, 120.30, 120.78, 122.50, 127.21, 136.70, 137.94.

### E. Synthesis of 1-(quinolin-5-yl)-3-ethyl-3-azabicyclo[3.1.0]hexane, hydrochloride

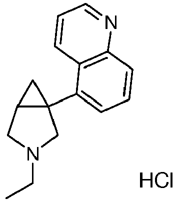

A stirred solution/suspension of 3-bromo-1-ethylmaleimide (0.7 g, 3.43mmol) and 5-quinolineboronic acid (1.2g, 5.9mmol) in dioxane (15mL) under nitrogen was degassed with a stream of nitrogen for 10 min, treated with cesium fluoride (1.6g, 10.8mmol) and Cl₂Pd(dppf).CH₂Cl₂ (0.25g, 0.3mmol then stirred at room temperature for 0.5h and at 45°C for 30min then at 65°C for 45min. The mixture was cooled and diluted with methylene chloride (50mL). The mixture was filtered through Celite^{®} (rinse filter cake with methylene chloride) and the brown filtrate concentrated *in vacuo.* The residue was dissolved in methylene chloride and filtered through a column of silica gel (eluted with methylene chloride 60% and ethyl acetate 40%) to afford a tan solid, which was triturated from cold petroleum ethers to afford the arylmaleimide intermediate (430 mg, 30%) as a tan solid.

A stirred suspension of sodium hydride oil dispersion (60%, 140mg, 3.5mmol) in anhydrous tetrahydrofuran (30mL) under nitrogen was treated with trimethyl-sulfoxonium chloride (0.55g, 4.25mmol), then refluxed for 2.5h and cooled (50°C). The above arylmaleimide (937mg, 3.5mmol) was added in one portion and the mixture stirred at 50°C for 3h, cooled on an ice bath, and quenched with saturated ammonium chloride (10mL). The product mixture was extracted with ether (2X50mL), and the combined extracts washed with water (30mL), dried (MgSO₄), and concentrated *in vacuo.* The residual solid was dissolved in 1:1 methylene chloride/heptane and loaded onto a silica gel column and eluted with 1:1, 2:1, then 3:1 methylene chloride/heptane to afford bicyclic diimide intermediate (628mg, 64%) as a very pale yellow oil. ¹H NMR (CDCl₃) δ 1.19 (t, 3 H) 1.85 - 1.95 (m, 1 H) 1.97 - 2.08 (m, 1 H) 2.82 (dd, *J*=8.40, 3.51 Hz, 1 H) 3.47 - 3.64 (m, 2 H) 7.35 - 7.87 (m, 4 H) 8.17 (dd, *J*=8.49, 3.81 Hz, 1 H) 8.82 - 9.03 (m, 1 H).

A stirred ice-cooled solution of 2.0N LAH/THF (0.58mL, 1.1mmol) under nitrogen was treated dropwise with a solution of the above bicyclic diimide intermediate (77mg, 0.29mmol) in anhydrous THF (10mL). The solution was stirred at room temperature for 15min, refluxed for 4h, cooled on an ice bath, and carefully treated dropwise with 30% NaOH (1.3mL, vigorous evolution of gas). The solution was filtered and the filtrate was reduced to a clear oil. The residue was dissolved in methanol (23mL), treated with 4N HCl/dioxane (7mL), then stirred at room temperature for 3h. The solution was concentrated *in vacuo* and the residue triturated from ether to afford 1-(quinolin-5-yl)-3-ethyl)-3-azabicyclo[3.1.0]hexane, hydrochloride (55mg, 81%) as a white solid. MS (M+1) 239. ¹H NMR (CDCl₃) δ 1.0 7 (t, *J*=7.03 Hz, 1 H) 1.27 (t, *J*=7.22 Hz, 3 H) 1.31 - 1.38 (m, 1 H) 1.61 - 1.82 (m, 1 H) 3.24 (s, 1 H) 3.48 - 3.57 (m, 1 H) 3.62 - 3.79 (m, 2 H) 4.06 (dd, *J*=11.13, 5.27 Hz, 1 H) 7.66 (t, *J*=7.61 Hz, 1 H) 7.69 - 7.83 (m, 1 H) 7.97 (s, 1 H) 8.06 (d, *J*=8.79 Hz, 1 H) 8.41 (s, 1 H) 8.95 (d, *J*=2.34 Hz, 1 H). ¹³C NMR (CDCl₃) δ 10.96, 29.17, 30.13, 42.07, 51.27, 55.11, 58.82, 122.30, 122.55, 123.77, 128.09, 131.10, 131.49, 134.91, 138.88, 143.98.

### Example IV

### Preparation of 1-heteroaryl-3-isopropyl-3-azabicyclo[3.1.0]hexanes using Reaction Scheme 2

### A. Synthesis of 3-bromo-1-isopropylmaleimide

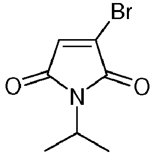

A cooled (5°C) stirred solution of maleic anhydride (29.4g, 0.30mole) in anhydrous ether (150mL) under nitrogen was treated dropwise over 45 min with a solution of isopropylamine (35.5g, 0.60mole) in anhydrous ether (100mL) at a rate to keep the pot temp <20°C, then the mixture was stirred at 10°C for 15 min, filtered, and the filter cake rinsed with anhydrous ether and dried *in vacuo* to afford a white solid. This was taken up in acetic anhydride (250mL), treated with anhydrous sodium acetate (12.3g, 0.15mole), and heated to 75°C with stirring for 4.5h, then at 100°C for 1.5h. The mixture was concentrated *in vacuo* and the residue taken up in methylene chloride (300mL), washed with saturated aqueous sodium bicarbonate (200mL), water (200mL), dried (MgSO₄), and concentrated *in vacuo.* The residue was distilled (approx. 5mm pressure) to afford two products; N-isopropylmaleimide at 82°C (13.0g) and an acetate adduct of N-isopropylmaleimide at 154°C (12.9g). The acetate adduct was dissolved in 4:1 acetonitrile/triethylamine (100mL), heated to 65°C for 4h, then concentrated *in vacuo.* The residue was dissolved in methylene chloride and filtered through a pad of silica gel (eluted with methylene chloride) to afford an additional 3.5g of N-isopropylmaleimide. Total yield was 16.5g of N-isopropylmaleimide (40%).

A stirred ice-cold solution of N-isopropylmaleimide (16.4g, 0.118mole) in carbon tetrachloride (12mL) under nitrogen was treated dropwise with bromine (6.41mL, 0.25mole) at a rate to keep the pot temp <9°C, then stirred at 3°C for 2h, during which time the mixture formed a solid cake. The cake was maintained under a stream of nitrogen to allow excess bromine and CCl₄ to evaporate, then the reaction mixture was placed under vacuum to remove the remaining solvent. Ethanol (100mL) was added to the flask, followed by sodium acetate (11g, 0.134mole), and the mixture was refluxed for 16h with stirring. The cooled solution was filtered through Celite^{®} (filter cake rinsed with methylene chloride), and the filtrate concentrated *in vacuo,* dissolved in methylene chloride, filtered through a pad of alumina (eluted with methylene chloride), and re-concentrated *in vacuo.* The residue was dissolved in 2:1 petroleum ether/methylene chloride, loaded onto a column of silica gel, and eluted successively with 2:1 petroleum ethers/CH₂Cl₂, 1:1 petroleum ethers/CH₂Cl₂, and CH₂Cl₂ alone to afford the subject compound (16.45g, 64% yield) as a pale yellow, low melting solid. No MS (M+1) peak observed. ¹H NMR (CDCl₃) δ 6.78 (s, 1H), 4.30-4.40 (m, 1H), 1.37 (d, 6H, J=8Hz).

### B. Synthesis of 1-(quinolin-3-yl)-3-isopropyl-3-azabicyclo[3.1.0]hexane, hydrochloride

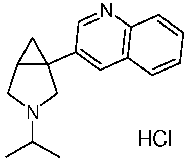

A stirred solution/suspension of 3-bromo-1-methylethylmaleimide (0.7 g, 3.43mmol) and 3-quinoline boronic acid (1.2g, 5.9mmol) in dioxane (15mL) under nitrogen was degassed with a stream of nitrogen for 10 min, treated with cesium fluoride (1.6g, 10.8mmol) and Cl₂Pd(dppf).CH₂Cl₂ (0.25g, 0.3mmol) then stirred at room temperature for 0.5h and at 45°C for 30min then at 65°C for 45min. The mixture was cooled and diluted with methylene chloride (50mL). The mixture was filtered through Celite^{®} (rinse filter cake with methylene chloride) and the brown filtrate concentrated *in vacuo.* The residue was dissolved in methylene chloride and filtered through a column of silica gel (eluted with methylene chloride 60% and ethyl acetate 40%) to afford a tan solid, which was triturated from cold petroleum ethers to afford arylmaleimide intermediate (120mg, 30%) as a tan solid.

A stirred suspension of trimethyl-sulfoxonium chloride (0.062g, 0.48mmol) under nitrogen was treated with 2.5M n-butyl lithium (0.19mL, 0.48mmol) in anhydrous tetrahydrofuran (30mL), then refluxed for 2.5h and cooled (50°C). The above arylmaleimide (115mg, 0.43mmol) was added in one portion and the mixture stirred at 50°C for 3h, cooled on an ice bath, and quenched with saturated ammonium chloride (10mL). The product mixture was extracted with ether (2X50mL), and the combined extracts washed with water (30mL), dried (MgSO₄), and concentrated *in vacuo.* The residual solid was dissolved in 1:1 methylene chloride/heptane and loaded onto a silica gel column and eluted with 1:1, 2:1, then 3:1 methylene chloride/heptane to afford the bicyclic diimide intermediate (75mg, 62%) as a very pale yellow oil. ¹H NMR (CDCl₃) δ 1.31 - 1.43 (m, 6 H) 1.80 - 1.91 (m, 2 H) 2.87 (dd, *J*=7.61, 4.30 Hz, 1 H) 4.17 - 4.37 (m, 1 H) 7.51 - 7.62 (m, 1 H) 7.68 - 7.77 (m, 1 H) 7.81 (d, *J*=8.00 Hz, 1 H) 8.11 (d, *J*=8.59 Hz, 1 H) 8.25 (d, *J*=1.95 Hz, 1 H) 8.89 (d, *J*=1.17 Hz, 1H).

A stirred ice-cooled solution of 2.0N LAH/THF (0.58mL, 1.1mmol) under nitrogen was treated dropwise with a solution of the above bicyclic diimide intermediate (72mg, 0.27mmol) in anhydrous THF (10mL). The solution was stirred at room temperature for 15min, refluxed for 4h, cooled on an ice bath, and carefully treated dropwise with 30% NaOH (0.5mL, vigorous evolution of gas). The solution was filtered and the filtrate was reduced to a clear oil. The residue was dissolved in methanol (23mL), treated with 4N HCl/dioxane (7mL), then stirred at room temperature for 3h. The solution was concentrated *in vacuo* and the residue triturated from ether to afford 1-(quinolin-3-yl)-3-(2-propyl)-3-azabicyclo[3.1.0]hexane, hydrochloride (50mg, 65%) as a white solid. MS (M+1) 253. ¹H NMR (CD₃OD) δ 1.37 - 1.45 (m, 1 H) 1.49 (d, *J*=5.94 Hz, 6 H) 1.59 (t, *J*=7.73 Hz, 1 H) 1.81 (dd, *J*=6.74, 4.76 Hz, 1 H) 2.46 - 2.60 (m, 1 H) 3.79 - 3.87 (m, 1 H) 3.88 - 3.95 (m, 2 H) 4.26 (d, *J*=11.10 Hz, 1 H) 7.99 (t, *J*=7.73 Hz, 1 H) 8.16 (t, *J*=7.33 Hz, 1 H) 8.26 (d, *J*=8.32 Hz, 1 H) 8.33 (d, *J*=8.32 Hz, 1 H) 9.22 (s, 1 H) 9.37 (d, *J*=1.98 Hz, 1 H). ¹³C NMR (CD₃OD) δ 16.44, 19.42, 25.07, 29.93, 55.59, 57.94, 61.11, 121.95, 129.64, 131.44, 135.91, 138.94, 146.66.

### C. Synthesis of 1-(1-methyl-1H-indol-5-yl)-3-isopropyl-3-azabicyclo[3.1.0]hexane, hydrochloride

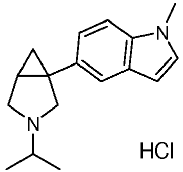

A stirred solution/suspension of 3-bromo-1-methylethylmaleimide (0.7g, 3.43mmol) and 5-quinoline-boronic acid (1.2g, 5.9mmol) in dioxane (15mL) under nitrogen was degassed with a stream of nitrogen for 10 min, treated with cesium fluoride (1.6g, 10.8mmol) and Cl₂Pd(dppf).CH₂Cl₂ (0.25g, 0.3mmol) then stirred at room temperature for 0.5h and at 45°C for 30min then at 65°C for 45min. The mixture was cooled and diluted with methylene chloride (50mL). The mixture was filtered through Celite^{®} (rinse filter cake with methylene chloride) and the brown filtrate concentrated *in vacuo.* The residue was dissolved in methylene chloride and filtered through a column of silica gel (eluted with methylene chloride 60% and ethyl acetate 40%) to afford a tan solid, which was triturated from cold petroleum ethers to afford arylmaleimide intermediate (430 mg, 30%) as a tan solid.

A stirred suspension of trimethylsulfoxonium chloride (0.290g, 2.28mmol) under nitrogen was treated with 2.5M n-butyllithium (0.95mL, 2.18 mmol) in anhydrous tetrahydrofuran (30mL), then refluxed for 2.5h and cooled (50°C). The above arylmaleimide (530mg, 1.98 mmol) was added in one portion and the mixture stirred at 50°C for 3h, cooled on an ice bath, and quenched with saturated ammonium chloride (10mL). The product mixture was extracted with ether (2X50mL), and the combined extracts washed with water (30mL), dried (MgSO₄), and concentrated *in vacuo.* The residual solid was dissolved in 1:1 methylene chloride/heptane and loaded onto a silica gel column and eluted with 1:1, 2:1, then 3:1 methylene chloride/heptane to afford bicyclic diimide intermediate (120mg, 21%) as a very pale yellow oil. ¹H NMR (CDCl₃) δ 1.22 - 1.39 (m, 6 H) 1.73 (dd, *J*=4.21, 3.48 Hz, 1 H) 1.83 (dd, *J*=8.14, 4.30 Hz, 1 H) 2.67 (dd, *J*=8.05, 3.48 Hz, 1 H) 3.79 (s, 3 H) 4.14 - 4.31 (m, 1 H) 6.43 - 6.47 (m, 1 H) 7.04 - 7.08 (m, 1 H) 7.23 - 7.27 (m, 1 H) 7.28 - 7.35 (m, 1 H) 7.63 (d, *J*=1.10 Hz, 1 H).

A stirred ice-cooled solution of 2.0N LAH/THF (0.820mL, 1.6mmol) under nitrogen was treated dropwise with a solution of the above bicyclic diimide intermediate (116mg, 0.41mmol) in anhydrous THF (10mL). The solution was stirred at room temperature for 15min, refluxed for 4h, cooled on an ice bath, and carefully treated dropwise with 30% NaOH (0.5mL, vigorous evolution of gas). The solution was filtered and the filtrate was reduced to a clear oil. The residue was dissolved in methanol (23mL), treated with 4N HCl/dioxane (7mL), then stirred at room temperature for 3h. The solution was concentrated *in vacuo* and the residue triturated from ether to afford 1-(1-methyl-1H-indol-5-yl)-3-(2-propyl)-3-azabicyclo[3.1.0]-hexane, hydrochloride (60mg, 62%) as a white solid. MS (M+1) 255. ¹H NMR (CDCl₃) δ 1.10 - 1.20 (m, 1 H) 1.51 (t, *J*=6.54 Hz, 6 H) 1.89 - 2.00 (m, 1 H) 2.29 (dd, *J*=6.44, 4.49 Hz, 1 H) 3.17 - 3.40 (m, 3 H) 3.74 - 3.79 (m, 3 H) 3.86 (dd, *J*=10.84, 5.56 Hz, 1 H) 4.06 (dd, *J*=10.93, 5.47 Hz, 1 H) 6.41 (d, *J*=3.12 Hz, 1 H) 7.00 - 7.10 (m, 2 H) 7.21 - 7.29 (m, 1 H) 7.46 (d, *J*=1.37 Hz, 1 H). ¹³C NMR (CDCl₃) δ 15.95, 18.97, 22.62, 31.96, 33.16, 54.32, 58.59, 59.54, 109.88, 120.14, 121.45, 128.84, 129.06, 130.15, 136.18.

### D. Synthesis of 1-(1-Methyl-2-indolyl)-3-isopropyl-3-azabicyclo[3.1.0]hexane, hydrochloride

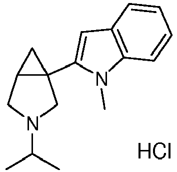

A stirred solution/suspension of 3-bromo-1-(1-methylethyl)maleimide (1.09g, 5mmol) and (1-methylindol-2-yl)tributyltin (2.63g, 6.25mmol) in dioxane (30mL) under nitrogen was degassed with a stream of nitrogen for 10 min, then treated with cesium fluoride (1.8g, 11.8mmol) and Cl₂Pd(PPh₃)₂ (0.21g, 0.3mmol), stirred at room temperature for 30min, 40°C for 30min, and 60°C for 1.5h. The mixture was cooled to room temperature, taken up in methylene chloride (100mL), and filtered through Celite^{®} (rinse filter cake with methylene chloride) and the filtrate concentrated *in vacuo.* The residue was dissolved in methylene chloride and filtered through a column of silica gel (eluted with methylene chloride) to afford a dark solid, which was triturated from ether to afford the arylmaleimide intermediate (1.236g, 92%) as a yellow solid. MS (M+1) 269.2. ¹H NMR (CDCl₃) δ 7.69 (m, 1H), 7.59 (s, 1H), 7.34 (m, 2H), 7.14 (m, 1H), 6.52 (s, 1H), 4.44 (m, 1H), 3.87 (s, 3H), 1.46 (d, 6H, J=7Hz).

A stirred suspension of sodium hydride oil dispersion (60%, 160mg, 4.0mmol) in anhydrous tetrahydrofuran (35mL) under nitrogen was treated with trimethylsulfoxonium chloride (0.643g, 5.0mmol), then refluxed for 2.5h and cooled (50°C). The above arylmaleimide (1.073g, 4.0mmol) was added in one portion and the mixture stirred at 50°C for 3h, cooled on an ice bath, and quenched with saturated ammonium chloride (12mL). The product mixture was extracted with ether (2X50mL), and the combined extracts washed with water (35mL), dried (MgSO₄), and concentrated *in vacuo.* The residue was dissolved in methylene chloride and loaded onto a silica gel column and eluted with methylene chloride to afford the bicyclic diimide intermediate (640mg, 57%) as a pale orange solid. MS (M+1) 283.1. ¹H NMR (CDCl₃) δ 7.56 (m, 1H), 7.32 (m, 1H), 7.20-7.27 (m, 2H), 7.10 (m, 1H), 4.28 (m, 1H), 3.80 (s, 3H), 2.72 (m, 1H), 1.87 (m, 2H), 1.37 (m, 6H).

A stirred cooled (5°C) mixture of 2.0N lithium aluminum hydride/THF (5.2mL, 10.4mmol) in a 3-neck 100 mL round bottom flask under nitrogen was treated dropwise with a solution of the above bicyclic diimide intermediate (0.367g, 1.30 mmol) in anhydrous THF (5mL), and the mixture was stirred 1h at room temperature and 3h at reflux, then recooled on an ice bath. Water (0.4mL), 15% sodium hydroxide (0.4mL), and water (1.2mL) were carefully added dropwise, and the mixture was stirred for a few minutes and filtered (rinse filter cake with methylene chloride). The filtrate was concentrated *in vacuo* and the residue dissolved in methylene chloride and loaded onto a silica gel column and eluted with 5% (9:1 ethanol/ammonia)/methylene chloride, then 10% (9:1 ethanol/ammonia)/methylene chloride to afford the free base of the title compound as a colorless oil. This was dissolved in ether (15mL) and treated with 2.0N HCl/ether (1.25mL, 2.5mmol), the suspension diluted with a few mL of methylene chloride, stirred a few minutes, and the solid filtered, collected and dried *in vacuo* to afford 1-(1-methyl-2-indolyl)-3-(2-propyl)-3-azabicyclo[3.1.0]hexane, hydrochloride (321mg, 85%) as a white solid. MS (M+1) 255.2. ¹H NMR (CDCl₃) δ 7.52 (d, 1H, J=8Hz), 7.20-7.30 (m, 2H), 7.09 (m, 1H), 6.35 (br s, 1H), 4.08 (m, 1H), 3.91 (m, 1H), 3.79 (s, 3H), 3.41 (m, 1H), 3.30 (m, 1H), 3.15 (m, 1H), 2.52 (m, 1H), 2.13 (m, 1H), 1.52 (m, 6H), 1.14 (m, 1H). ¹³C NMR (CDCl₃) δ 137.93, 136.96, 127.22, 122.49, 120.76, 120.30, 109.43, 101.26, 59.71, 57.36, 53.55, 30.77, 24.43, 22.61, 18.97, 15.36.

### E. Synthesis of 1-(quinolin-5-yl)-3-isopronyl-3-azabicyclo[3.1.0]hexane, hydrochloride

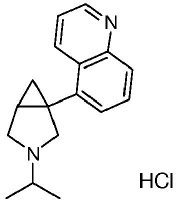

A stirred solution/suspension of 3-bromo-1-methylethylmaleimide (0.7 g, 3.43mmol) and 5-quinolineboronic acid (1.2g, 5.9mmol) in dioxane (15mL) under nitrogen was degassed with a stream of nitrogen for 10 min, treated with cesium fluoride (1.6g, 10.8mmol) and Cl₂Pd(dppf).CH₂Cl₂ (0.25g, 0.3mmol) then stirred at room temperature for 0.5h and at 45°C for 30min then at 65°C for 45min. The mixture was cooled and diluted with methylene chloride (50mL). The mixture was filtered through Celite^{®} (rinse filter cake with methylene chloride) and the brown filtrate concentrated *in vacuo.* The residue was dissolved in methylene chloride and filtered through a column of silica gel (eluted with methylene chloride 60% and ethyl acetate 40%) to afford a tan solid, which was triturated from cold petroleum ethers to afford the arylmaleimide intermediate (430 mg, 30%) as a tan solid.

A stirred suspension of trimethylsulfoxonium chloride (0.062g, 0.48mmol) under nitrogen was treated with 2.5M n-butyllithium (0.19mL, 0.48mmol) in anhydrous tetrahydrofuran (30mL), then refluxed for 2.5h and cooled (50°C). The above arylmaleimide (115mg, 0.43mmol) was added in one portion and the mixture stirred at 50°C for 3h, cooled on an ice bath, and quenched with saturated ammonium chloride (10mL). The product mixture was extracted with ether (2X50mL), and the combined extracts washed with water (30mL), dried (MgSO₄), and concentrated *in vacuo.* The residual solid was dissolved in 1:1 methylene chloride/heptane and loaded onto a silica gel column and eluted with 1:1, 2:1, then 3:1 methylene chloride/heptane to afford the bicyclic diimide intermediate (75mg, 62%) as a very pale yellow oil. ¹H NMR (CDCl₃) δ 1.31 - 1.43 (m, 6 H) 1.80 - 1.91 (m, 2 H) 2.87 (dd, *J*=7.61, 4.30 Hz, 1 H) 4.17 - 4.37 (m, 1 H) 7.51 - 7.62 (m, 1 H) 7.68 - 7.77 (m, 1 H) 7.81 (d, *J*=8.00 Hz, 1 H) 8.11 (d, *J*=8.59 Hz, 1 H) 8.25 (d, *J*=1.95 Hz, 1 H) 8.89 (d, *J*=1.17 Hz, 1H).

A stirred ice-cooled solution of 2.0N LAH/THF (0.58mL, 1.1mmol) under nitrogen was treated dropwise with a solution of the above bicyclic diimide intermediate (68mg, 0.24mmol) in anhydrous THF (10mL). The solution was stirred at room temperature for 15min, refluxed for 4h, cooled on an ice bath, and carefully treated dropwise with 30% NaOH (0.5mL, vigorous evolution of gas). The solution was filtered and the filtrate was reduced to a clear oil. The residue was dissolved in methanol (23mL), treated with 4N HCl/dioxane (7mL), then stirred at room temperature for 3h. The solution was concentrated *in vacuo* and the residue triturated from ether to afford 1-(quinolin-5-yl)-3-(2-propyl)-3-azabicyclo[3.1.0]hexane, hydrochloride (50mg, 75%) as a white solid. MS (M+1) 254. ¹H NMR (CD₃OD) δ 1.37 - 1.45 (m, 1 H) 1.49 (d, *J*=5.94 Hz, 6 H) 1.59 (t, *J*=7.73 Hz, 1 H) 1.81 (dd, *J*=6.74, 4.76 Hz, 1 H) 2.46 - 2.60 (m, 1 H) 3.79 - 3.87 (m, 1 H) 3.88 - 3.95 (m, 2 H) 4.26 (d, *J*=11.10 Hz, 1 H) 7.99 (t, *J*=7.73 Hz, 1 H) 8.16 (t, *J*=7.33 Hz, 1 H) 8.26 (d, *J*=8.32 Hz, 1 H) 8.33 (d, *J*=8.32 Hz, 1 H) 9.22 (s, 1 H) 9.37 (d, *J*=1.98 Hz, 1 H). ¹³C NMR (CD₃OD) δ 15.24, 18.91, 22.07, 29.31, 29.63, 54.20, 57.34, 60.05, 121.83, 122.56, 129.41, 131.70, 135.21, 136.73, 138.52, 143.76, 144.49.

### Example V

### Preparation of 1-heteroaryl-3-azabicyclo[3.1.0]hexanes using Reaction Scheme 3

### A. Synthesis of 1-(benzothiophen-2-yl)-3-azabicyclo[3.1.0]hexane hydrochloride

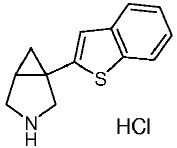

A stirred solution of 3-bromo-1-(3,4-dimethoxybenzyl)maleimide (3.26g, 10 mmol) and thionaphthene-2-boronic acid (2.13g, 12 mmol) in dioxane (30mL) was degassed under a stream of nitrogen for 10 minutes, then treated with cesium fluoride (4g, 26 mmol) and Cl₂Pd(dppf)CH₂Cl (0.5g, 0.61 mmol), then stirred at room temperature for 1 hour and at 40°C for 2h. The mixture was cooled and diluted with methylene chloride, stirred a few minutes and filtered through celite. The filtrate was concentrated *in vacuo* and purified by silica gel chromatography using 2% ethyl acetate/ methylene chloride as the eluting solvent to afford the arylmaleimide intermediate (3.3g, 87.3%). ¹H NMR (CDCl₃) δ 8.34-8.27 (m, 1H), 7.92-7.81 (m, 2H), 7.48-7.36 (m, 2H), 7.07-6.80 (m, 3H), 6.66-6.60 (m, 1H), 4.73-4.65 (m, 2H), 3.89 (s, 3H), 3.86 (s, 3H).

A cooled (-20°C) stirred solution of trimethylsulfoxonium chloride (964mg, 7.5 mmol) in anhydrous tetrahydrofuran (30mL) under nitrogen was treated with n-butyllithium/hexane (2.5N, 2.4mL, 6.0 mmol) and warmed to 50°C over 30 minutes. A solution of the arylmaleimide intermediate (2g, 5.4 mmol) in anhydrous tetrahydrofuran (20mL) was warmed to 50°C and added all at once. After 2h at 50°C, the mixture was cooled on ice and quenched with saturated aqueous ammonium chloride (2mL). The mixture was stirred at room temperature for 15 minutes, then it was added to methylene chloride (100mL) and the organic solution was dried (Na₂SO₄) and concentrated *in vacuo.* The residue was dissolved in methylene chloride, loaded onto a silica gel column and eluted with 2% ethyl acetate/methylene chloride to afford the bicyclic diimide intermediate (702mg, 33%). ¹H NMR (CDCl₃) δ 7.80-7.76 (m, 1H), 7.73-7.69 (m, 1H), 7.37-7.30 (m, 3H), 6.94-6.89 (m, 2H), 6.81-6.77 (m, 1H), 4.54 (q, 2H), 3.86 (s, 3H), 3.84 (s, 3H), 2.88-2.84 (m, 1H), 1.96-1.92 (m, 1H), 1.87-1.83 (m, 1H).

A stirred, ice-cooled (2°C) solution of 1N lithium aluminum hydride/THF (16mL, 16 mmol) under nitrogen was treated dropwise with a solution of the above bicyclic diimide (1.014g, 2.58 mmol) in anhydrous tetrahydrofuran (20mL), stirred 3h at room temperature and 1h at reflux, then cooled on an ice bath. Water (0.60mL), 15% NaOH (0.60mL), and water (1.80mL) were added dropwise together with more tetrahydrofuran to facilitate stirring. The mixture was stirred at room temperature for 15 minutes, filtered through celite and the filtrate concentrated *in vacuo.* The residue was dissolved in methylene chloride, loaded onto a silica gel column, and eluted with 3:1 methylene chloride/ethyl acetate to afford the bicyclic intermediate (754mg, 80%). ¹H NMR (CDCl₃) δ 7.74-7.59 (m, 2H), 7.31-7.19 (m, 2H), 6.99-6.97 (m, 1H), 6.90-6.78 (m, 3H), 3.90 (s, 3H), 3.85 (s, 3H), 3.65-3.60 (m, 2H), 3.30 (d, 1H), 3.05 (d, 1H), 2.75 (d, 1H), 2.60-2.54 (m, 1H), 1.77- 1.71 (m, 1H), 1.68-1.62 (m, 1H), 1.08-1.03 (m, 1H).

A solution of the above bicyclic intermediate (365mg, 1 mmol) and anhydrous potassium carbonate (276mg, 2 mmol) in anhydrous methylene chloride (6mL) in a pressure tube was treated with 1-chloroethyl chloroformate (0.3mL, 3 mmol). The tube was closed under nitrogen flow, stirred at 40°C for 4h, then cooled and uncapped. The contents were filtered and the filtrate concentrated *in vacuo,* dissolved in methanol (12mL), refluxed for 1h, then cooled to room temperature and treated with DOWEX 550A-OH. The mixture was filtered and the filtrate concentrated *in vacuo* to debenzylated free base of 1-(benzothiophen-2-yl)-3-azabicyclo[3.1.0]hexane (120mg, 56%). 45 mg of compound was converted to the hydrochloride salt by treating with 2N HCl/ether. MS (M+1) 215.8. ¹H NMR (MeOH-*d*₄) δ 7.82-7.67 (m, 2H), 7.36-7.23 (m, 3H), 3.85-3.66 (m, 3H), 3.57-3.49 (m, 1H), 3.32-3.27 (m, 1H), 2.24-2.17 (m, 1H), 1.54-1.47 (m, 1H), 1.37-1.31 (m, 1H). ¹³C NMR (MeOD-*d*₄) δ 142.58, 140.10, 138.95, 124.52, 124.35, 123.14, 121.95, 121.76, 50.52, 47.69, 28.06, 25.82, 16.16.

### B. Synthesis of 1-(benzothiophen-2-yl)-3-methyl-3-azabicyclo[3.1.0]hexane hydrochloride

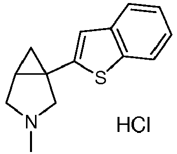

A stirred solution of 1-(benzothiophen-2-yl)-3-azabicyclo[3.1.0]hexane (72mg, 0.348 mmol) in 1,2-dichloroethane (7ml) was treated with 37% formaldehyde (0.21 mL, 2.8 mmol), then with sodium triacetoxyborohydride (294.5mg, 1.39 mmol) and this reaction mixture was stirred at room temperature for 3h. 1N sodium hydroxide (7mL) was then added, stirring continued for a few minutes, and the organic layer was separated. The aqueous layer was washed with methylene chloride, and the organic layers were combined, washed with brine, dried (Na₂SO₄), filtered and concentrated *in vacuo.* The residue was chromatographed on a silica gel column (eluted with 90:9:1 methylene chloride/ethanol/ammonia). The oily 1-(benzothiophen-2-yl)-3-methyl-3-aza-bicyclo[3.1.0]hexane (71.6mg, 90%) was converted to the hydrochloride salt by treating with 2N HCl/ether (49mg, 53%). MS (M+1) 229.79. ¹H NMR (MeOD-*d*₄) δ 7.84-7.66 (m, 2H), 7.39-7.23 (m, 3H), 4.11- 4.02 (m, 1H), 3.85-3.71 (m, 2H), 3.68-3.60 (m, 1H), 3.07-2.97 (s, 3H), 2.31-2.21 (m, 1H), 1.56 - 1.44 (m, 2H). ¹³C NMR (METHANOL-*d*₄) δ 142.20, 140.06, 138.95, 124.56, 124.41, 123.18, 121.96, 121.92, 59.69, 57.19, 40.00, 27.81, 25.6, 16.21.

### C. Synthesis of 1-(quinolin-5-yl)-3-azabicyclo[3.1.0]hexane, hydrochloride

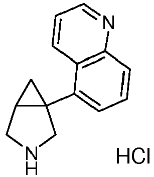

A stirred solution/suspension of 3-bromo-1-(3,4-dimethoxybenzyl)maleimide (3.8 g, 11.57 mmol) and quinoline-5-boronic acid (2g, 11.56 mmol) in dioxane (15mL) under nitrogen was degassed with a stream of nitrogen for 10 min, treated with cesium fluoride (3.8g, 25.4 mmol) and Cl₂Pd(dppf).CH₂Cl₂ (0.50g, 0.6 mmol), then stirred at room temperature for 0.5h and at 45°C for 30min then at 65°C for 45min. The mixture was cooled and diluted with methylene chloride (50mL). The mixture was filtered through Celite^{®} (rinse filter cake with methylene chloride) and the brown filtrate concentrated *in vacuo.* The residue was dissolved in methylene chloride and filtered through a column of silica gel (eluted with methylene chloride 60% and ethyl acetate 40%) to afford a pale yellow solid, which was triturated from cold petroleum ethers to afford the arylmaleimide intermediate (3.3 g, 77%) as pale yellow solid.

A cooled (-20°C) stirred solution of trimethylsulfoxonium chloride (1.25g, 9.7 mmol) in anhydrous tetrahydrofuran (15mL) under nitrogen was treated dropwise with n-butyllithium/hexane (2.4N, 3.9mL, 9.73 mmol) and gradually warmed to 50°C over 30 minutes. Meanwhile, a solution of the intermediate arylmaleimide (3.3 g, 8.8 mmol) in anhydrous THF (10mL) was heated to 50°C, then added quickly in one portion to the above heated suspension, and the mixture was stirred at 50°C for 2h, then cooled on an ice bath. Saturated aqueous ammonium chloride (1mL) was added to quench, and the mixture was diluted with methylene chloride (75mL), dried (MgSO₄), filtered through Celite^{®} (rinse with methylene chloride), and concentrated *in vacuo.* The residue was dissolved in methylene chloride, loaded onto a silica gel column, and the product eluted with 3% ethyl acetate/methylene chloride to afford the intermediate bicyclic diimide (300mg, 10%) as a very pale yellow viscous oil.

A cooled (5°C) stirred solution of 1N lithium aluminum hydride/THF (3.1mL, 3.1 mmol) under nitrogen was treated slowly with a solution of the above intermediate bicyclic diimide (300mg, 0.77 mmol) in anhydrous THF (7mL), stirred 1h at room temperature, refluxed for 6h, and cooled (5°C). Water (0.4mL), 15% sodium hydroxide (0.4mL), and water (1.2mL) were carefully added dropwise, followed by additional THF to facilitate stirring. The suspension was stirred 15min, filtered through Celite^{®} (filter cake rinsed with THF), and the filtrate concentrated *in vacuo.* The residue was dissolved in methylene chloride, loaded onto a silica gel column, and eluted with 3:1 methylene chloride/ethyl acetate to afford the intermediate dimethoxybenzyl bicyclic amine (110mg, 40%) as a colorless viscous oil.

A mixture of the intermediate dimethoxybenzyl bicyclic amine (100mg, 0.3 mmol) and anhydrous potassium carbonate (83mg, 0.6 mmol) in anhydrous methylene chloride (5mL) in a pressure tube equipped with a stirbar was treated with 1-chloroethyl chloroformate (0.130mL, 0.9mmol), closed, and stirred at 45°C for 4h. The tube was cooled, opened, and the contents filtered (rinse with methylene chloride), and the filtrate concentrated *in vacuo.* The residue was dissolved in methanol (7mL), refluxed for 1h, cooled, treated with DOWEX^{®} 550A-OH resin (2.0g, prerinsed with methanol), stirred a few minutes, filtered, and the filtrate concentrated *in vacuo.* The residue was taken up in ether, filtered through Celite^{®}, and the filtrate treated with 2N HCl/ether (0.6mL, 1.2 mmol). The suspension was stirred a few minutes, the solid salt collected by filtration, rinsed with ether, and dried *in vacuo* to afford 1-(quinolin-5-yl)-3-azabicyclo[3.1.0]- hexane, hydrochloride (70mg, 47%) as a light beige solid. MS (M+1) 225.1. ¹H NMR (DMSO-*d*₆) δ 9.33 (s, 1H), 8.94 (s, 1H), 8.36 (d,1H, *J*=8.5Hz), 8.17 (d, 1H, *J*=8Hz), 8.01 (t, 1H, *J*=7.5Hz), 7.86 (t, 1H, *J*=7.5Hz), 3.97 (dd, 1H, *J*=11Hz, 5Hz), 3.42-3.86 (m, 3H), 2.24-2.43 (m, 1H), 1.74-2.14 (m, 1H), 1.44 (t, 1H, *J*=7.5Hz). ¹³C NMR (DMSO-*d*₆) δ 147.12, 142.14, 134.04, 133.52, 129.91, 129.10,128.57, 123.24, 58.85, 56.63, 29.36, 24.70, 15.32.

### D. Synthesis of 1-(quinolin-3-yl)-3-azabicyclo[3.1.0]hexane, hydrochloride

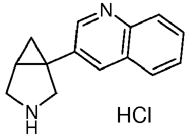

A stirred solution of 3-bromo-1-(3,4-dimethoxybenzyl)maleimide (1.0g, 3.06 mmol) and quinoline-3-boronic acid (0.52g, 3.4 mmol) in anhydrous dioxane (10mL) under nitrogen was degassed over 10min with a stream of nitrogen, then treated with cesium fluoride (1.3g, 8.5 mmol) and Cl₂Pd(dppf).CH₂Cl₂ (Aldrich, 0.17g, 0.21 mmol), stirred 1h at room temperature, then 2h at 40°C. The mixture was cooled, diluted with methylene chloride (50mL), stirred a few minutes, filtered through Celite^{®} (rinse with methylene chloride), and the filtrate concentrated *in vacuo*. The residue was dissolved in methylene chloride and loaded onto a silica gel column and the product eluted with 3% ethyl acetate/methylene chloride to afford a yellow solid, which was triturated from petroleum ethers to afford the intermediate arylmaleimide (940g, 79%) as a pale yellow solid.

A cooled (-20°C) stirred solution of trimethylsulfoxonium chloride (370mg, 2.86 mmol) in anhydrous tetrahydrofuran (15mL) under nitrogen was treated dropwise with n-butyllithium/hexane (2.4N, 1.1mL, 2.03 mmol) and gradually warmed to 50°C over 30 minutes. Meanwhile, a solution of the intermediate arylmaleimide (0.94g, 2.6 mmol) in anhydrous THF (10mL) was heated to 50°C, then added quickly in one portion to the above heated suspension, and the mixture was stirred at 50°C for 2h, then cooled on an ice bath. Saturated aqueous ammonium chloride (1mL) was added to quench, and the mixture was diluted with methylene chloride (75mL), dried (MgSO₄), filtered through Celite^{®} (rinse with methylene chloride), and concentrated *in vacuo*. The residue was dissolved in methylene chloride, loaded onto a silica gel column, and the product eluted with 3% ethyl acetate/methylene chloride to afford the intermediate bicyclic diimide (400mg, 50%) as a very pale yellow viscous oil.

A cooled (5°C) stirred solution of 1N lithium aluminum hydride/THF (3.6mL, 10 mmol) under nitrogen was treated slowly with a solution of the above intermediate bicyclic diimide (400mg, 1.2 mmol) in anhydrous THF (7mL), stirred 1h at room temperature, refluxed for 6h, and cooled (5°C). Water (0.4mL), 15% sodium hydroxide (0.4mL), and water (1.2mL) were carefully added dropwise, followed by additional THF to facilitate stirring. The suspension was stirred 15min, filtered through Celite^{®}(filter cake rinsed with THF), and the filtrate concentrated *in vacuo*. The residue was dissolved in methylene chloride, loaded onto a silica gel column, and eluted with 3:1 methylene chloride/ethyl acetate to afford the intermediate dimethoxybenzyl bicyclic amine (280mg, 58%) as a colorless viscous oil.

A mixture of the intermediate dimethoxybenzyl bicyclic amine (280mg, 0.76 mmol) and anhydrous potassium carbonate (215mg, 1.55 mmol) in anhydrous methylene chloride (5mL) in a pressure tube equipped with a stirbar was treated with 1-chloroethyl chloroformate (0.221mL, 1.55 mmol), closed, and stirred at 45°C for 4h. The tube was cooled, opened, and the contents filtered (rinse with methylene chloride), and the filtrate concentrated *in vacuo*. The residue was dissolved in methanol (7mL), refluxed for 1h, cooled, treated with DOWEX^{®} 550A-OH resin (2.0g, prerinsed with methanol), stirred a few minutes, filtered, and the filtrate concentrated *in vacuo*. The residue was taken up in ether, filtered through Celite^{®}, and the filtrate treated with 2N HCl/ether (0.6mL, 1.2 mmol). The suspension was stirred a few minutes, the solid salt collected by filtration, rinsed with ether, and dried *in vacuo* to afford 1-(quinolin-3-yl)-3-azabicyclo- [3.1.0]hexane, hydrochloride (100mg, 47%) as a light beige solid. MS (M+1) 192.1. ¹H NMR (DMSO-*d*₆) δ 9.33 (s, 1H), 8.94 (s, 1H), 8.36 (d, 1H, *J*=8.5Hz), 8.17 (d, 1H, J=8Hz), 8.01 (t, 1H, *J*=7.5Hz), 7.86 (t, 1H, *J*=7.5Hz), 3.97 (dd, 1H, *J*=11Hz,5Hz), 3.42-3.86 (m, 3H), 2.24-2.43 (m, 1H), 1.74-2.14 (m, 1H) 1.44 (t, 1H, *J*=7.5Hz). ¹³C NMR (DMSO-*d*₆) δ ppm 147.12, 142.14, 134.04, 133.52, 129.91, 129.10,128.57, 123.24, 58.85, 56.63, 29.36, 24.70, 15.32.

### Synthesis of 6-fluoro-benzo[b]thiophene-2-carboxylic acid ethyl ester

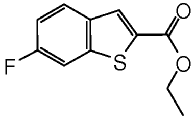

To a mixture of 2, 5-difluorobenzaldehyde (280 g, 1.97 mol), potassium carbonate (272 g, 1.97 mol) and anhydrous DMF (1260 ml) was added mercaptoacetic acid ethyl ester (236 g, 1.97 mol). The mixture was stirred at 90°C for 4 h. After cooling water (4200 ml) was added and the solution was extracted with ethyl acetate (3x1500 ml), the combined extracts dried over sodium sulfate and the solvent was removed to give crude product, which was flash-chromatographed (eluent - ethyl acetate (1):hexane (9). Yield of 6-fluoro-benzo[b]thiophene-2-carboxylic acid ethyl ester 96.49 g (21.8%) as a yellow solid. ¹H-NMR (400 MHz, CDCl₃); δ (ppm): 1.40 (t, 3H), 4.40 (q, 2H), 7.15 (t, 1H), 7.52 (d, 1H), 7.81(dd, 1H), 8.00 (s, 1H).

### Synthesis of 6-fluoro-benzo[b]thiophen-2-yl)-methanol

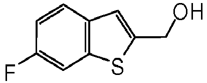

To a suspension of lithium aluminum hydride (15.5 g, 0.43 mol) in dry THF (600 ml) was added a solution of 6-fluoro-benzo[b]thiophene-2-carboxylic acid ethyl ester (96.49 g, 0.43 mol) in dry THF (960 ml), the mixture was stirred for 3 h at 20°C. The reaction was carefully quenched by the dropwise addition of water (15.5 ml), 15% NaOH (15.5 ml) and water (46.5 ml), stirred for 0.5 h, then filtered and washed with THF: water (1:1) (100 ml). The filtrate was concentrated to give 58.25 g (74 %) of 6-fluoro-benzo[b]thiophen-2-yl)-methanol as white powder. ¹H-NMR (400 MHz, DMSO-d6); δ (ppm): 4.67 (s, 2H), 5.51 (s, 1H), 7.17 (t, 1H), 7.22 (s, 1H), 7.72-7.80 (m, 2H).

### Synthesis of 2-chloromethyl-6-fluoro-benzo[b]thiophene

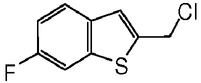

To a solution of 6-fluoro-benzo[b]thiophen-2-yl)-methanol (60.89 g, 0.33 mol) in dry dioxane (30 ml) was added thionyl chloride (39 ml, 0.53 mol) while keeping the temperature below 15°C and stirred at for 4 hours (TLC control). The mixture was evaporated at 40°C under reduced pressure to give 2-chloromethyl-6-fluoro-benzo[b]thiophene as white solid in essentially quantitative yield.

### Synthesis of 6-fluoro-benzo[b]thiophen-2-yl)-acetonitrile

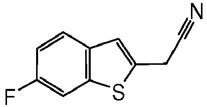

To a solution of NaCN (21.8 g, 0.446 mol) in DMSO (600 ml) was added portionwise 2-chloromethyl- 6-fluoro-benzo[b]thiophene (71.5g, 0.357 mol) while keeping the temperature below 15°C and stirred for 1.5 h at 40-45°C (TLC 7:3; Heptane: ethyl acetate), then diluted with cold water (2 L), which lead to the formation of a precipitate. The precipitate was filtered off, washed with water and dried in vacuo at 50 °C for 12 hours. The crude material was still very wet and dissolved in dichloromethane (1L) and dried over magnesium sulfate. The crude product was isolated by removal of the solvent under reduced pressure, yielding 28.1 g (41%) of 6-fluorobenzo[b]thiophen-2-yl)-acetonitrile as a light yellow powder. ¹H-NMR (400 MHz, CDCl₃); δ (ppm): 3.95 (s, 2H), 7.11 (t, 1H), 7.26 (s, 1H), 7.45 (d, 1H), 7.67 (dd, 1H).

### Synthesis of (1S,5S)-1-(6-fluoro-benzo[b]thiophen-2-yl)-3-aza-bicyclo[3.1.0]hexane hydrochloride (scheme 4)

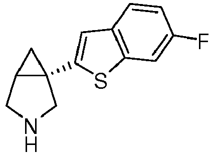

To a stirring solution of 6-fluoro-benzo[b]thiophen-2-yl)-acetonitrile (8 g, 0.0418 mol) in anhydrous THF (42 ml) at -30°C under nitrogen was added 20.9 ml (0.0418 mmol) of NaHMDS (2M in THF) slowly while keeping the temperature below -20°C. The resulting mixture was stirred for 0.5 hr between -15°C and -25°C. (S)-(+)-Epichlorohydrin (5.03 g, 0.0418 mol) was added at-25°C, the mixture was stirred between -15°C and -25°C for 0.5 h then NaHMDS (25.1 ml, 0.0502 mol) was added at -25°C. The mixture was stirred for 1 hr between -10° and -20°C, then for 2 hr between -10° and 5°C, quenched with 2N HCl (60 ml) and the layers were separated. The aqueous layer was re-extracted with EtOAc (1x50 ml). The organics were combined, washed with saturated NaCl (50 ml), dried over Na₂SO₄, filtered and concentrated to provide crude product as a brown oil.

The brown oil was dissolved in THF (42 ml) and 10 M borane dimethylsulfide (9.3 ml, 0.0836) was added dropwise via syringe. The mixture was heated to reflux for 2 hr. The mixture was cooled to <40 °C and quenched with 4N HCl (85 ml). The organic portion was separated and discarded. The aqueous portion was re-extracted with ethyl acetate (3 x 50 ml) and organic portion discarded. The aqueous portion was basified with ammonium hydroxide (35 ml) and then extracted with ethyl acetate (2 x 50 ml). The combined organic portion was washed with 5% sodium phosphate dibasic (1 x 50 ml) and brine (1 x 50 ml). The organic portion was dried over magnesium sulfate, filtered and concentrated under reduced pressure to a yellow oil (5.24 g). The oil was purified by column chromatography with dichloromethane (93): MeOH (6):NH₄O (1) and the major product (*cis* isomer) was isolated (1S, 2S)-[2-aminomethyl-2-(6-fluoro-benzo[b]thiophen-2-yl)-2-cyclopropyl]-methanol 2.13 g (20%) as an off white solid. MS *m*/*z* 252 (MH+).

The hydrochloride salt was prepared with [2-aminomethyl-2-(6-fluoro-benzo[b]thiophen-2-yl)-2-cyclopropyl]-methanol (170 mg) by dissolving in isopropanol (2 ml) and adding 5M isopropanolic HCl (2 ml) and blowing dry with nitrogen. The hydrochloride salt was dried in vacuo at 50 °C for 12 hours to yield (1S, 2S)-[2-aminomethyl-2-(6-fluoro-benzo[b]thiophen-2-yl)-2-cyclopropyl]-methanol hydrochloride which was 98 8% pure by HPLC. MS *m*/*z* 252 (MH+). 1H NMR (400 MHz, DMSO-*d*₆) δ ppm 1.03 (d) 1.19-1.25 (m) 1.23 (t) 1.20 - 1.25 (m) 1.20 - 1.25 (m) 1.28 (dd) 1.59 - 1.70 (m) 3.20 - 3.39 (m) 3.42 (dd) 3.89 (dd) 7.23 (td) 7.77 (dd) 7.84 (dd) 8.07 (br. s.)

To a stirring solution of [2-aminomethyl-2-(6-fluoro-benzo[b]thiophen-2-yl)-2-cyclopropyl]- methanol (1.96 g, 7.8 mmol) in dry dioxane (130 ml) at 15°C was added slowly thionyl chloride (1.88 ml, 25.7 mmol). After 2.5 hr at room temperature TLC (9:1; Dichloromethane: methanol) showed the reaction was complete. The mixture was evaporated at 40°C, dissolved in dichloromethane (200 ml) and stirred vigorously with aqueous sodium carbonate until LC/MS showed that the compound was completely cyclized (0.5 hr). The layers were separated and the organic portion dried over magnesium sulfate and concentrated under reduced pressure. The crude product was suspended in diethyl ether (100 ml), added 5M isopropanolic HCl (5 ml), and stirred for 30 min. The precipitate was filtered, washed with diethyl ether to give (1S, 5S)-1-(6-fluoro-benzo[b]thiophen-2-yl)-3-aza-bicyclo[3.1.0]hexane hydrochloride as an off white solid. Yield 1.2 g (57%) and 100 % pure by HPLC. Chiral Purity 100 %. MS *m*/*z* 234 (MH+) ¹H-NMR (400 MHz, DMSO-d6); δ (ppm):1.32 (t, 1H), 1.60 (t, 1H), 2.11 (m, 1H), 3.30-3.75 (m, 4H), 7.22 (t, 1H), 7.35 (s, 1H), 7.76 (dd, 1H), 7.82 (d, 1H), 9.80 (br. s, 2H). ¹³C NMR (101 MHz, DMSO-*d*₆) d ppm 16.39, 25.98, 27.63, 49.15, 108.49, 108.75, 113.23, 113.47, 120.88, 124.36, 124.45, 136.34, 138.91, 139.02, 143.63, 143.66, 158.38, 160.78.

### Synthesis of (1R, 5R)-1-(6-fluoro-benzo[b]thiophen-2-yl)-3-aza-bicyclo[3.1.0]hexane hydrochloride (scheme 5)

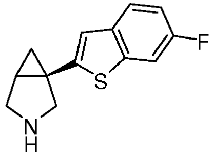

### Procedure as described above except (R)-(-)-epichlorohydrin used.

(1R, 2R)-[2-aminomethyl-2-(6-fluoro-benzo[b]thiophen-2-yl)-2-cyclopropyl]-methanol 2.13 g (20%) as an off white solid. MS *m*/*z* 252 (MH+). The hydrochloride salt was prepared with (1R, 2R)-[2-aminomethyl-2-(6-fluoro-benzo[b]thiophen-2-yl)-2-cyclopropyl]-methanol (111 mg) by dissolving in isopropanol (2 ml) and adding 5M isopropanolic HCl (2 ml) and blowing dry with nitrogen. The hydrochloride salt was dried in vacuo at 50 °C for 12 hours to yield (1R,2R)-[2-aminomethyl-2-(6-fluoro-benzo[b]thiophen-2-yl)-2-cyclopropyl]-methanol hydrochloride which was 98.4 % pure by HPLC. MS *mlz* 252 (MH+). 1H NMR (400 MHz, DMSO-*d*₆) δ ppm 1.03 (d) 1.19 - 1.25 (m) 1.23 (t) 1.20 - 1.25 (m) 1.20 - 1.25 (m) 1.28 (dd) 1.59 - 1.70 (m) 3.20 - 3.39 (m) 3.42 (dd) 3.89 (dd) 7.23 (td) 7.77 (dd) 7.84 (dd) 8.07 (br. s.)

(1R, 5R)-1-(6-fluoro-benzo[b]thiophen-2-yl)-3-aza-bicyclo[3.1.0]hexane hydrochloride as an off white solid. Yield 1.0 g (48%) and 100 % pure by HPLC. Chiral purity 99.6%. MS *m*/*z* 234 (MH+). ¹H-NMR (400 MHz, DMSO-d6); δ (ppm):1.32 (t, 1H), 1.60 (t, 1H), 2.11 (m, 1H), 3.30-3.75 (m, 4H), 7.22 (t, 1H), 7.35 (s, 1H), 7.76 (dd, 1H), 7.82 (d, 1H), 9.80 (br. s, 2H). ¹³C NMR (101 MHz, DMSO-*d*₆) d ppm 16.39, 25.98, 27.63, 49.15, 108.49, 108.75, 113.23, 113.47, 120.88, 124.36, 124.45, 136.34, 138.91, 139.02, 143.63, 143.66, 158.38, 160.78.

### 1. Preparation of 6-bromo-benzo[b]thiophene-2-carboxylic acid ethyl ester

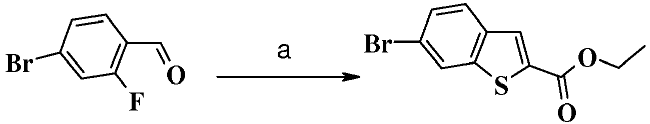

### a) Ethyl thioglycolate, Et₃N, DMSO, 95°C, 2 h

The substance was obtained according to the ***general procedure**,* yielding the title compound as a light yellow solid (70%).
¹H-NMR (400 MHz, DMSO-d₆); δ (ppm): 1.34 (t, 3H), 4.34 (dd, 2H), 7.62 (d, 1H), 7.96 (d, 1H), 7.18 (s, 1H), 7.37 (s, 1H)

### 2. Preparation of 5-bromo-benzo[b]thiophene-2-carboxylic acid ethyl ester

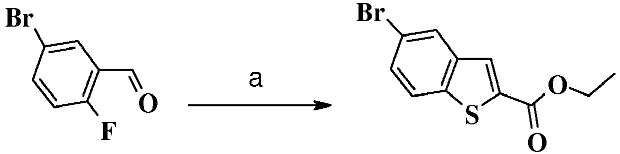

### a) Ethyl thioglycolate, Et₃N, DMSO, 95°C, 2 h

The substance was obtained according to the ***general procedure**,* yielding the title compound as a off-white crystals (75%).
¹H-NMR (400 MHz, DMSO-d₆); δ (ppm): 1.33 (t, 3H), 4.36 (dd, 2H), 7.63 (d, 1H), 8.04 (d, 1H), 7.13 (s, 1H), 7.25 (s, 1H)

### 3. Preparation of (6-bromo-benzo[b]thiophen-2-yl)-methanol

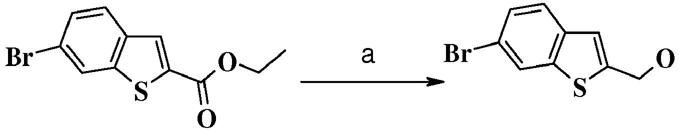

### a) tetrahydrofuran, LiBH₄, 60°C, 3 h

The substance was obtained according to the ***general procedure**,* yielding the title compound as a white solid (96%)

### 4. Preparation of (5-bromo-benzo[b]thiophen-2-yl)-methanol

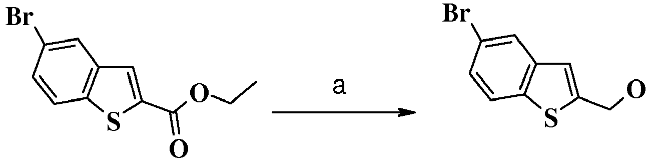

### a) Tetrahydrofuran, LiBH₄, 60°C, 3 h

The substance was obtained according to the ***general procedure**,* yielding the title compound as a white solid (73%)

### 5. Preparation of (6-bromo-benzo[b]thiophen-2-yl)-acetonitrile

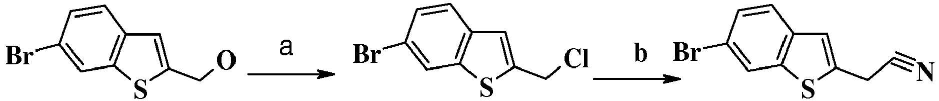

SOCl₂, dichloromethane, RT, 2 h

### b) NaCN, acetonitrile, 18-crown-6, RT, overnight

The substance was obtained according to the ***general procedure**.* Overall yield of (6-bromo-benzo[b]thiophen-2-yl)-acetonitrile (30 % on three previous steps). Light yellow solid.
¹H-NMR (400 MHz, DMSO-d₆); δ (ppm): 4.39 (s, 2H), 7.4 (s, 1H), 7.53 (d, 1H), 7.78 (d, 1H), 8.23 (s, 1H)

### 6. Preparation of (5-bromo-benzo[b]thiophen-2-yl)-acetonitrile

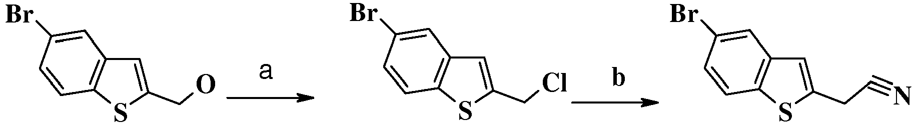

SOCl₂, dichloromethane, RT, 2 h

### b) NaCN, acetonitrile, 18-crown-6, RT, overnight

The substance was obtained according to the ***general procedure**.* Overall yield of (6-bromo-benzo[b]thiophen-2-yl)-acetonitrile (26 % on three previous steps). Light yellow solid.
¹H-NMR (400 MHz, DMSO-d₆); δ (ppm): 4.43 (s, 2H), 7.39 (s, 1H), 7.48 (d, 1H), 7.9 (d, 1H), 8.06 (s, 1H)

### 7. Preparation of 1-(6-bromo-benzo[b]thiophen-2-yl)-2-hydroxymethyl-cyclopropanecarbonitrile and 5-(6-bromo-benzo[b]thiophen-2-yl)-3-oxa-bicyclo[3.1.0]hexan-2-one

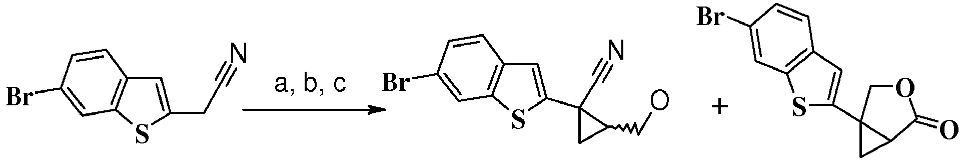

a) NaHMDS, THF, -20°C, 1 h
b) Epibromohydrin, -20°C, 1 h
c) NaHMDS, THF, from -20°C to 5 °C, overnight

The substances were obtained according to the ***general procedure***.

Yield of the lactone 17%. Light brown crystalline solid.

Yield of the nitrile 39%. Dark brown liquid.

### 8. Preparation of 1-(5-bromo-benzo[b]thiophen-2-yl)-2-hydroxymethyl-cyclopropanecarbonitrile and 5-(5-bromo-benzo[b]thiophen-2-yl)-3-oxa-bicyclo[3.1.0]hexan-2-one

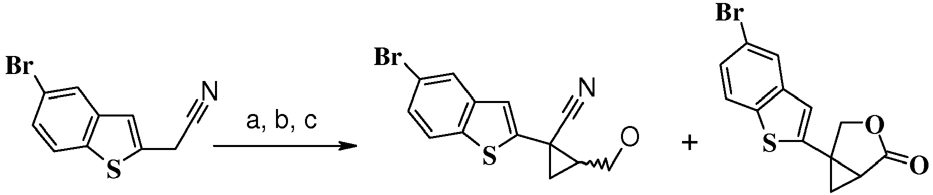

a) NaHMDS, THF, -20°C, 1 h
b) Epibromohydrin, -20°C, 1 h
c) NaHMDS, THF, from -20°C to 5 °C, overnight

The substances were obtained according to the ***general procedure**.*

Yield of the lactone 20%. Light brown crystalline solid.

CI MS *m*/*z* 308.8, 310.78 (MH+).

Yield of the nitrile 37%. Dark brown liquid.

CI MS *m*/*z* 307.89, 309.86 (MH+).

### 9. Preparation of -2-aminomethyl-2-(6-bromo-benzo[b]thiophen-2-yl)-cyclopropyl]-methanol

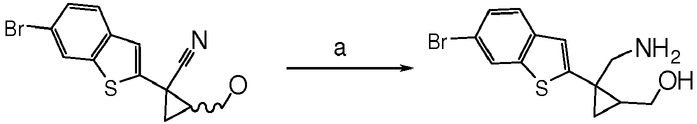

### a) BH₃-THF complex, from RT to 50°C, 1.5 h

The substances were obtained according to the ***general procedure**,* yielding the title compound as a light yellow liquid (42%).

### 10. Preparation of 2-aminomethyl-2-(5-bromo-benzo[b]thiophen-2-yl)-cyclopropyl]-methanol

### Method A:

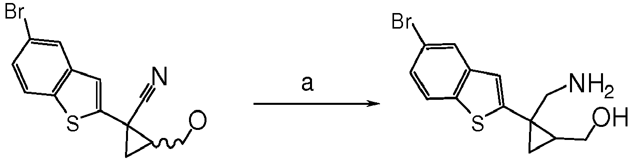

BH₃-THF complex, from RT to 50°C, 1.5 h

The substance was obtained according to the ***general procedure**,* yielding the title compound as a light yellow liquid (40%).

### Method B:

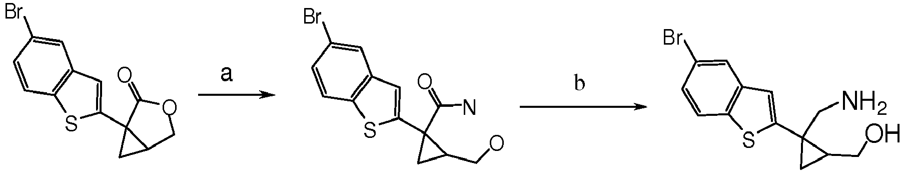

a) NH₃/MeOH, 50°C, 12h
b) BH₃-THF complex, reflux, 8 h

The substance was obtained according to the ***general procedure**.* Overall yield of the title compound 41%. A light yellow liquid
CI MS *m*/*z* 311.89, 313.87 (MH+).

### 11. Preparation of 1-(6-bromo-benzo[b]thiophen-2-yl)-3-aza-bicyclo[3.1.0]hexane

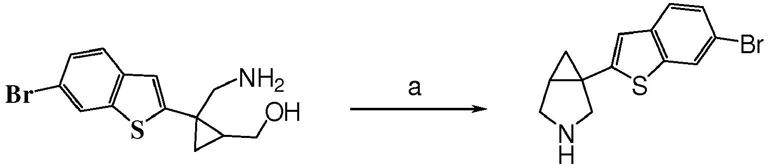

SOCl₂, dioxane, from 0°C to RT, 1.5 h

The substance and its hydrochloride salt were obtained according to the ***general procedure**.* Overall yield of the title compound as a white crystalline solid (60%).
CI MS *m*/*z* 293.97, 295.95 (MH+).
¹H-NMR (400 MHz, DMSO-d₆); δ (ppm):1.33 (t, 1H), 1.61 (t, 1H), 2.15 (m, 1H), 3.35-3.76 (m, 4H), 7.38 (s, 1H), 7.51 (d, 1H), 7.69 (d, 1H), 8.2 (s, 1H), 9.80 (br. s, 2H).
HPLC (Column C18 3.9x150 mm 5mkm) - mobile phase: acetonitrile/water+TFA 5/95 (0min)-100/0(18min)-100/0(25min); flow 1.0 ml/min; RT; Detection UV at 220 nm: First Peak with Retention Time of 10.123 min, area 98.9%; Second Peak with Retention Time of 10.892 min, area 0.6%; Third Peak with Retention Time of 11.058, area 04%; Fourth Peak with Retention Time of 11.752, area 01%.

### 12. Preparation of 1-(5-bromo-benzo[b]thiophen-2-yl)-3-aza-bicyclo[3.1.0]hexane

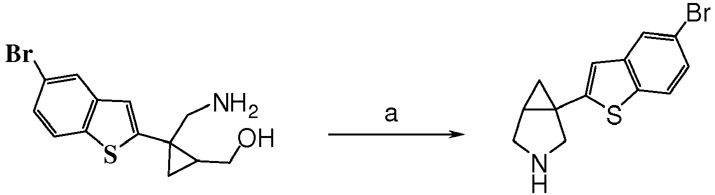

SOCl₂, dioxane, from 0°C to RT, 1.5 h

The substance and its hydrochloride salt were obtained according to the ***general procedure**.* Overall yield of the title compound as a white crystalline solid (72%).
CI MS *m*/*z* 293.96, 295.94 (MH+).
¹H-NMR (400 MHz, DMSO-d₆); δ (ppm):1.35 (t, 1H), 1.69 (t, 1H), 2.15 (m, 1H), 3.33-3.80 (m, 4H), 7.35 (s, 1H), 7.44 (d, 1H), 7.87 (d, 1H), 7.94 (s, 1H), 9.70 (br. s, 2H).
HPLC (Column C18 3.9x150 mm 5mkm) - mobile phase: acetonitrile/water+TFA 5/95 (0min)-100/0(18min)-100/0(25min); flow 1.0 ml/min; RT; Detection UV at 254 nm: First Peak with Retention Time of 9.127 min, area 1.8%; Second Peak with Retention Time of 10.113 min, area 98.2%

### 13. Preparation of 1-(6-bromo-benzo[b]thiophen-2-yl)-3-methyl-3-aza-bicyclo[3.1.0]hexane

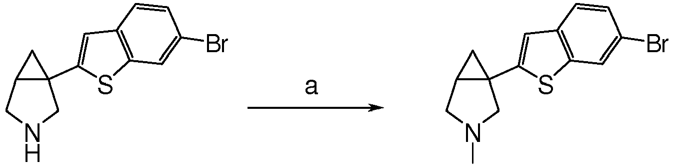

40% CH₂O aqueous solution, Et₃N, CH₃COOH, NaBH(OAc)₃, CH₃CN, RT, overnight

The substance and its hydrochloride salt were obtained according to the ***general procedure**.* Overall yield of the title compound as a white crystalline solid (80%).
CI MS *m*/*z* 307.95, 309.95 (MH+).
¹H-NMR (400 MHz, DMSO-d₆); δ (ppm):1.31 (s, 1H), 1.97 (s, 1H), 2.24 (m, 1H), 2.82 (t, 3H), 3.45-3.98 (m, 4H), 7.35 (s, 1H), 7.50 (d, 1H), 7.70 (d, 1H), 8.20 (s, 1H), 11.30 (br. s, 1H).
HPLC (Column C18 3.9x150 mm 5mkm) - mobile phase: acetonitrile/water+TFA 5/95 (0min)-100/0(18min)-100/0(25min); flow 1.0 ml/min; RT; Detection UV at 254 nm: First Peak with Retention Time of 9.294 min, area 0.13%; Second Peak with Retention Time of 10.231 min, area 99.87%.

### 14. Reparation of 1-(5-bromo-benzo[b]thiophen-2-yl)-3-methyl-3-aza-bicyclo[3.1.0]hexane

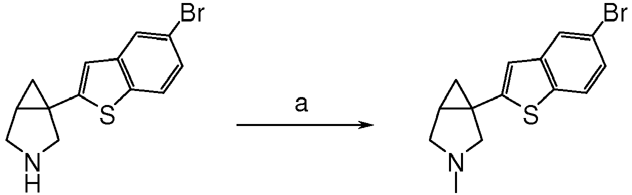

40% CH₂O aqueous solution, Et₃N, CH₃COOH, NaBH(OAc)₃, CH₃CN, RT, overnight

The substance and its hydrochloride salt were obtained according to the ***general procedure**.* Overall yield of the title compound as a white crystalline solid (75%).
CI MS *m*/*z* 307.96, 309.95 (MH+).
¹H-NMR (400 MHz, DMSO-d₆); δ (ppm):1.30 (s, 1H), 1.96 (s, 1H), 2.18 (m, 1H), 2.82 (t, 3H), 3.46-4.00 (m, 4H), 7.33 (s, 1H), 7.44 (d, 1H), 7.87 (d, 1H), 7.86 (s, 1H), 11.25 (br. s, 1H).
HPLC (Column C18 3.9x150 mm 5mkm) - mobile phase: acetonitrile/water+TFA 5/95 (0min)-100/0(18min)-100/0(25min); flow 1.0 ml/min; RT; Detection UV at 254 nm: First Peak with Retention Time of 9.242 min, area 1.4%; Second Peak with Retention Time of 10.169 min, area 98.6%

### 15. Preparation of 1-(6-bromo-benzo[b]thiophen-2-yl)-3-aza-bicyclo[3.1.0]hexane-3-carboxylic acid tert-butyl ester

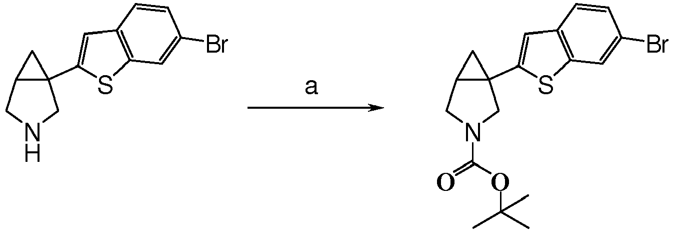

Boc₂O, dicloromethane, RT, overnight

The substance was obtained according to the ***general procedure*** and was used on the next step without any purification.

### 16. Preparation of 1-(5-Bromo-benzo[b]thiophen-2-yl)-3-aza-bicyclo[3.1.0]hexane-3-carboxylic acid tert-butyl ester

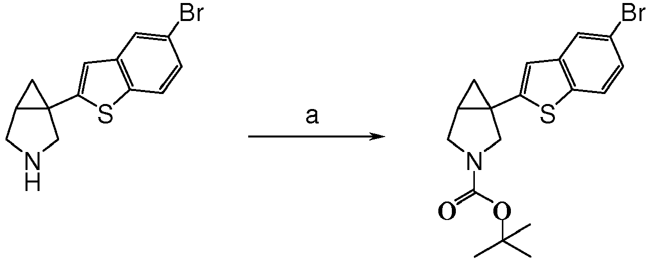

Boc₂O, dicloromethane, RT, overnight

The substance was obtained according to the ***general procedure*** and was used on the next step without any purification.

### 17. Preparation of [2-(3-Aza-bicyclo[3.1.0]hex-1-yl)-benzo[b]thiophen-6-yl]-dimethyl-amine dihydrochloride

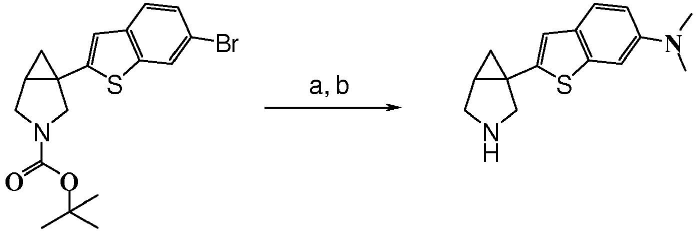

Dimethylamine solution in THF (20%), NaO(i-Am), bis(tri-*tert*-butylphosphine)palladium(0) 6-8 mole%, microwave irradiation at 100°C, 3 h.
5-6 M HCl solution in isopropanol
Boc derivative and the title compound was obtained according to the ***general procedure**.*
Yield of the Boc-derivative (87%). Light yellow liquid.
Yield of the title compound (86%). White crystalline solid.
CI MS *m*/*z* 259.17 (MH+).
¹H-NMR (400 MHz, DMSO-d₆); δ (ppm):1.34 (t, 1H), 1.64 (t, 1H), 2.12 (m, 1H), 3.07 (s, 6H), 3.32-3.72 (m, 4H), 7.31 (s, 1H), 7.46 (s, 1H), 7.75 (d, 1H), 7.94 (m, 1H), 9.78 (br. s, 1H), 10.08 (br. s, 1H)
HPLC (Column C18 3.9x150 mm 5mkm) - mobile phase: acetonitrile/water+TFA 5/95 (0min)-100/0(18min)-100/0(25min); flow 1.0 ml/min; RT; Detection UV at 254 nm: First Peak with Retention Time of 5.348 min, area 96.9%; Second Peak with Retention Time of 8.537 min, area 2.4%; Third Peak with Retention Time of 9.162 min, area 08%.

### 18. Preparation of [2-(3-aza-bicyclo[3.1.0]hex-1-yl)-benzo[b]thiophen-5-yl]-dimethyl-amine dihydrochloride

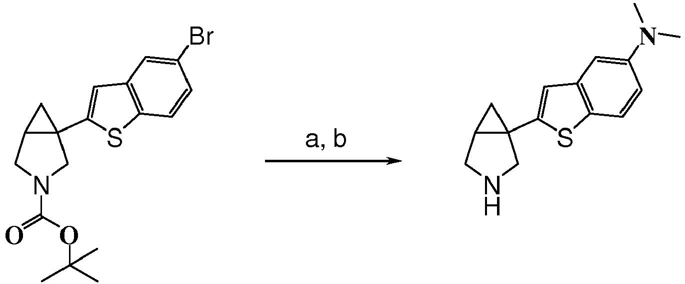

Dimethylamine solution in THF (20%), NaO(i-Am), bis(tri-*tert*-butylphosphine)palladium(0) 6-8 mole%, microwave irradiation at 100°C, 3 h.
5-6 M HCl solution in isopropanol

The Boc derivative and the title compound were obtained according to the ***general procedure**.*
Yield of the Boc-derivative (63%). Light yellow liquid.
Yield of the title compound (80%). White crystalline solid.
CI MS *m*/*z* 259.00 (MH+).
¹H-NMR (400 MHz, DMSO-d₆); δ (ppm):1.35 (t, 1H), 1.68 (s, 1H), 2.17 (m, 1H), 3.12 (s, 6H), 3.33-3.72 (m, 4H), 7.4 (s, 1H), 7.54 (s, 1H), 7.88 (m, 1H), 8.00 (d, 1H), 9.80 (br. s, 1H), 10.05 (br. s, 1H)
¹³C -NMR (100MHz, DMSO-d6) RT; δ (ppm): 16.71, 26.14, 27.59 , 44.63, 46.41, 49.05, 113.20, 115.62, 121.07, 123.42, 135.75, 139.93, 142.23, 146.22.
HPLC (Column C18 3.9x150 mm 5mkm) - mobile phase: acetonitrile/water+TFA 5/95 (0min)-100/0(18min)-100/0(25min); flow 1.0 ml/min; RT; Detection UV at 220 nm: First Peak with Retention Time of 5.404 min, area 95.9%; Second Peak with Retention Time of 8.992 min, area 4.1%.

### 19. Preparation of dimethyl-[2-(3-methyl-3-aza-bicyclo[3.1.0]hex-1-yl)-benzo[b]thiophen-6-yl]-amine dihydrochloride

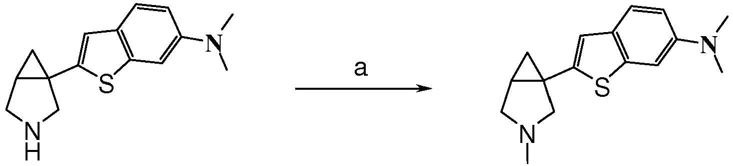

40% CH₂O aqueous solution, Et₃N, CH₃COOH, NaBH(OAc)₃, CH₃CN, RT, overnight

The title compound was obtained according to the ***general procedure**,* yielding the title compound as a white crystalline solid (77%).
CI MS *m*/*z* 273.00 (MH+).
¹H-NMR (400 MHz, DMSO-d₆); δ (ppm):1.30 (t, 1H), 2.02 (m, 1H), 2.17 (m, 1H), 2.84 (s, 3H), 3.08 (s, 6H), 3.5-3.97 (m, 4H), 7.4 (s, 1H), 7.43 (s, 1H), 7.75 (d, 1H), 7.90 (m, 1H), 11.40 (br. s, 1H)
HPLC (Column C18 3.9x150 mm 5mkm) - mobile phase: acetonitrile/water+TFA 5/95 (0min)-100/0(18min)-100/0(25min); flow 1.0 ml/min; RT; Detection UV at 254 nm: First Peak with Retention Time of 5.383 min, area 96.5%; Second Peak with Retention Time of 8.813 min, area 2.1%; Third Peak with Retention Time of 16.781, area 1.4%.

### 20. Preparation of [2-(3-isopropyl-3-aza-bicyclo[3.1.0]hex-1-yl)-benzo[b]thiophen-6-yl]-dimethyl-amine dihydrochloride

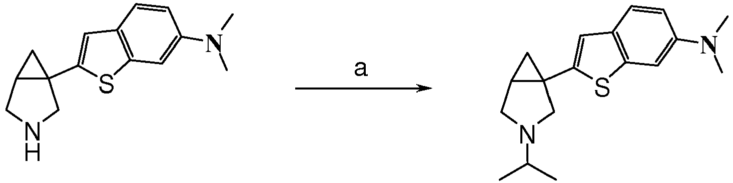

acetone, Et₃N, CH₃COOH, NaBH(OAc)₃, dichloroethane, RT, overnight

The title compound was obtained according to the ***general procedure**,* yielding the title compound as a white crystalline solid (75%).
CI MS *m*/*z* 301.04 (MH+).
¹H-NMR (400 MHz, DMSO-d₆); δ (ppm):1.20-1.38 (m, 7H), 2.05-2.02 (m, 2H), 3.04 (s, 6H), 3.15-4.00 (m, 4H), 7.28-7.48 (m, 2H), 7.67-7.94 (m, 2H), 11.35 (br. s, 1H)
HPLC (Column C18 3.9x150 mm 5mkm) - mobile phase: acetonitrile/water+TFA 5/95 (0min)-100/0(18min)-100/0(25min); flow 1.0 ml/min; RT; Detection UV at 254 nm: First Peak with Retention Time of 5.946 min, area 98.0%; Second Peak with Retention Time of 9.650 min, area 2.0%.

### 21. Preparation [2-(3-isopropyl-3-aza-bicyclo[3.1.0]hex-1-yl)-benzo[b]thiophen-5-yl]-dimethyl-amine dihydrochloride

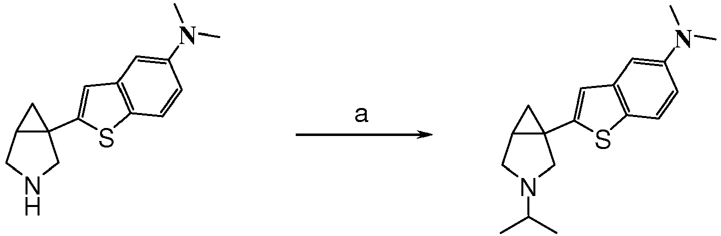

acetone, Et₃N, CH₃COOH, NaBH(OAc)₃, dichloroethane, RT, overnight

The title compound was obtained according to the ***general procedure**,* yielding the title compound as a white crystalline solid (65%).
CI MS *m*/*z* 301.07 (MH+).
¹H-NMR (400 MHz, DMSO-d₆); δ (ppm):1.20-1.42 (m, 8H), 2.02 (s, 1H), 3.04 (s, 6H), 3.48-4.05 (m, 4H), 7.38-7.59 (m, 2H), 7.75-7.99 (m, 2H), 11.28 (br. s, 1H)
HPLC (Column C18 3.9x150 mm 5mkm) - mobile phase: acetonitrile/water+TFA 5/95 (0min)-100/0(18min)-100/0(25min); flow 1.0 ml/min; RT; Detection UV at 254 nm: First Peak with Retention Time of 5.888 min, area 97.9%; Second Peak with Retention Time of 9.917 min, area 2.1%.

### 22. Preparation of [2-(3-ethyl-3-aza-bicyclo[3.1.0]hex-1-yl)-benzo[b]thiophen-6-yl]-dimethyl-amine dihydrochloride

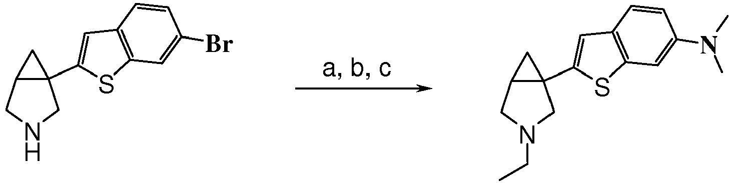

acetyl chloride, Et₃N, methylene chloride, RT, 20 min

Dimethylamine solution in THF (20%), NaO(i-Am), bis(tri-*tert*-butylphosphine)palladium(0) 6-8 mole%, microwave irradiation at 100°C, 3 h.
LiAlH₄, THF, RT, 2 h

The title compound was obtained according to the ***general procedure**,* yielding the title compound as a white crystalline solid (62%).
CI MS *m*/*z* 287.13 (MH+).
¹H-NMR (400 MHz, DMSO-d₆); δ (ppm):1.02-1.50 (m, 7H), 2.12 (m, 2H), 3.03 (s, 6H), 3.31-4.1 (m, 4H), 7.28-7.51 (m, 2H), 7.69-7.98 (m, 2H), 11.28 (br. s, 1H)
HPLC (Column C18 3.9x150 mm 5mkm) - mobile phase: acetonitrile/water+TFA 5/95 (0min)-100/0(18min)-100/0(25min); flow 1.0 ml/min; RT; Detection UV at 254 nm: First Peak with Retention Time of 4.079 min, area 96.2%; Second Peak with Retention Time of 8.990 min, area 3.8%.

### 23. Preparation [2-(3-Ethyl-3-aza-bicyclo[3.1.0]hex-1-yl)-benzo[b]thiophen-5-yl]-dimethyl-amine dihydrochloride ASE 22130163

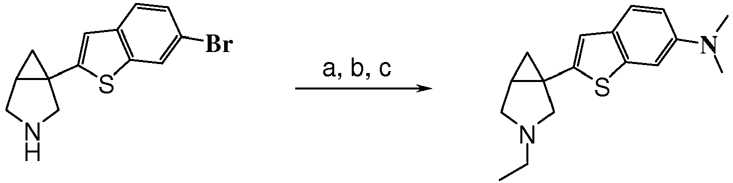

acetyl chloride, Et₃N, methylene chloride, RT, 20 min

Dimethylamine solution in THF (20%), NaO(i-Am), bis(tri-*tert*-butylphosphine)palladium(0) 6-8 mole%, microwave irradiation at 100°C, 3 h.
LiAlH₄, THF, RT, 2 h

The title compound was obtained according to the ***general procedure**,* yielding the title compound as a white crystalline solid (68%).
CI MS *m*/*z* 287.03 (MH+).
¹H-NMR (400 MHz, DMSO-d₆); δ (ppm):1.22-1.34 (m, 5H), 2.04 (d, 1H), 2.18 (s, 1H), 3.04 (s, 6H), 3.15-3.98 (m, 4H), 7.36 (s, 1H), 7.49 (s, 1H), 7.73-7.92 (m, 1H), 7.98 (d, 1H), 11.38 (br. s, 1H)

### Example VI

### Activity, Selectivity, and Potency of 1-heteroaryl-3-azabicyclo[3.1.0]hexanes for Inhibiting Monoamine Neurotransmitter Transport

The ability of the 1-heteroaryl-3-azabicyclo[3.1.0.]hexane of the invention for inhibiting transport of norepinephrine (NE) and/or dopamine (DA) and/or serotonin (5-HT) were evaluated using cell lines (such as the HEK 293 cell line) that stably express recombinant human NE, DA, and 5-HT transporters, respectively. The techniques for stably transfecting mammalian cell lines with neurotransmitter transporters and measuring the effects of drugs on amine uptake have been described in the literature (e.g., Gu, H., et al. J. Biol. Chem. 269:7124-7130, 1994; Eshleman AJ, J Pharmacol Exp Ther 289:877-885, 1999) and well known to those skilled in the art.

A basic technique for measuring the ability of a compound to inhibit [³H]NE, DA, and 5-HT uptake in cell lines recombinantly expressing human transporters has been described (Eshleman, et al., 1999). In brief, cell suspensions (HEK 293 cells expressing the 5-HT transporter; MDCK cells expressing the NE transporter, and CHO-K1 cells expressing the DA transporter, respectively) can be prepared for assay by removing the medium from cells grown on tissue culture dishes, then washing the plates with buffered medium. Cells can then be gently scraped from the plates and cell clusters separated by trituration with a pipette for 5-10 aspiration/ejection cycles. To these cell suspensions were added test drugs (final concentrations generally from 0.01-5 µM) and Tris/Hepes buffer, pH 7.1 (also containing 120 mM NaCl, 1.2 mM CalCl₂, 1.2 mM MgSO₄, 5 mM D-glucose, and 1 mM ascorbic acid). Following a preincubation of the isolated cells at 25°C, either [³H]DA, [³H]5-HT, or [³H]NE was added and the incubation continued an additional 10 minutes. Radiolabelled neurotransmitter uptake was terminated by vacuum filtration and washing through Whatman glass fiber filters (GF/C), and the radioactivity retained on the filters was measured by liquid scintillation spectrometry. Specific uptake was defined as the difference in uptake observed in the absence and presence of a large molar excess of nomifensine, desipramine, or fluoxetine (for [³H]DA, [³H]NE or [³H]5-HT, respectively].

The results of these assays are shown in Table 2, below, which indicates, for each of the exemplary compounds, the structure of the substituent, and levels of observed uptake inhibition percentage for each of the indicated neurotransmitters at a fixed concentration of compound, or the IC₅₀ value, defined as that concentration of compound necessary to inhibit uptake by 50%. These are standard terms known to those skilled in the art.

**Table 2**

| **Inhibition of Biogenic Amine Uptake By Exemplary Substituted 1-Aryl-3-Azabicyclo[3.1.0.]hexanes** | | | | | | |
|---|---|---|---|---|---|---|
| **Structure** | **Uptake inh.% at 1 µM NE** | **Uptake inh.% at 1 µM 5-HT** | **Uptake inh.% at 1 µM DA** | **Uptake IC50 (nM) NE** | **Uptake IC50 (nM) 5-HT** | **Uptake IC50 (nM) DA** |
| 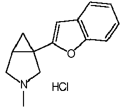 | | | | 400 | 340 | 630 |
| 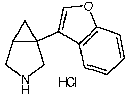 | 77 | 57 | 63 | 82 | 188 | 770 |
| 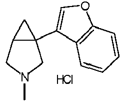 | 82 | 45 | 67 | 138 | 1450 | 475 |
| 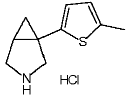 | 59 | 35 | 20 | | | |
| 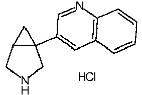 | 64 | 14 | 10 | | | |
| 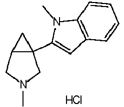 | 35 | 64 | 19 | | | |
| 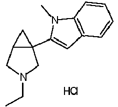 | 29 | 65 | 32 | | | |

| **Structure** | **Uptake inh.% at 1 µM NE** | **Uptake inh.% at 1 µM 5-HT** | **Uptake inh.% at 1 µM DA** | **Uptake IC50 (nM) NE** | **Uptake IC50 (nM) 5-HT** | **Uptake IC50 (nM) DA** |
|---|---|---|---|---|---|---|
| 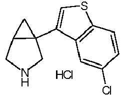 | 50 | 18 | 13 | | | |
| 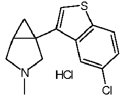 | 39 | 50 | 1 | | | |
| 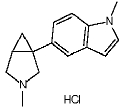 | 53 | 71 | 45 | 186 | 154 | 811 |
| 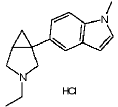 | 39 | 59 | 65 | 138 | 308 | 215 |
| 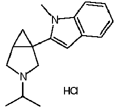 | 13 | 83 | 7 | | | |
| 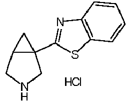 | 72 | 7 | 5 | | | |
| 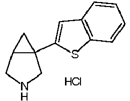 | 90 | 66 | 91 | 20 | 88 | 82 |
| | | | | | | |
| 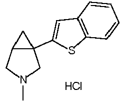 | 71 | 92 | 88 | 60 | 34 | 98 |
| 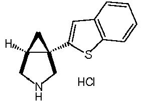 | 97 | 96 | 90 | 14 | 193 | 52 |
| 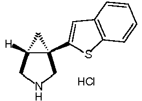 | 84 | 76 | 90 | 99 | 481 | 149 |
| 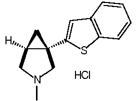 | 89 | 99 | 82 | 71 | 54 | 224 |
| 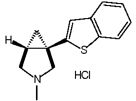 | 75 | 89 | 83 | 107 | 263 | 195 |
| 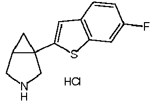 | 98 | 100 | 99 | | | |
| 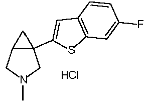 | 88 | 99 | 94 | | | |
| 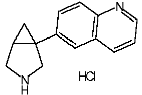 | 68 | 63 | 17 | | | |
| 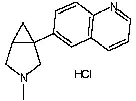 | 13 | 93 | 12 | | | |
| 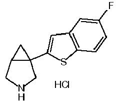 | 91 | 98 | 78 | | | |
| 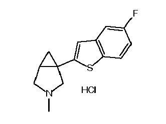 | 70 | 94 | 63 | | | |

Readily discernable from the foregoing results is the high degree of diversity with respect to the biological activity changes that were achieved by differentially altering N-substituents to yield novel 1-heteroaryl-3-azabicyclo[3.1.0.]hexanes according to the invention--whereby the absolute potency at any one transporter may be altered dramatically, and in distinct patterns among the exemplified compounds. These different relative potencies at the three transporters yield profound and distinct therapeutic potentials among the different, novel compounds of the invention. Both the absolute changes in potency and the changes in relative potency among the three transporters described herein for exemplary compounds of the invention would not have been expected or predictable with a reasonable expectation of success by persons of ordinary skill in the art

The data provided in Table 2 demonstrate that several of the 1-heteroaryl-3-azabicyclo[3.1.0.]hexanes of the invention are potent (nM) inhibitors of norepinephrine and/or serotonin and/or dopamine uptake. As such, the compounds and related formulations and methods of the invention provide neurobiologically active tools for modulating biogenic amine transport in mammalian subjects. These subjects may include *in vitro* or *ex vivo* mammalian cell, cell culture, tissue culture, or organ explants, as well as human and other mammalian individuals presenting with, or at heightened risk for developing, a central nervous system (CNS) disorder, such as pain, anxiety, or depression.

In certain embodiments, neurobiologically active compositions comprising a 1-heteroaryl-3-azabicyclo[3.1.0.]hexane of the invention are effective to inhibit cellular uptake of norepinephrine in a mammalian subject. In other embodiments, these compositions will effectively inhibit cellular uptake of serotonin in mammals. Other compositions of the invention will be effective to inhibit cellular uptake of dopamine in mammalian subjects.

As illustrated by the foregoing examples, additional neurobiologically active compositions of the invention will be effective to inhibit cellular uptake of multiple biogenic amine neurotransmitters in mammalian subjects, for example, norepinephrine and serotonin, norepinephrine and dopamine, or serotonin and dopamine. In additional embodiments, the compositions of the invention are effective to inhibit cellular uptake of norepinephrine, serotonin and dopamine in mammalian subjects.

In further-detailed embodiments, as exemplified by the results presented in Table 2, neurobiologically active compositions of the invention surprisingly inhibit cellular reuptake of two, or three, biogenic amines selected from norepinephrine, serotonin and dopamine in a mammalian subject "non-uniformly" across the affected range of multiple targets. The distinct double and triple reuptake inhibition activity profiles demonstrated herein for exemplary compounds of the invention illustrate the powerful and unpredictable nature of structural variations for the compounds, and further evince the ability to follow the teachings of the present disclosure to produce, select, and employ other substituted candidates according to the invention having distinct activity profiles to fulfill additional therapeutic uses within the invention for treating diverse CNS disorders.

In exemplary aspects of the present disclosure, this differential inhibition may yield a profile of reuptake inhibition activities for all three neurotransmitters, norepinephrine, serotonin, and dopamine, respectively, in approximate reuptake inhibition profiles as determined in Table 2 selected from the following relative potencies: (1:1:10); (1:1:6); (1:2:1); (1:0.5:2); (1:1:3); (1:3:3); (1:1:2); and (1:1:1)-which values will correlate in a measurable way with novel in vivo reuptake inhibition profiles/ratios as will be readily determined by those skilled in the art.

In related aspects of the present disclosure, neurobiologically active compositions of the present disclosure inhibit cellular uptake of two, or three, biogenic amine neurotransmitters non-uniformly, for example by inhibiting uptake of at least one member of a group of transmitters including norepinephrine, serotonin, and dopamine by a factor of two- to ten-fold greater than a potency of the same composition to inhibit uptake of one or more different neurotransmitter(s). In exemplary aspects of the present disclosure, compositions of the present disclosure comprising a 1-heteroaryl-3-azabicyclo[3.1.0.]hexane inhibit cellular uptake of serotonin by a factor of at least approximately two-fold, or three-fold, greater than a potency of the same composition to inhibit uptake of norepinephrine, dopamine, or both norepinephrine and dopamine. In other aspects of the present disclosure, different 1-heteroaryl-3-azabicyclo[3.1.0.]hexanes of the present disclosure inhibit cellular uptake of dopamine by a factor of at least approximately two-fold, six-fold, or ten-fold, greater than a potency of the composition for inhibiting uptake of norepinephrine, serotonin, or both norepinephrine and serotonin. In additional exemplary aspects of the present disclosure, the compositions described herein inhibit cellular uptake of norepinephrine by a factor of at least approximately two-fold greater than a potency of the same composition for inhibiting uptake of serotonin. In different aspects of the present disclosure, compositions are provided that inhibit cellular uptake of dopamine by a factor of at least approximately two-fold greater than potency of the composition for inhibiting uptake of serotonin. In yet additional aspects of the present disclosure, neurobiologically active compositions are provided that exhibit approximately equivalent potency for inhibiting cellular uptake of norepinephrine and serotonin, while at the same time inhibiting dopamine uptake by a factor of at least approximately two-fold, or six-fold, greater than the potency for inhibiting uptake of norepinephrine and serotonin. In still other aspects of the present disclosure, compositions of the invention exhibit approximately equivalent potency for inhibiting cellular uptake of serotonin and dopamine, while at the same time inhibiting norepinephrine by a factor of no greater than approximately half the potency for inhibiting uptake of serotonin and dopamine. In certain aspects of the present disclosure, compositions of the present disclosure exhibit approximately equivalent potency for inhibiting cellular uptake of norepinephrine, serotonin, and dopamine.

Compounds of the present disclosure that inhibit the uptake of norepinephrine, serotonin, and/or dopamine have a wide range of therapeutic uses, principally to treat CNS disorders as described above. Certain CNS disorders contemplated herein will be more responsive to a compound of the present disclosure that preferentially inhibits, for example, dopamine uptake relative to norepinephrine and/or serotonin uptake, as in the case of some forms of depression. Other disorders, for example pain, will be determined to be more responsive to compounds of the present disclosure that more potently inhibit norepinenephrine reuptake relative to serotonin reuptake and dopamine reuptake. Other CNS disorders, for example, attention deficit hyperactivity disorder (ADHD), may respond better to compounds of the present disclosure that preferentially inhibit dopamine and norepinephrine reuptake relative to serotonin reuptake. Thus, the host of disclosed compounds described herein, which provide a range of reuptake inhibition profiles/ratios, will provide useful drug candidates for a diverse range of CNS disorders, and will effectively treat specific disorders with lower side effect profiles than currently available drugs.

It is to be understood that this invention is not limited to the particular formulations, process steps, and materials disclosed herein as such formulations, process steps, and materials may vary somewhat. It is also to be understood that the terminology employed herein is used for the purpose of describing particular embodiments only and is not intended to be limiting since the scope of the present invention will be limited only by the appended claims.

### References

Basile, A.S., et al., .J. Pharmacol. Exp. Ther., 321:1208-1225 (2007)
Skolnick, P. et al., Eur. J. Pharmacol. 461:99 (2003)
Skolnick, P. et al., Life Sci. 73: 3175-3179 (2003)
Skolnick, P., et al., CNS Drug Reviews (2006)
Briley, M., Hum. Psychopharmacol. Clin. Exp. 19:S21-S25 (2004)
Skolnick, P. in "Dopamine and glutamate in psychiatric disorders," W. Schmidt, Editor; Humana Press, Totowa, Chapter 9, pp. 199-214 (2005)
Atkinson, J.H. et al., Pain 83:137-145 (1999)
Povlock, S.L. and Amara, S.G., in "Neurotransmitter transporters: structure, function, and regulation," Reith MEA, Editor, Humana Press, Totowa, pp.1-28 (1997)
Eshleman, A.J. et al., Journal of Pharmacology & Experimental Therapeutics 289:877-885 (1999)
"Nitrogen Protecting Groups in Organic Synthesis", John Wiley and Sons, New York, N.Y., 1981, Chapter 7
"Nitrogen Protecting Groups in Organic Chemistry", Plenum Press, New York, N.Y., 1973, Chapter 2
Green, T.W. and Wuts, P.G.M. in "Protective Groups in Organic Chemistry", 3rd edition, John Wiley & Sons, New York, N.Y., 1999
Quick Reference to the Diagnostic Criteria From DSM-IV (Diagnostic and Statistical Manual of Mental Disorders, Fourth Edition), The American Psychiatric Association, Washington, D.C., 1994
Perovic, S. and Muller, W.E., Arzneimittelforschung 45: 1145-1148 (1995)
Gu, H., et al. J. Biol. Chem. 269:7124-7130 (1994)
US Patent No. 4,122,193; Scherm et al.; October 24, 1978
US Patent No. 6,132,724; Blum; October 17, 2000
U.S. Patent Application Serial No. 11/371,178; Skolnick, et al.; filed March 7, 2006

## Claims

1. A compound of the following Formula I: 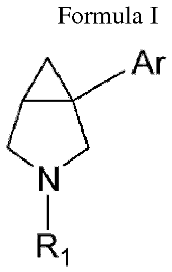 and pharmaceutically acceptable salts, enantiomers, polymorphs, solvates, or hydrates thereof, wherein:
Ar is a heterocyclic aryl group selected from furan, methylfuran, pyrrole, imidazole, pyrazole, triazole, tetrazole, thiazole, isothiazole, methoxypyridine, pyridazine, pyrazine, triazine, indole, methylindole, benzofuran, benzothiophene, benzothiazole, isoquinoline, cinnoline, phthalazine, quinazoline, chromane and isochromane, and Ar is either unsubstituted or substituted with one or more substituents independently selected from fluoro, chloro, bromo, iodo, -NO₂, -CN, -NH₂, carboxy, C₂₋₈ alkenyl, C₂₋₈ alkynyl, halo(C₁₋₈)alkyl, hydroxy, trifluoromethyl, C₃₋₈ cycloalkyl, C₁₋₃ alkoxy, C₁₋₃ alkoxy(C₁₋₃)alkyl, carboxy(C₁₋₃)alkyl, C₁₋₃ alkanoyl, halo(C₁₋₃)alkoxyl, C₁₋₈ alkylamino, and di(C₁₋₈)alkylamino; and
R₁ is selected from hydrogen, unsubstituted C₁₋₁₀ alkyl, C₃₋₈ cycloalkyl, C₂₋₁₀ alkenyl, and C₃₋₁₀ alkynyl, and substituted C₁₋₁₀ alkyl, C₃₋₁₀ alkenyl and C₃₋₁₀ alkynyl wherein the substituent is one or more of hydroxy, cyano, halogen, C₁₋₆ alkoxy, aryl substituted C₁₋₆ alkoxy, aryloxy, aryloxy substituted with one or more halogens, C₁₋₆ alkyl, C₁₋₆ alkyl independently substituted with one or more of cyano and halogen, C₁₋₄ alkoxy, and C₁₋₄ haloalkoxy.

2. The compound according to claim 1 and pharmaceutically acceptable salts, enantiomers, polymorphs, solvates, or hydrates thereof, wherein Ar is furan, methylfuran, fluorobenzothiophene, methoxypyridine, benzofuran, benzothiophene, chlorobenzothiophene, indole or methylindole and R₁ is hydrogen or unsubstituted C₁₋₁₀ alkyl.

3. The compound according to claim 2 and pharmaceutically acceptable salts, enantiomers, polymorphs, solvates, or hydrates thereof, wherein R₁ is hydrogen, methyl, ethyl, propyl or isopropyl.

4. The compound according to claim 1, wherein the compound is selected from the group consisting of:
1-(5-methylfuran-2-yl)-3-azabicyclo[3.1.0]hexane;
1-(6-methoxypyridin-3-yl)-3-azabicyclo[3.1.0]hexane;
1-(6-methoxypyridin-3-yl)-3-methyl-3-azabicyclo[3.1.0]hexane;
1-(benzofuran-2-yl)-3-methyl-3-azabicyclo[3.1.0]hexane;
1-(benzofuran-3-yl)-3-azabicyclo[3.1.0]hexane;
1-(benzofuran-3-yl)-3-methyl-3-azabicyclo[3.1.0]hexane;
1-methyl-2-(3-methyl-3-azabicyclo[3.1.0]hexan-1-yl)-1*H*-indole;
2-(3-ethyl-3-azabicyclo[3.1.0]hexan-1-yl)-1-methyl-1*H*-indole;
2-(3-isopropyl-3-azabicyclo[3.1.0]hexan-1-yl)-1-methyl-1*H*-indole;
1-methyl-5-(3-methyl-3-azabicyclo[3.1.0]hexan-1-yl)-1*H*-indole;
5-(3-ethyl-3-azabicyclo[3.1.0]hexan-1-yl)-1-methyl-1*H*-indole;
5-(3-isopropyl-3-azabicyclo[3.1.0]hexan-1-yl)-1-methyl-1*H*-indole;
1-(5-chlorobenzo[*b*]thiophen-3-yl)-3-azabicyclo[3.1.0]hexane;
1-(5-chlorobenzo[*b*]thiophen-2-yl)-3-methyl-3-azabicyclo[3.1.0]hexane;
1-(5-chlorobenzo[*b*]thiophen-3-yl)-3-methyl-3-azabicyclo[3.1.0]hexane;
1-(benzo[*b*]thiophen-2-yl)-3-aza-bicyclo[3.1.0]hexane;
1-(benzo[*b*]thiophen-2-yl)-3-methyl-3-aza-bicyclo[3.1.0]hexane;
1-(6-fluorobenzo[*b*]thiophen-2-yl)-3-aza-bicyclo[3.1.0]hexane;
1-(6-fluorobenzo[*b*]thiophen-2-yl)-3-methyl-3-aza-bicyclo[3.1.0]hexane;
1-(5-fluorobenzo[*b*]thiophen-2-yl)-3-aza-bicyclo[3.1.0]hexane;
1-(5-fluorobenzo[*b*]thiophen-2-yl)-3-methyl-3-aza-bicyclo[3.1.0]hexane;
N,N-dimethyl-2-(3-methyl-3-azabicyclo[3.1.0]hexane-1-yl)benzo[*b*]thiophen-5-amine;
1-(5-bromobenzo[*b*]thiophen-2-yl)-3-azabicyclo[3.1.0]hexane;
1-(5-bromobenzo[*b*]thiophen-2-yl)-3-methyl-3-azabicyclo[3.1.0]hexane;
2-(3-azabicyclo[3.1.0]hexan-1-yl)-*N*,*N*-dimethylbenzo[*b*]thiophen-5-amine;
2-(3-ethyl-3-azabicyclo[3.1.0]hexan-1-yl)-*N*,*N*-dimethylbenzo[*b*]thiophen-5-amine;
2-(3-isopropyl-3-azabicyclo[3.1.0]hexan-1-yl)-*N*,*N*-dimethylbenzo[*b*]thiophen-5-amine;
1-(6-bromobenzo[*b*]thiophen-2-yl)-3-azabicylco[3.1.0]hexane;
1-(6-bromobenzo[*b*]thiophen-2-yl)-3-methyl-3-azabicyclo[3.1.0]hexane;
2-(3-azabicyclo[3.1.0]hexan-1-yl)-*N*,*N*-dimethylbenzo[*b*]thiophen-6-amine;
*N*,*N*-dimethyl-2-(3-methyl-3-azabicyclo[3.1.0]hexan-1-yl)benzo[*b*]thiophen-6-amine;
2-(3-ethyl-3-azabicyclo[3.1.0]hexan-1-yl)-*N*,*N*-dimethylbenzo[*b*]thiophen-6-amine;
2-(3-isopropyl-3-azabicyclo[3.1.0]hexan-l-yl)-*N*,*N*-dimethylbenzo[*b*]thiophen-6-amine; and
2-(3-azabicyclo[3.1.0]hexan-1-yl)-benzo[*d*]thiazole;
and pharmaceutically acceptable salts, enantiomers, polymorphs, solvates, or hydrates thereof.

5. The compound according to claim 1 selected from the group consisting of:
(1*S*,5*S*)-1-(benzo[*b*]thiophen-2-yl)-3-azabicyclo[3.1.0]hexane;
(1*R*,5*R*)-1-(benzo[*b*]thiophen-2-yl)-3-azabicyclo[3.1.0]hexane;
(1*S*,5*S*)-1-(benzo[*b*]thiophen-2-yl)-3-methyl-3-azabicyclo[3.1.0]hexane;
(1*R*,5*R*)-1-(benzo[*b*]thiophen-2-yl)-3-methyl-azabicyclo[3.1.0]hexane;
(1*S*,5*S*)-1-(6-fluorobenzo[*b*]thiophen-2-yl)-3-aza-bicyclo[3.1.0]hexane; and
(1*R*,5*R*)-1-(6-fluorobenzo[*b*]thiophen-2-yl)-3-aza-bicyclo[3.1.0]hexane;
and pharmaceutically acceptable salts polymorphs, solvates, or hydrates thereof.

6. The compound according to claim 1 selected from the group consisting of:
1-(5-methylfuran-2-yl)-3-azabicyclo[3.1.0]hexane;
1-(6-methoxypyridin-3-yl)-3-azabicyclo[3.1.0]hexane;
1-(6-methoxypyridin-3-yl)-3-methyl-3-azabicyclo[3.1.0]hexane;
1-(benzofuran-2-yl)-3-methyl-3-azabicyclo[3.1.0]hexane;
1-(benzofuran-3-yl)-3-azabicyclo[3.1.0]hexane;
1-(benzofuran-3-yl)-3-methyl-3-azabicyclo[3.1.0]hexane;
1-methyl-2-(3-methyl-3-azabicyclo[3.1.0]hexan-1-yl)-1*H*-indole;
2-(3-ethyl-3-azabicyclo[3.1.0]hexan-1-yl)-1-methyl-1*H*-indole;
2-(3-isopropyl-3-azabicyclo[3.1.0]hexan-1-yl)-1-methyl-1*H*-indole;
1-methyl-5-(3-methyl-3-azabicyclo[3.1.0]hexan-1-yl)-1*H*-indole;
5-(3-ethyl-3-azabicyclo[3.1.0]hexan-1-yl)-1-methyl-1*H*-indole;
5-(3-isopropyl-3-azabicyclo[3.1.0]hexan-1-yl)-1-methyl-1*H*-indole;
1-(5-chlorobenzo[*b*]thiophen-3-yl)-3-azabicyclo[3.1.0]hexane;
1-(5-chlorobenzo[*b*]thiophen-2-yl)-3-methyl-3-azabicyclo[3.1.0]hexane;
1-(5-chlorobenzo[*b*]thiophen-3-yl)-3-methyl-3-azabicyclo[3.1.0]hexane;
1-(benzo[*b*]thiophen-2-yl)-3-aza-bicyclo[3.1.0]hexane;
1-(benzo[*b*]thiophen-2-yl)-3-methyl-3-aza-bicyclo[3.1.0]hexane;
1-(6-fluorobenzo[*b*]thiophen-2-yl)-3-aza-bicyclo[3.1.0]hexane;
1-(6-fluorobenzo[*b*]thiophen-2-yl)-3-methyl-3-aza-bicyclo[3.1.0]hexane;
1-(5-fluorobenzo[*b*]thiophen-2-yl)-3-aza-bicyclo[3.1.0]hexane;
1-(5-fluorobenzo[*b*]thiophen-2-yl)-3-methyl-3-aza-bicyclo[3.1.0]hexane;
N,N-dimethyl-2-(3-methyl-3-azabicyclo[3.1.0]hexane-1-yl)benzo[*b*]thiophen-5-amine;
1-(5-bromobenzo[*b*]thiophen-2-yl)-3-azabicyclo[3.1.0]hexane;
1-(5-bromobenzo[*b*]thiophen-2-yl)-3-methyl-3-azabicyclo[3.1.0]hexane;
2-(3-azabicyclo[3.1.0]hexan-1-yl)-*N*,*N*-dimethylbenzo[*b*]thiophen-5-amine;
2-(3-ethyl-3-azabicyclo[3.1.0]hexan-1-yl)-*N*,*N*-dimethylbenzo[b]thiophen-5-amine;
2-(3-isopropyl-3-azabicyclo[3.1.0]hexan-1-yl)-*N*,*N*-dimethylbenzo[*b*]thiophen-5-amine;
1-(6-bromobenzo[*b*]thiophen-2-yl)-3-azabicylco[3.1.0]hexane;
1-(6-bromobenzo[*b*]thiophen-2-yl)-3-methyl-3-azabicyclo[3.1.0]hexane;
2-(3-azabicyclo[3.1.0]hexan-1-yl)-*N*,*N*-dimethylbenzo[*b*]thiophen-6-amine;
*N*,*N*-dimethyl-2-(3-methyl-3-azabicyclo[3.1.0]hexan-1-yl)benzo[*b*]thiophen-6-amine;
2-(3-ethyl-3-azabicyclo[3.1.0]hexan-1-yl)-*N*,*N*-dimethylbenzo[*b*]thiophen-6-amine;
2-(3-isopropyl-3-azabicyclo[3.1.0]hexan-1-yl)-*N*,*N*-dimethylbenzo[*b*]thiophen-6-amine; and
2-(3-azabicyclo[3.1.0]hexan-1-yl)-benzo[*d*]thiazole;
or a pharmaceutically acceptable salt thereof.

7. The compound according to claim 6 selected from the group consisting of:
(1*S*,5*S*)-1-(benzo[*b*]thiophen-2-yl)-3-azabicyclo[3.1.0]hexane;
(1*R*,5*R*)-1-(benzo[*b*]thiophen-2-yl)-3-azabicyclo[3.1.0]hexane;
(1*S*,5*S*)-1-(benzo[*b*]thiophen-2-yl)-3-methyl-3-azabicyclo[3.1.0]hexane;
(1*R*,5*R*)-1-(benzo[*b*]thiophen-2-yl)-3-methyl-azabicyclo[3.1.0]hexane;
(1*S*,5*S*)-1-(6-fluorobenzo[*b*]thiophen-2-yl)-3-aza-bicyclo[3.1.0]hexane; and
(1*R*,5*R*)-1-(6-fluorobenzo[*b*]thiophen-2-yl)-3-aza-bicyclo[3.1.0]hexane;
or a pharmaceutically acceptable salt thereof.

8. The compound according to claim 6, wherein the compound is 1-(benzo[*b*]thiophen-2-yl)-3-methyl-3-aza-bicyclo[3.1.0]hexane or a pharmaceutically acceptable salt thereof.

9. The compound according to claim 6, wherein the compound is 1-(6-fluorobenzo[*b*]thiophen-2-yl)-3-aza-bicyclo[3.1.0]hexane or a pharmaceutically acceptable salt thereof.

10. The compound according to claim 6, wherein the compound is 1-(6-fluorobenzo[*b*]thiophen-2-yl)-3-aza-bicyclo[3.1.0]hexane hydrochloride or 1-(benzo[*b*]thiophen-2-yl)-3-methyl-3-aza-bicyclo[3.1.0]hexane hydrochloride.

11. The compound according to claim 6, wherein the compound is 1-(benzo[*b*]thiophen-2-yl)-3-aza-bicyclo[3.1.0]hexane or a pharmaceutically acceptable salt thereof.

12. The compound according to claim 11, wherein the compound is 1-(benzo[*b*]thiophen-2-yl)-3-aza-bicyclo[3.1.0]hexane hydrochloride.

13. The compound according to claim 11, wherein the compound is (1*S*,5*S*)-(1-benzo[*b*]thiophen-2-yl)-3-azabicyclo[3.1.0]hexane or a pharmaceutically acceptable salt thereof.

14. The compound according to claim 13, wherein the compound is (1*S*,5*S*)-(1-benzo[*b*]thiophen-2-yl)-3-azabicyclo[3.1.0]hexane hydrochloride.

15. A pharmaceutical composition comprising a compound according to claims 1-14 or a pharmaceutically acceptable salt, enantiomer, polymorph, solvate, or hydrate thereof, and a pharmaceutically acceptable carrier or vehicle therefore.

16. The pharmaceutical composition according to claim 15 comprising a compound selected from the group consisting of:
1-(6-fluorobenzo[*b*]thiophen-2-yl)-3-aza-bicyclo[3.1.0]hexane;
1-(benzo[*b*]thiophen-2-yl)-3-aza-bicyclo[3.1.0]hexane; and
1-(benzo[*b*]thiophen-2-yl)-3-methyl-3-aza-bicyclo[3.1.0]hexane;or a pharmaceutically acceptable salt thereof.

17. The pharmaceutical composition according to claim 16 comprising (1*S*,5*S*)-(1-benzo[*b*]thiophen-2-yl)-3-azabicyclo[3.1.0]hexane or a pharmaceutically acceptable salt thereof.

18. A compound according to any one of claims 1-14 or a pharmaceutically acceptable salt, enantiomer, polymorph, solvate, or hydrate thereof, or a composition according to any one of claims 15, 16, or 17, for use in therapy.

19. A compound according to any one of claims 1-14 or a pharmaceutically acceptable salt, enantiomer, polymorph, solvate, or hydrate thereof, or a composition according to any one of claims 15, 16, or 17, for use in treating or preventing a central nervous system (CNS) disorder; optionally, wherein the CNS disorder is: depression, an anxiety disorder, an attention defecit disorder, obesity, substance abuse, PMDD (Premenstrual dysphoric disorder) or a cognitive disorder.

20. The compound for use according to claim 19, wherein the compound is selected from the group consisting of:
1-(6-fluorobenzo[*b*]thiophen-2-yl)-3-aza-bicyclo[3.1.0]hexane;
1-(benzo[*b*]thiophen-2-yl)-3-aza-bicyclo[3.1.0]hexane; and
1-(benzo[*b*]thiophen-2-yl)-3-methyl-3-aza-bicyclo[3.1.0]hexane;
or a pharmaceutically acceptable salt thereof.

21. The compound for use according to claim 20, wherein the compound is (1*S*,5*S*)-(1-benzo[*b*]thiophen-2-yl)-3-azabicyclo[3.1.0]hexane or a pharmaceutically acceptable salt thereof.

22. A method of making a 1-heteroaryl-3-azabicyclo[3.1.0]hexane of the following Formula III, 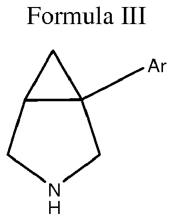 or a pharmaceutically acceptable salt thereof, wherein Ar is a heterocyclic aryl group selected from furan, methylfuran, pyrrole, imidazole, pyrazole, triazole, tetrazole, thiazole, isothiazole, methoxypyridine, pyridazine, pyrazine, triazine, indole, methylindole, benzofuran, benzothiophene, benzothiazole, isoquinoline, cinnoline, phthalazine, quinazoline, chromane and isochromane, and Ar is either unsubstituted or substituted with one or more substituents independently selected from fluoro, chloro, bromo, iodo, -NO₂, -CN, -NH₂, carboxy, C₂₋₈ alkenyl, C₂₋₈ alkynyl, halo(C₁₋₈)alkyl, hydroxy, trifluoromethyl, C₃₋₈ cycloalkyl, C₁₋₃ alkoxy, C₁₋₃ alkoxy(C₁₋₃)alkyl, carboxy(C₁₋₃)alkyl, C₁₋₃ alkanoyl, halo(C₁₋₃)alkoxyl, C₁₋₈ alkylamino, and di(C₁₋₈)alkylamino, comprising the steps of:
(a) reacting a compound of the following formula (i), 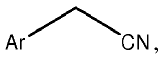 wherein Ar is defined as above, with epichlohydrin or an enantiomer thereof, to produce a compound of the following formula (ii), 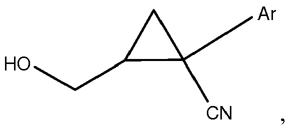 or an enantiomer or diastereomer thereof, wherein Ar is as defined above;
(b) reducing the compound of formula (ii) to produce a compound of the following formula (iii), 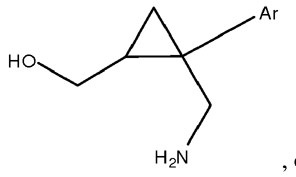 or an enantiomer or diastereomer thereof, wherein Ar is as defined above; and
(c) causing cyclization of the compound of formula (iii) to produce the 1-heteroaryl-3-azabicyclo[3.1.0]hexane, or an enantiomer or diastereomer thereof.

23. The method according to claim 22 further comprising:
(d) alkylating the 1-heteroaryl-3-azabicyclo[3.1.0]hexane produced in step (c) to produce a compound of the following Formula I, 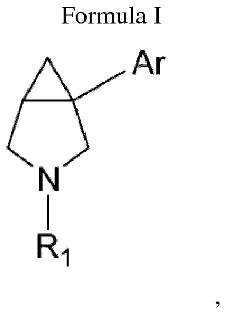 or a pharmaceutically acceptable salt thereof, wherein Ar is as defined in claim 22 and R₁ is selected from hydrogen, unsubstituted C₁₋₁₀ alkyl, C₃₋₈ cycloalkyl, C₂₋₁₀ alkenyl, and C₃₋₁₀ alkynyl, and substituted C₁₋₁₀ alkyl, C₃₋₁₀ alkenyl and C₃₋₁₀ alkynyl wherein the substituent is one or more of hydroxy, cyano, halogen, C₁₋₆ alkoxy, aryl substituted C₁₋₆ alkoxy, aryloxy, aryloxy substituted with one or more halogens, C₁₋₆ alkyl, C₁₋₆ alkyl independently substituted with one or more of cyano and halogen, C₁₋₄ alkoxy, and C₁₋₄ haloalkoxy.

24. A method of making a 1-heteroaryl-3-azabicyclo[3.1.0]hexane of the following Formula I, 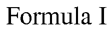 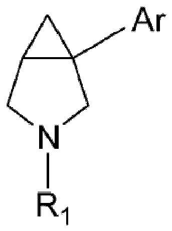 or a pharmaceutically acceptable salt thereof, wherein Ar is a heterocyclic aryl group selected from furan, methylfuran, pyrrole, imidazole, pyrazole, triazole, tetrazole, thiazole, isothiazole, methoxypyridine, pyridazine, pyrazine, triazine, indole, methylindole, benzofuran, benzothiophene, benzothiazole, isoquinoline, cinnoline, phthalazine, quinazoline, chromane and isochromane, and Ar is either unsubstituted or substituted with one or more substituents independently selected from fluoro, chloro, bromo, iodo, -NO₂, -CN, -NH₂, carboxy, C₂₋₈ alkenyl, C₂₋₈ alkynyl, halo(C₁₋₈)alkyl, hydroxy, trifluoromethyl, C₃₋₈ cycloalkyl, C₁₋₃ alkoxy, C₁₋₃ alkoxy(C₁₋₃)alkyl, carboxy(C₁₋₃)alkyl, C₁₋₃ alkanoyl, halo(C₁₋₃)alkoxyl, C₁₋₈ alkylamino, and di(C₁₋₈)alkylamino and R₁ is selected from hydrogen, unsubstituted C₁₋₁₀ alkyl, C₃₋₈ cycloalkyl, C₂₋₁₀ alkenyl, and C₃₋₁₀ alkynyl, and substituted C₁₋₁₀ alkyl, C₃₋₁₀ alkenyl and C₃₋₁₀ alkynyl wherein the substituent is one or more of hydroxy, cyano, halogen, C₁₋₆ alkoxy, aryl substituted C₁₋₆ alkoxy, aryloxy, aryloxy substituted with one or more halogens, C₁₋₆ alkyl, C₁₋₆ alkyl independently substituted with one or more of cyano and halogen, C₁₋₄ alkoxy, and C₁₋₄ haloalkoxy, comprising the steps of:
(a) reacting a compound of the following formula (iv), 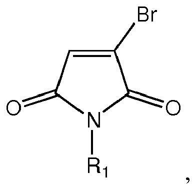 wherein R₁ is as defined above, with 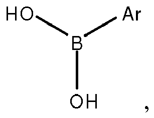 wherein Ar is as defined above, to produce a compound of the following formula (v), 9 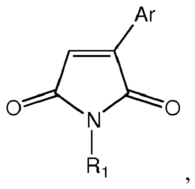 wherein R₁ and Ar are as defined above;
(b) causing cyclopropanation of the compound of formula (v) to produce a
compound of the following formula (vi), 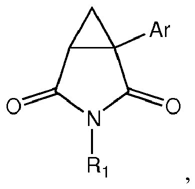 wherein R₁ and Ar are as defined above; and
(c) reducing the compound of formula (vi) to produce the 1-heteroaryl-3-azabicyclo[3.1.0]hexane.

25. A method of making a 1-heteroaryl-3-azabicyclo[3.1.0]hexane of the following Formula III, 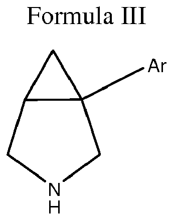 or a pharmaceutically acceptable salt thereof, wherein Ar is a heterocyclic aryl group selected from furan, methylfuran, pyrrole, imidazole, pyrazole, triazole, tetrazole, thiazole, isothiazole, methoxypyridine, pyridazine, pyrazine, triazine, indole, methylindole, benzofuran, benzothiophene, benzothiazole, isoquinoline, cinnoline, phthalazine, quinazoline, chromane and isochromane, and Ar is either unsubstituted or substituted with one or more substituents 10
independently selected from fluoro, chloro, bromo, iodo, -NO₂, -CN, -NH₂, carboxy, C₂₋₈ alkenyl, C₂₋₈ alkynyl, halo(C₁₋₈)alkyl, hydroxy, trifluoromethyl, C₃₋₈ cycloalkyl, C₁₋₃ alkoxy, C₁₋₃ alkoxy(C₁₋₃)alkyl, carboxy(C₁₋₃)alkyl, C₁₋₃ alkanoyl, halo(C₁₋₃)alkoxyl, C₁₋₈ alkylamino, and di(C₁₋₈)alkylamino, comprising the steps of:
(a) coupling a compound of the following formula (vii), 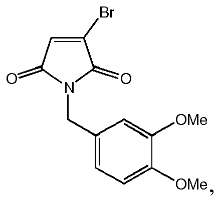 with 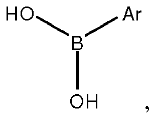 wherein Ar is as defined above, to produce a compound of
the following formula (viii), 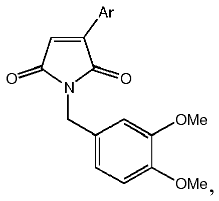 wherein Ar is as defined above;
(b) causing cyclopropanation of the compound of formula (viii) to produce a
compound of the following formula (ix), 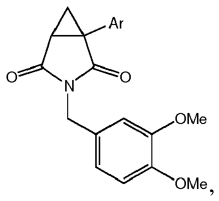 wherein Ar is as defined above;
(c) reducing the compound of formula (ix) to produce a compound of the
following formula (x), 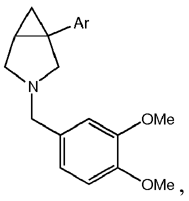 wherein Ar is as defined above; and
(d) deprotecting the compound of formula (x) to produce the 1-heteroaryl-3-azabicyclo[3.1.0]hexane.

26. The method according to claim 25 further comprising:
(e) alkylating the 1-heteroaryl-3-azabicyclo[3.1.0]hexane produced in step (d) to produce a compound of the following Formula I, 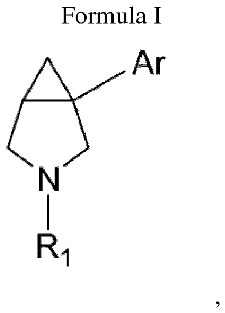 or a pharmaceutically acceptable salt thereof, wherein Ar is as defined in claim 25 and R₁ is selected from hydrogen, unsubstituted C₁₋₁₀ alkyl, C₃₋₈ cycloalkyl, C₂₋₁₀ alkenyl, and C₃₋₁₀ alkynyl, and substituted C₁₋₁₀ alkyl, C₃₋₁₀ alkenyl and C₃₋₁₀ alkynyl wherein the substituent is one or more of hydroxy, cyano, halogen, C₁₋₆ alkoxy, aryl substituted C₁₋₆ alkoxy, aryloxy, aryloxy substituted with one or more halogens, C₁₋₆ alkyl, C₁₋₆ alkyl independently substituted with one or more of cyano and halogen, C₁₋₄ alkoxy, and C₁₋₄ haloalkoxy.

## Patentansprüche

1. Verbindung der folgenden Formel I: 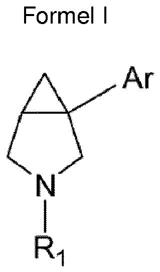 und pharmazeutisch annehmbare Salze, Enantiomere, Polymorphe, Solvate oder Hydrate davon, worin:
Ar eine heterozyklische Arylgruppe ist, die aus Furan, Methylfuran, Pyrrol, Imidazol, Pyrazol, Triazol, Tetrazol, Thiazol, Isothiazol, Methoxypyridin, Pyridazin, Pyrazin, Triazin, Indol, Methylindol, Benzofuran, Benzothiophen, Benzothiazol, Isoquinolin, Cinnolin, Phthalazin, Quinazolin, Chroman und Isochroman ausgewählt wird, und Ar entweder unsubstituiert oder substituiert ist mit einem oder mehr Substituenten, die unabhängig aus Fluor, Chlor, Brom, Jod, -NO₂, -CN, -NH₂, Carboxy, C₂₋₈-Alkenyl, C₂₋₈-Alkynyl, Halo(C₁₋₈)alkyl, Hydroxy, Trifluormethyl, C₃₋₈-Cycloalkyl, C₁₋₃-Alkoxy, C₁₋₃-Alkoxy(C₁₋₃)alkyl, Carboxy(C₁₋₃)alkyl, C₁₋₃-Alkanoyl, Halo(C₁₋₃)alkoxyl, C₁₋₈-Alkylamino und Di(C₁₋₈)alkylamino ausgewählt werden; und
R₁ aus Wasserstoff, unsubstituiertem C₁₋₁₀-Alkyl, C₃₋₈-Cycloalkyl, C₂₋₁₀-Alkenyl und C₃₋₁₀-Alkynyl und substituiertem C₁₋₁₀-Alkyl, C₃₋₁₀-Alkenyl und C₃₋₁₀-Alkynyl ausgewählt wird, worin der Substituent eines oder mehr von Hydroxy, Cyano, Halogen, C₁₋₆-Alkoxy, arylsubstituiertem C₁₋₆-Alkoxy, Aryloxy, Aryloxy substituiert mit einem oder mehr Halogenen, C₁₋₆-Alkyl, C₁₋₆-Alkyl unabhängig substituiert mit einem oder mehr von Cyano und Halogen, C₁₋₄-Alkoxy und C₁₋₄-Haloalkoxy ist.

2. Verbindung nach Anspruch 1 und pharmazeutisch annehmbare Salze, Enantiomere, Polymorphe, Solvate oder Hydrate davon, worin Ar Furan, Methylfuran, Fluorbenzothiophen, Methoxypyridin, Benzofuran, Benzothiophen, Chlorbenzothiophen, Indol oder Methylindol ist und R₁ Wasserstoff oder unsubstituiertes C₁₋₁₀-Alkyl ist.

3. Verbindung nach Anspruch 2 und pharmazeutisch annehmbare Salze, Enantiomere, Polymorphe, Solvate oder Hydrate davon, worin R₁ Wasserstoff, Methyl, Ethyl, Propyl oder Isopropyl ist.

4. Verbindung nach Anspruch 1, worin die Verbindung aus der aus Folgendem bestehenden Gruppe:
1-(5-Methylfuran-2-yl)-3-azabicyclo[3.1.0]hexan;
1-(6-Methoxypyridin-3-yl)-3-azabicyclo[3.1.0]hexan;
1-(6-Methoxypyridin-3-yl)-3-methyl-3-azabicyclo[3.1.0]hexan;
1-(Benzofuran-2-yl)-3-methyl-3-azabicyclo[3.1.0]hexan;
1-(Benzofuran-3-yl)-3-azabicyclo[3.1.0]hexan;
1-(Benzofuran-3-yl)-3-methyl-3-azabicyclo[3.1.0]hexan;
1-Methyl-2-(3-methyl-3-azabicyclo[3.1.0]hexan-1-yl)-1*H*-indol;
2-(3-Ethyl-3-azabicyclo[3.1.0]hexan-1-yl)-1-methyl-1*H-*indol;
2-(3-Isopropyl-3-azabicyclo[3.1.0]hexan-1-yl)-1-methyl-1*H*-indol;
1-Methyl-5-(3-methyl-3-azabicyclo[3.1.0]hexan-1-yl)-1*H*-indol;
5-(3-Ethyl-3-azabicyclo[3.1.0]hexan-1-yl)-1-methyl-1*H*-indol;
5-(3-Isopropyl-3-azabicyclo[3.1.0]hexan-1-yl)-1methyl-1*H*-indol;
1-(5-Chlorbenzo[*b*]thiophen-3-yl)-3-azabicyclo[3.1.0]hexan;
1-(5-Chlorbenzo[*b*]thiophen-2-yl)-3-methyl-3-azabicyclo[3.1.0]hexan;
1-(5-Chlorbenzo[*b*]thiophen-3-yl)-3-methyl-3-azabicyclo[3.1.0]hexan;
1-(Benzo[*b*]thiophen-2-yl)-3-aza-bicyclo[3.1.0]hexan;
1-(Benzo[*b*]thiophen-2-yl)-3-methyl-3-aza-bicyclo[3.1.0]hexan;
1-(6-Fluorbenzo[*b*]thiophen-2-yl)-3-aza-bicyclo[3.1.0]hexan;
1-(6-Fluorbenzo[*b*]thiophen-2-yl)-3-methyl-3-aza-bicyclo[3.1.0]hexan;
1-(5-Fluorbenzo[*b*]thiophen-2-yl)-3-aza-bicyclo[3.1.0]hexan;
1-(5-Fluorbenzo[*b*]thiophen-2-yl)-3-methyl-3-aza-bicyclo[3.1.0]hexan;
N,N-Dimethyl-2-(3-methyl-3-azabicyclo[3.1.0]hexan-1-yl)benzo[*b*]thiophen-5-amin;
1-(5-Brombenzo[*b*]thiophen-2-yl)-3-azabicyclo[3.1.0]hexan;
1-(5-Brombenzo[*b*]thiophen-2-yl)-3-methyl-3-azabicyclo[3.1.0]hexan;
2-(3-Azabicyclo[3.1.0]hexan-1-yl)-*N*,*N*-dimethylbenzo[*b*]thiophen-5-amin;
2-(3-Ethyl-3-azabicyclo[3.1.0]hexan-1-yl)-*N*,*N*-dimethylbenzo[*b*]thiophen-5-amin;
2-(3-Isopropyl-3-azabicyclo[3.1.0]hexan-1-yl)-*N*,*N*-dimethylbenzo[*b*]thiophen-5-amin;
1-(6-Brombenzo[*b*]thiophen-2-yl)-3-azabicylco[3.1.0]hexan;
1-(6-Brombenzo[*b*]thiophen-2-yl)-3-methyl-3-azabicyclo[3.1.0]hexan;
2-(3-Azabicyclo[3.1.0]hexan-1-yl)-*N*,*N*-dimethylbenzo[*b*]thiophen-6-amin;
*N*,*N*-Dimethyl-2-(3-methyl-3-azabicyclo[3.1.0]hexan-1-yl)benzo[*b*]thiophen-6-amin;
2-(3-Ethyl-3-azabicyclo[3.1.0]hexan-1-yl)-*N*,*N*-dimethylbenzo[*b*]thiophen-6-amin;
2-(3-Isopropyl-3-azabicyclo[3.1.0]hexan-1-yl)-*N*,*N*dimethylbenzo[*b*]thiophen-6-amin; und
2-(3-Azabicyclo[3.1.0]hexan-1-yl)-benzo*[d]*thiazol;
und pharmazeutisch annehmbaren Salzen, Enantiomeren, Polymorphen, Solvaten oder
Hydraten davon ausgewählt wird.

5. Verbindung nach Anspruch 1, die aus der aus Folgendem bestehenden Gruppe:
(1*S*,5*S*)-1-(Benzo[*b*]thiophen-2-yl)-3-azabicyclo[3.1.0]hexan;
(1*R*,5*R*)-1-(Benzo[*b*]thiophen-2-yl)-3-azabicyclo[3.1.0]hexan;
(1*S*,5*S*)-1-(Benzo[*b*]thiophen-2-yl)-3-methyl-3-azabicyclo[3.1.0]hexan;
(1*R*,5*R*)-1-(Benzo[*b*]thiophen-2-yl)-3-methyl-azabicyclo[3.1.0]hexan;
(1*S*,5*S*)-1-(6-Fluorbenzo[*b*]thiophen-2-yl)-3-aza-bicyclo[3.1.0]hexan; und
(1*R*,5*R*)-1-(6-Fluorbenzo[*b*]thiophen-2-yl)-3-aza-bicyclo[3.1.0]hexan;
und pharmazeutisch annehmbaren Salzen, Polymorphen, Solvaten oder Hydraten davon ausgewählt wird.

6. Verbindung nach Anspruch 1, die aus der aus Folgendem bestehenden Gruppe:
1-(5-Methylfuran-2-yl)-3-azabicyclo[3.1.0]hexan;
1-(6-Methoxypyridin-3-yl)-3-azabicyclo[3.1.0]hexan;
1-(6-Methoxypyridin-3-yl)-3-methyl-3-azabicyclo[3.1.0]hexan;
1-(Benzofuran-2-yl)-3-methyl-3-azabicyclo[3.1.0]hexan;
1-(Benzofuran-3-yl)-3-azabicyclo[3.1.0]hexan;
1-(Benzofuran-3-yl)-3-methyl-3-azabicyclo[3.1.0]hexan;
1-Methyl-2-(3-methyl-3-azabicyclo[3.1.0]hexan-1-yl)-1*H*-indol;
2-(3-Ethyl-3-azabicyclo[3.1.0]hexan-1-yl)-1-methyl-1*H*-indol;
2-(3-Isopropyl-3-azabicyclo[3.1.0]hexan-1-yl)-1-methyl-1*H*-indol;
1-Methyl-5-(3-methyl-3-azabicyclo[3.1.0]hexan-1-yl)-1*H*-indol;
5-(3-Ethyl-3-azabicyclo[3.1.0]hexan-1-yl)-1-methyl-1*H*-indol;
5-(3-Isopropyl-3-azabicyclo[3.1.0]hexan-1-yl)-1-methyl-1*H*-indol;
1-(5-Chlorbenzo[*b*]thiophen-3-yl)-3-azabicyclo[3.1.0]hexan;
1-(5-Chlorbenzo[*b*]thiophen-2-yl)-3-methyl-3-azabicyclo[3.1.0]hexan;
1-(5-Chlorbenzo[*b*]thiophen-3-yl)-3-methyl-3-azabicyclo[3.1.0]hexan;
1-(Benzo[*b*]thiophen-2-yl)-3-aza-bicyclo[3.1.0]hexan;
1-(Benzo[*b*]thiophen-2-yl)-3-methyl-3-aza-bicyclo[3.1.0]hexan;
1-(6-Fluorbenzo[*b*]thiophen-2-yl)-3-aza-bicyclo[3.1.0]hexan;
1-(6-Fluorbenzo[*b*]thiophen-2-yl)-3-methyl-3-aza-bicyclo[3.1.0]hexan;
1-(5-Fluorbenzo[*b*]thiophen-2-yl)-3-aza-bicyclo[3.1.0]hexan;
1-(5-Fluorbenzo[*b*]thiophen-2-yl)-3-methyl-3-aza-bicyclo[3.1.0]hexan;
N,N-Dimethyl-2-(3-methyl-3-azabicyclo[3.1.0]hexan-1-yl)benzo[*b*]thiophen-5-amin;
1-(5-Brombenzo[*b*]thiophen-2-yl)-3-azabicyclo[3.1.0]hexan;
1-(5-Brombenzo[*b*]thiophen-2-yl)-3-methyl-3-azabicyclo[3.1.0]hexan;
2-(3-Azabicyclo[3.1.0]hexan-1-yl)-*N,N*-dimethylbenzo[*b*]thiophen-5-amin;
2-(3-Ethyl-3-azabicyclo[3.1.0]hexan-1-yl)-*N,N*-dimethylbenzo[*b*]thiophen-5-amin;
2-(3-Isopropyl-3-azabicyclo[3.1.0]hexan-1-yl)-*N,N*-dimethylbenzo[*b*]thiophen-5-amin;
1-(6-Brombenzo[*b*]thiophen-2-yl)-3-azabicylco[3.1.0]hexan;
1-(6-Brombenzo[*b*]thiophen-2-yl)-3-methyl-3-azabicyclo[3.1.0]hexan;
2-(3-Azabicyclo[3.1.0]hexan-1-yl)-*N,N*-dimethylbenzo[*b*]thiophen-6-amin;
*N,N*-Dimethyl-2-(3-methyl-3-azabicyclo[3.1.0]hexan-1-yl)benzo[*b*]thiophen-6-amin;
2-(3-Ethyl-3-azabicyclo[3.1.0]hexan-1-yl)-*N,N*-dimethylbenzo[*b*]thiophen-6-amin;
2-(3-Isopropyl-3-azabicyclo[3.1.0]hexan-1-yl)-*N,N*-dimethylbenzo[*b*]thiophen-6-amin; und
2-(3-Azabicyclo[3.1.0]hexan-1-yl)-benzo*[d]*thiazol;
oder einem pharmazeutisch annehmbaren Salz davon ausgewählt wird.

7. Verbindung nach Anspruch 6, die aus der aus Folgendem bestehenden Gruppe:
(1*S*,5*S*)-1-(Benzo[*b*]thiophen-2-yl)-3-azabicyclo[3.1.0]hexan;
(1*R*,5*R*)-1-(Benzo[*b*]thiophen-2-yl)-3-azabicyclo[3.1.0]hexan;
(1*S*,5*S*)-1-(Benzo[*b*]thiophen-2-yl)-3-methyl-3-azabicyclo[3.1.0]hexan;
(1*R*,5*R*)-1-(Benzo[*b*]thiophen-2-yl)-3-methyl-azabicyclo[3.1.0]hexan;
(1*S*,5*S*)-1-(6-Fluorbenzo[*b*]thiophen-2-yl)-3-aza-bicyclo[3.1.0]hexan; und
(1*R*,5*R*)-1-(6-Fluorbenzo[*b*]thiophen-2-yl)-3-aza-bicyclo[3.1.0]hexan;
oder einem pharmazeutisch annehmbaren Salz davon ausgewählt wird.

8. Verbindung nach Anspruch 6, worin die Verbindung 1-(Benzo[*b*]thiophen-2-yl)-3-methyl-3-aza-bicyclo[3.1.0]hexan oder ein pharmazeutisch annehmbares Salz davon ist.

9. Verbindung nach Anspruch 6, worin die Verbindung 1-(6-Fluorbenzo[*b*]thiophen-2-yl)-3-aza-bicyclo[3.1.0]hexan oder ein pharmazeutisch annehmbares Salz davon ist.

10. Verbindung nach Anspruch 6, worin die Verbindung 1-(6-Fluorbenzo[*b*]thiophen-2-yl)-3-aza-bicyclo[3.1.0]hexanhydrochlorid oder 1-(Benzo[*b*]thiophen-2-yl)-3-methyl-3-aza-bicyclo[3.1.0]hexanhydrochlorid ist.

11. Verbindung nach Anspruch 6, worin die Verbindung 1-(Benzo[*b*]thiophen-2-yl)-3-aza-bicyclo[3.1.0]hexan oder ein pharmazeutisch annehmbares Salz davon ist.

12. Verbindung nach Anspruch 11, worin die Verbindung 1-(Benzo[*b*]thiophen-2-yl)-3-aza-bicyclo[3.1.0]hexanhydrochlorid ist.

13. Verbindung nach Anspruch 11, worin die Verbindung (1*S*,5*S*)-(1-Benzo[*b*]thiophen-2-yl)-3-azabicyclo[3.1.0]hexan oder ein pharmazeutisch annehmbares Salz davon ist.

14. Verbindung nach Anspruch 13, worin die Verbindung (1*S*,5*S*)-(1-Benzo[*b*]thiophen-2-yl)-3-azabicyclo[3.1.0]hexanhydrochlorid ist.

15. Pharmazeutische Zusammensetzung umfassend eine Verbindung nach Anspruch 1-14 oder ein pharmazeutisch annehmbares Salz, Enantiomer, Polymorph, Solvat oder Hydrat davon und einen pharmazeutisch annehmbaren Träger oder ein pharmazeutisch annehmbares Vehikel dafür.

16. Pharmazeutische Zusammensetzung nach Anspruch 15 umfassend eine Verbindung, die aus der aus Folgendem bestehenden Gruppe:
1-(6-Fluorbenzo[*b*]thiophen-2-yl)-3-azabicyclo[3.1.0]hexan;
1-(Benzo[*b*]thiophen-2-yl)-3-aza-bicyclo[3.1.0]hexan; und
1-(Benzo[*b*]thiophen-2-yl)-3-methyl-3-aza-bicyclo[3.1.0]hexan; oder einem pharmazeutisch annehmbaren Salz davon ausgewählt wird.

17. Pharmazeutische Zusammensetzung nach Anspruch 16 umfassend (1 S,5S)-(1 - Benzo[*b*]thiophen-2-yl)-3-azabicyclo[3.1.0]hexan oder ein pharmazeutisch annehmbares Salz davon.

18. Verbindung nach irgendeinem Anspruch 1-14 oder ein pharmazeutisch annehmbares Salz, Enantiomer, Polymorph, Solvat oder Hydrat davon oder eine Zusammensetzung nach irgendeinem Anspruch 15, 16 oder 17 für den therapeutischen Einsatz.

19. Verbindung nach irgendeinem Anspruch 1-14 oder ein pharmazeutisch annehmbares Salz, Enantiomer, Polymorph, Solvat oder Hydrat davon oder eine Zusammensetzung nach irgendeinem Anspruch 15, 16 oder 17 zur Verwendung bei der Behandlung oder Prävention einer Erkrankung des zentralen Nervensystems (ZNS); wahlweise, worin die Erkrankung des ZNS Depression, eine Angststörung, eine Aufmerksamkeitsdefizitstörung, Adipositas, Substanzmissbrauch, PMDD (prämenstruelle Dysphorie) oder eine kognitive Störung ist.

20. Verbindung zur Verwendung nach Anspruch 19, worin die Verbindung aus der aus Folgendem bestehenden Gruppe:
1-(6-Fluorbenzo[*b*]thiophen-2-yl)-3-aza-bicyclo[3.1.0]hexan;
1-(Benzo[*b*]thiophen-2-yl)-3-aza-bicyclo[3.1.0]hexan; und
1-(Benzo[*b*]thiophen-2-yl)-3-methyl-3-aza-bicyclo[3.1.0]hexan;
oder einem pharmazeutisch annehmbaren Salz davon ausgewählt wird.

21. Verbindung zur Verwendung nach Anspruch 20, worin die Verbindung (1*S*,5*S*)-(1-Benzo[*b*]thiophen-2-yl)-3-azabicyclo[3.1.0]hexan oder ein pharmazeutisch annehmbares Salz davon ist.

22. Verfahren zur Herstellung eines 1-Heteroaryl-3-azabicyclo[3.1.0]hexans der folgenden Formel III, 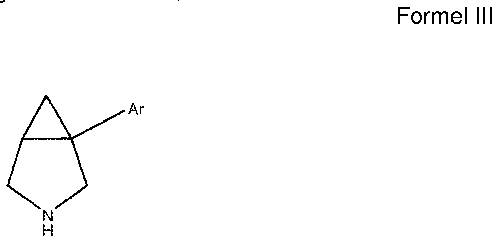 oder eines pharmazeutisch annehmbaren Salzes davon, worin Ar eine heterozyklische Arylgruppe ist, die aus Furan, Methylfuran, Pyrrol, Imidazol, Pyrazol, Triazol, Tetrazol, Thiazol, Isothiazol, Methoxypyridin, Pyridazin, Pyrazin, Triazin, Indol, Methylindol, Benzofuran, Benzothiophen, Benzothiazol, Isoquinolin, Cinnolin, Phthalazin, Quinazolin, Chroman und Isochroman ausgewählt wird, und Ar entweder unsubstituiert oder substituiert ist mit einem oder mehr Substituenten, die unabhängig aus Fluor, Chlor, Brom, Jod, -NO₂, - CN, -NH₂, Carboxy, C₂₋₈-Alkenyl, C₂₋₈-Alkynyl, Halo(C₁₋₈)alkyl, Hydroxy, Trifluoromethyl, C₃₋₈-Cycloalkyl, C₁₋₃-Alkoxy, C₁₋₃-Alkoxy(C₁₋₃)alkyl, Carboxy(C₁₋₃)alkyl, C₁₋₃-Alkanoyl, Halo(C₁₋₃)alkoxyl, C₁₋₈-Alkylamino und Di(C₁₋₈)alkylamino ausgewählt werden, umfassend die folgenden Schritte:
(a) Reagieren einer Verbindung der folgenden Formel (i), 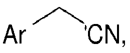 worin Ar wie oben definiert ist, mit Epichlohydrin oder einem Enantiomer davon, um
eine Verbindung der folgenden Formel (ii), 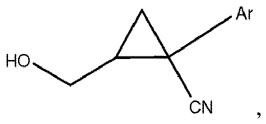 oder ein Enantiomer oder Diastereomer davon zu erzeugen, worin Ar wie oben definiert ist;
(b) Reduzieren der Verbindung von Formel (ii), um eine Verbindung der folgenden Formel (iii) 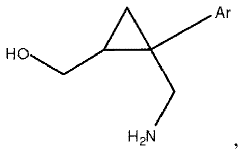 oder ein Enantiomer oder Diastereomer davon zu erzeugen, worin Ar wie oben definiert ist; und
(c) Herbeiführen von Cyclisierung der Verbindung von Formel (iii), um das 1-Heteroaryl-3-azabicyclo[3.1.0]hexan oder ein Enantiomer oder Diastereomer davon zu erzeugen.

23. Verfahren nach Anspruch 22, ferner umfassend:
(d) Alkylieren des in Schritt (c) erzeugten 1-Heteroaryl-3-azabicyclo[3.1.0]hexans, um eine Verbindung der folgenden Formel I, 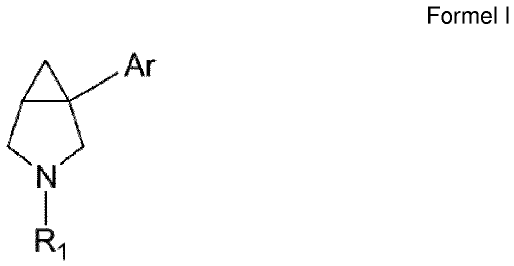 oder ein pharmazeutisch annehmbares Salz davon zu erzeugen, worin Ar wie in Anspruch 22 definiert ist und R₁ aus Wasserstoff, unsubstituiertem C₁₋₁₀-Alkyl, C₃₋₈-Cycloalkyl, C₂₋₁₀-Alkenyl und C₃₋₁₀-Alkynyl und substituiertem C₁₋₁₀-alkyl, C₃₋₁₀-Alkenyl und C₃₋₁₀-Alkynyl ausgewählt wird, worin der Substituent eines oder mehr von Hydroxy, Cyano, Halogen, C₁₋₆-Alkoxy, arylsubstituiertem C₁₋₆-Alkoxy, Aryloxy, Aryloxy substituiert mit einem oder mehr Halogenen, C₁₋₆-Alkyl, C₁₋₆-Alkyl unabhängig substituiert mit einem oder mehr von Cyano und Halogen, C₁₋₄-Alkoxy und C₁₋₄-Haloalkoxy ist.

24. Verfahren zur Herstellung eines 1-Heteroaryl-3-azabicyclo[3.1.0]hexans der folgenden Formel I, 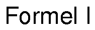 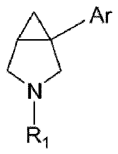 oder eines pharmazeutisch annehmbaren Salzes davon, worin Ar eine heterozyklische Arylgruppe ist, die aus Furan, Methylfuran, Pyrrol, Imidazol, Pyrazol, Triazol, Tetrazol, Thiazol, Isothiazol, Methoxypyridin, Pyridazin, Pyrazin, Triazin, Indol, Methylindol, Benzofuran, Benzothiophen, Benzothiazol, Isoquinolin, Cinnolin, Phthalazin, Quinazolin, Chroman und Isochroman ausgewählt wird, und Ar entweder unsubstituiert oder substituiert ist mit einem oder mehr Substituenten, die unabhängig aus Fluor, Chlor, Brom, Jod, -NO₂, - CN, -NH₂, Carboxy, C₂₋₈-Alkenyl, C₂₋₈-Alkynyl, Halo(C₁₋₈)alkyl, Hydroxy, Trifluoromethyl, C₃₋₈-Cycloalkyl, C₁₋₃-Alkoxy, C₁₋₃-Alkoxy(C₁₋₃)alkyl, Carboxy(C₁₋₃)alkyl, C₁₋₃-Alkanoyl, Halo(C₁₋₃)alkoxyl, C₁₋₈-Alkylamino und Di(C₁₋₈)alkylamino ausgewählt werden, und R₁ aus Wasserstoff, unsubstituiertem C₁₋₁₀-Alkyl, C₃₋₈-Cycloalkyl, C₂₋₁₀-Alkenyl und C₃₋₁₀-Alkynyl und substituiertem C₁₋₁₀Alkyl, C₃₋₁₀-Alkenyl und C₃₋₁₀-Alkynyl ausgewählt wird, worin der Substituent eines oder mehr von Hydroxy, Cyano, Halogen, C₁₋₆-Alkoxy, arylsubstituiertem C₁₋₆-Alkoxy, Aryloxy, Aryloxy substituiert mit einem oder mehr Halogenen, C₁₋₆-Alkyl, C₁₋₆-Alkyl unabhängig substituiert mit einem oder mehr von Cyano und Halogen, C₁₋₄-Alkoxy und C₁₋₄-Haloalkoxy ist, umfassend die folgenden Schritte:
(a) Reagieren einer Verbindung der folgenden Formel (iv), 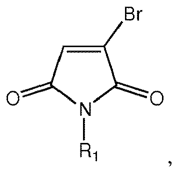 worin R₁ wie oben definiert ist, mit 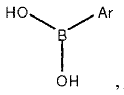 worin Ar wie oben definiert ist, um eine Verbindung der folgenden Formel (v) zu erzeugen, 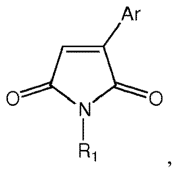 worin R₁ und Ar wie oben definiert sind;
(b) Herbeiführen von Cyclopropanation der Verbindung von Formel (v), um eine Verbindung der folgenden Formel (vi) zu erzeugen, 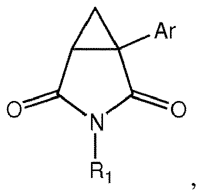 worin R₁ und Ar wie oben definiert sind; und
(c) Reduzieren der Verbindung von Formel (vi), um das 1-Heteroaryl-3-azabicyclo [3.1.0]hexan zu erzeugen.

25. Verfahren zur Herstellung eines 1-Heteroaryl-3-azabicyclo[3.1.0]hexans der folgenden Formel III, 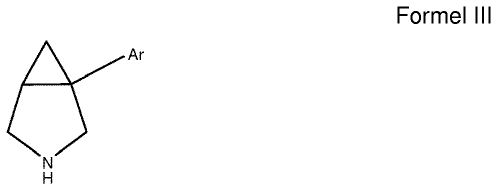 oder eines pharmazeutisch annehmbaren Salzes davon, worin Ar eine heterozyklische Arylgruppe ist, die aus Furan, Methylfuran, Pyrrol, Imidazol, Pyrazol, Triazol, Tetrazol, Thiazol, Isothiazol, Methoxypyridin, Pyridazin, Pyrazin, Triazin, Indol, Methylindol, Benzofuran, Benzothiophen, Benzothiazol, Isoquinolin, Cinnolin, Phthalazin, Quinazolin, Chroman und Isochroman ausgewählt wird, und Ar entweder unsubstituiert oder substituiert ist mit einem oder mehr Substituenten, die unabhängig aus Fluor, Chlor, Brom, Jod, -NO₂, - CN, -NH₂, Carboxy, C₂₋₈-Alkenyl, C₂₋₈-Alkynyl, Halo(C₁₋₈)alkyl, Hydroxy, Trifluoromethyl, C₃₋₈-Cycloalkyl, C₁₋₃-Alkoxy, C₁₋₃-Alkoxy(C₁₋₃)alkyl, Carboxy(C₁₋₃)alkyl, C₁₋₃-Alkanoyl, Halo(C₁₋₃)alkoxyl, C₁₋₈-Alkylamino und Di(C₁₋₈)alkylamino ausgewählt werden, umfassend die folgenden Schritte:
(a) Koppeln einer Verbindung der folgenden Formel (vii), 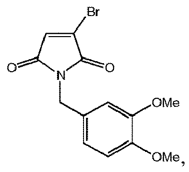 mit 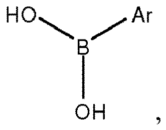 worin Ar wie oben definiert ist, um eine Verbindung der folgenden Formel (viii) zu erzeugen, 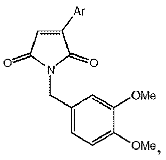 worin Ar wie oben definiert ist;
(b) Herbeiführen von Cyclopropanation der Verbindung von Formel (viii), um eine Verbindung der folgenden Formel (ix) zu erzeugen, 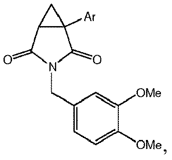 worin Ar wie oben definiert ist;
(c) Reduzieren der Verbindung von Formel (ix), um eine Verbindung der folgenden Formel (x) zu erzeugen, 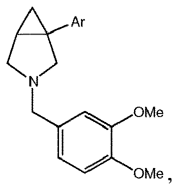 worin Ar wie oben definiert ist; und
Deprotektieren der Verbindung von Formel (x), um das 1-Heteroaryl-3-azabicyclo[3.1.0]hexan zu erzeugen.

26. Verfahren nach Anspruch 25, ferner umfassend:
(e) Alkylieren des in Schritt (d) erzeugten 1-Heteroaryl-3-azabicyclo[3.1.0]hexans, um eine Verbindung der folgenden Formel I, 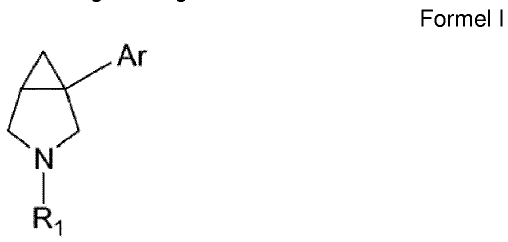 oder ein pharmazeutisch annehmbares Salz davon zu erzeugen, worin Ar wie in Anspruch 25 definiert ist und R₁ aus Wasserstoff, unsubstituiertem C₁₋₁₀-Alkyl, C₃₋₈-Cycloalkyl, C₂₋₁₀-Alkenyl und C₃₋₁₀-Alkynyl und substituiertem C₁₋₁₀Alkyl, C₃₋₁₀-Alkenyl und C₃₋₁₀-Alkynyl ausgewählt wird, worin der Substituent eines oder mehr von Hydroxy, Cyano, Halogen, C₁₋₆-Alkoxy, arylsubstituiertem C₁₋₆-Alkoxy, Aryloxy, Aryloxy substituiert mit einem oder mehr Halogenen, C₁₋₆-Alkyl, C₁₋₆-Alkyl unabhängig substituiert mit einem oder mehr von Cyano und Halogen, C₁₋₄-Alkoxy und C₁₋₄-Haloalkoxy ist.

## Revendications

1. Composé de formule I suivante : 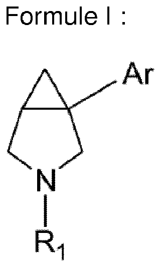 et sels, énantiomères, polymorphes, solvates ou hydrates pharmaceutiquement acceptables correspondants, où :
Ar est un groupe aryle hétérocyclique choisi parmi un furane, un méthylfurane, un pyrrole, un imidazole, un pyrazole, un triazole, un tétrazole, un thiazole, un isothiazole, une méthoxypyridine, une pyridazine, une pyrazine, une triazine, un indole, un méthylindole, un benzofurane, un benzothiophène, un benzothiazole, une isoquinoléine, une cinnoline, une phthalazine, une quinazoline, un chromane et un isochromane, et Ar est non substitué ou substitué par un ou plusieurs substituants choisis indépendamment parmi un groupe fluoro, chloro, bromo, iodo, -NO₂, -CN, -NH₂, carboxy, alcényle en C₂₋₈, alcynyle en C₂₋₈, halo(C₁₋₈)alkyle, hydroxy, trifluorométhyle, cycloalkyle en C₃₋₈, alcoxy en C₁₋₃, alkoxy(C₁₋₃)alkyle en C₁₋₃, carboxy(C₁₋₃)alkyle, alkanoyle en C₁₋₃, halo(C₁₋₃)alcoxyle, alkylamino en C₁₋₈ et di(C₁₋₈)alkylamino ; et
R₁ est choisi parmi l'hydrogène, un alkyle en C₁₋₁₀, un cycloalkyle en C₃₋₈, un alcényle en C₂₋₁₀ et un alcynyle en C₃₋₁₀ non substitué, et un alkyle en C₁₋₁₀, un alcényle en C₃₋₁₀ et un alcynyle en C₃₋₁₀ substitué, le substituant étant un ou plusieurs éléments choisis parmi un groupe hydroxy, cyano, halogène, alcoxy en C₁₋₆, alcoxy en C₁₋₆ substitué par de l'aryle, aryloxy, aryloxy substitué par un ou plusieurs halogènes, alkyle en C₁₋₆, alkyle en C₁₋₆ indépendamment substitué par un ou plusieurs groupes cyano et halogène, alcoxy en C₁₋₄ et haloalcoxy en C₁₋₄.

2. Composé selon la revendication 1 et sels, énantiomères, polymorphes, solvates ou hydrates correspondants pharmaceutiquement acceptables, dans lequel Ar est un furane, un méthylfurane, un fluorobenzothiophène, une méthoxypyridine, un benzofurane, un benzothiophène, un chlorobenzothiophène, un indole ou un méthylindole, et R₁ est de l'hydrogène ou un alkyle en C₁₋₁₀ non substitué.

3. Composé selon la revendication 2 et sels, énantiomères, polymorphes, solvates ou hydrates pharmaceutiquement acceptables correspondants, dans lequel R₁ est de l'hydrogène, un méthyle, un éthyle, un propyle ou un isopropyle.

4. Composé selon la revendication 1, ledit composé étant choisi dans le groupe constitué de :
1-(5-méthylfuran-2-yl)-3-azabicyclo[3,1,0]hexane ;
1-(6-méthoxypyridin-3-yl)-3-azabicyclo[3,1,0]hexane ;
1-(6-méthoxypyridin-3-yl)-3-méthyl-3-azabicyclo[3,1,0]hexane ;
1-(benzofuran-2-yl)-3-méthyl-3-azabicyclo[3,1,0]hexane ;
1-(benzofuran-3-yl)-3-azabicyclo[3,1,0]hexane ;
1-(benzofuran-3-yl)-3-méthyl-3-azabicyclo[3,1,0]hexane ;
1-méthyl-2-(3-méthyl-3-azabicyclo[3,1,0]hexan-1-yl)-1*H*-indole ;
2-(3-éthyl-3-azabicyclo[3,1,0]hexan-1-yl)-1-méthyl-1*H*-indole ;
2-(3-isopropyl-3-azabicyclo[3,1,0]hexan-1-yl)-1-méthyl-1*H*-idole
1-méthyl-5-(3-méthyl-3-azabicyclo[3,1,0]hexan-1-yl)-1*H*-indole ;
5-(3-éthyl-3-azabicyclo[3,1,0]hexan-1-yl)-1-méthyl-1*H*-indole ;
5-(3-isopropyl-3-azabicyclo[3,1,0]hexan-1-yl)-1méthyl-1*H*-idole
1-(5-chlorobenzo[*b*]thiophèn-3-yl)-3-azabicyclo[3,1,0]hexane ;
1-(5-chlorobenzo[*b*]thiophèn-2-yl)-3-méthyl-3-azabicyclo[3,1,0]hexane ;
1-(5-chlorobenzo[*b*]thiophèn-3-yl)-3-méthyl-3-azabicyclo[3,1,0]hexane ;
1-(benzo[*b*]thiophèn-2-yl)-3-aza-bicyclo[3,1,0]hexane ;
1-(benzo[*b*]thiophèn-2-yl)-3-méthyl-3-aza-bicyclo[3,1,0]hexane ;
1-(6-fluorobenzo[*b*]thiophèn-2-yl)-3-aza-bicyclo[3,1,0]hexane ;
1 -(6-fluorobenzo[*b*]thiophèn-2-yl)-3-méthyl-3-aza-bicyclo[3,1,0]hexane ;
1-(5-fluorobenzo[*b*]thiophèn-2-yl)-3-aza-bicyclo[3,1,0]hexane ;
1-(5-fluorobenzo[*b*]thiophèn-2-yl)-3-méthyl-3-aza-bicyclo[3,1,0]hexane ;
N,N-diméthyl-2-(3-méthyl-3-azabicyclo[3,1,0]hexane-1-yl)benzo[*b*]thiophèn-5-amine ;
1-(5-bromobenzo[*b*]thiophèn-2-yl)-3-azabicyclo[3,1,0]hexane ;
1-(5-bromobenzo[*b*]thiophèn-2-yl)-3-méthyl-3-azabicyclo[3,1,0]hexane ;
2-(3-azabicyclo[3,1,0]hexan-2-yl)-N,N-diméthylbenzo[*b*]thiophèn-5-amine ;
2-(3-éthyl-3-azabicyclo[3,1,0]hexan-1-yl)-N,N-diméthylbenzo[*b*]thiophèn-5-amine ;
2-(3-isopropyl-3-azabicyclo[3,1,0]hexan-1-yl)-N,N-diméthylbenzo[*b*]thiophèn-5-amine ;
1-(6-bromobenzo[*b*]thiophèn-2-yl)-3-azabicylco[3,1,0]hexane ;
1-(6-bromobenzo[*b*]thiophèn-2-yl)-3-méthyl-3-azabicyclo[3,1,0]hexane ;
2-(3-azabicyclo[3,1,0]hexan-1-yl)-N,N-diméthylbenzo[*b*]thiophèn-6-amine ;
N,N-diméthyl2-(3-méthyl-3-azabicyclo[3,1,0]hexan-1-yl)benzo[*b*]thiophèn-6-amine ;
2-(3-éthyl-3-azabicyclo[3,1,0]hexan-1-yl)-N,N-diméthylbenzo[*b*]thiophèn-6-amine ;
2-(3-isopropyl-3-azabicyclo[3,1,0]hexan-1-yl)-N,N-diméthylbenzo[*b*]thiophèn-6-amine ; et
2-(3-azabicyclo [3,1,0]hexan-1-yl)-benzo[d]thiazole ;
et sels, énantiomères, polymorphes, solvates ou hydrates pharmaceutiquement acceptables correspondants.

5. Composé selon la revendication 1, choisi dans le groupe constitué de :
(1S,5S,)-1-(benzo[*b*]thiophèn-2-yl)-3-azabicyclo[3,1,0]hexane ;
(1R,5R)-1-(benzo[*b*]thiophèn-2-yl)-3-azabicyclo[3,1,0]hexane ;
(1S,5S,)-1-(benzo[*b*]thiophèn-2-yl)-3-méthyl-3-azabicyclo[3,1,0]hexane;
(1R,5R)-1-(benzo[*b*]thiophèn-2-yl)-3-méthyl-azabicyclo[3,1,0]hexane ;
(1S,5S,)-1-(6-fluorobenzo[*b*]thiophèn-2-yl)-3-aza-bicyclo[3,1,0]hexane ; et
(1R,5R)-1-(6-fluorobenzo[*b*]thiophèn-2-yl)-3-aza-bicyclo[3,1,0]hexane ;
et sels, polymorphes, solvates ou hydrates pharmaceutiquement acceptables correspondants.

6. Composé selon la revendication 1, choisi dans le groupe constitué de :
1-(5-méthylfuran-2-yl)-3-azabicyclo[3,1,0]hexane ;
1-(6-méthoxypyridin-3-yl)-3-azabicyclo[3,1,0]hexane ;
1-(6-méthoxypyridin-3-yl)-3-méthyl-3-azabicyclo[3,1,0]hexane ;
1-(benzofuran-2-yl)-3-méthyl-3-azabicyclo[3,1,0]hexane ;
1-(benzofuran-3-yl)-3-azabicyclo[3,1,0]hexane ;
1-(benzofuran-3-yl)-3-méthyl-3-azabicyclo[3,1,0]hexane ;
1-méthyl-2-(3-méthyl-3-azabicyclo[3,1,0]hexan-1-yl)-1*H*-indole ;
2-(3-éthyl-3-azabicyclo[3,1,0]hexan-1-yl)-1-méthyl-1*H*-indole ;
2-(3-isopropyl-3-azabicyclo[3,1,0]hexan-1-yl)-1-méthyl-1*H*-indole ;
1-méthyl-5-(3-méthyl-3-azabicyclo[3,1,0]hexan-1-yl)-1*H*-indole ;
5-(3-éthyl-3-azabicyclo[3,1,0]hexan-1-yl)-1-méthyl-1*H*-indole ;
5-(3-isopropyl-3-azabicyclo[3,1,0]hexan-1-yl)-1-méthyl-1*H*-indole ;
1-(5-chlorobenzo[*b*]thiophèn-3-yl)-3-azabicyclo[3,1,0]hexane ;
1-(5-chlorobenzo[*b*]thiophèn-2-yl)-3-méthyl-3-azabicyclo[3,1,0]hexane ;
1-(5-chlorobenzo[*b*]thiophèn-3-yl)-3-méthyl-3-azabicyclo[3,1,0]hexane ;
1-(benzo[*b*]thiophèn-2-yl)-3-aza-bicyclo[3,1,0]hexane ;
1-(benzo[*b*]thiophèn-2-yl)-3-méthyl-3-aza-bicyclo[3,1,0]hexane ;
1-(6-fluorobenzo[*b*]thiophèn-2-yl)-3-aza-bicyclo[3,1,0]hexane ;
1-(6-fluorobenzo[*b*]thiophèn-2-yl)-3-méthyl-3-aza-bicyclo[3,1,0]hexane ;
1-(5-fluorobenzo[*b*]thiophèn-2-yl)-3-aza-bicyclo[3,1,0]hexane ;
1-(5-fluorobenzo[*b*]thiophèn-2-yl)-3-méthyl-3-aza-bicyclo[3,1,0]hexane ;
N,N-diméthyl-2-(3-méthyl-3-azabicyclo[3,1,0]hexane-1-yl)benzo[*b*]thiophèn-5-amine ;
1-(5-bromobenzo[*b*]thiophèn-2-yl)-3-azabicyclo[3,1,0]hexane ;
1-(5-bromobenzo[*b*]thiophèn-2-yl)-3-méthyl-3-azabicyclo[3,1,0]hexane ;
2-(3-azabicyclo[3,1,0]hexan-1-yl)-N,N-diméthylbenzo[*b*]thiophèn-5-amine ;
2-(3-éthyl-3-azabicyclo[3,1,0]hexan-1-yl)-N,N-diméthylbenzo[*b*]thiophèn-5-amine ;
2-(3-isopropyl-3-azabicyclo[3,1,0]hexan-1-yl)-N,N diméthylbenzo[*b*]thiophèn-5-amine ;
1-(6-bromobenzo[*b*]thiophèn-2-yl)-3-azabicylco[3,1,0]hexane ;
1-(6-bromobenzo[*b*]thiophèn-2-yl)-3-méthyl-3-azabicyclo[3,1,0]hexane;
2-(3-azabicyclo[3,1,0]hexan-1-yl)-N,N diméthylbenzo[*b*]thiophèn-6-amine ;
N,N-diméthyl-2-(3-méthyl-3-azabicyclo[3,1,0]hexan-1-yl)benzo[*b*]thiophèn-6-amine ;
2-(3-éthyl-3-azabicyclo[3,1,0]hexan-1-yl)-N,N-diméthylbenzo[*b*]thiophèn-6-amine ;
2-(3-isopropyl-3-azabicyclo[3,1,0]hexan-1-yl)-N,N-diméthylbenzo[*b*]thiophèn-6-amine ; et
2-(3-azabicyclo [3,1,0]hexan-1-yl)-benzo[d]thiazole ;
ou sel pharmaceutiquement acceptable correspondant.

7. Composé selon la revendication 6, choisi dans le groupe constitué de :
(1S,5S)-1-(benzo[*b*]thiophèn-2-yl)-3-azabicyclo[3,1,0]hexane ;
(1R,5R)-1-(benzo[*b*]thiophèn-2-yl)-3-azabicyclo[3,1,0]hexane ;
(1S,5S)-1-(benzo[*b*]thiophèn-2-yl)-3-méthyl-3-azabicyclo[3,1,0]hexane ; (1R,5R)-1-(benzo[*b*]thiophèn-2-yl)-3-méthyl-azabicyclo[3,1,0]hexane ;
(1S,5S)-1-(6-fluorobenzo[*b*]thiophèn-2-yl)-3-aza-bicyclo[3,1,0]hexane ; et
(1R,5R)-1-(6-fluorobenzo[*b*]thiophén-2-yl)-3-aza-bicyclo[3,1,0]hexane ;
ou sel pharmaceutiquement acceptable correspondant.

8. Composé selon la revendication 6, le composé étant du 1-(benzo[*b*]thiophèn-2-yl)-3-méthyl-3-aza-bicyclo[3,1,0]hexane ou un sel pharmaceutiquement acceptable correspondant.

9. Composé selon la revendication 6, le composé étant du 1-(6-fluorobenzo[*b*]thiophen-2-yl)-3-aza-bicyclo[3.1.0]hexane ou un sel pharmaceutiquement acceptable correspondant.

10. Composé selon la revendication 6, le composé étant de l'hydrochlorure de 1-(6-fluorobenzo[*b*]thiophèn-2-yl)-3-aza-bicyclo[3,1,0]hexane ou de l'hydrochlorure de 1-(benzo[*b*]thiophèn-2-yl)-3-méthyl-3-aza-bicyclo[3,1,0]hexane.

11. Composé selon la revendication 6, le composé étant du 1-(benzo[*b*]thiophèn-2-yl)-3-aza-bicyclo[3,1,0]hexane ou un sel pharmaceutiquement acceptable correspondant.

12. Composé selon la revendication 11, le composé étant de l'hydrochlorure de 1-(benzo[*b*]thiophèn-2-yl)-3-aza-bicyclo[3,1,0]hexane.

13. Composé selon la revendication 11, le composé étant du (1S,5S)-(1-benzo[*b*]thiophèn-2-yl)-3-azabicyclo[3,1,0]hexane ou un sel pharmaceutiquement acceptable correspondant.

14. Composé selon la revendication 13, le composé étant de l'hydrochlorure de (1S,5S)-(1-benzo[*b*]thiophén-2-yl)-3-azabicyclo[3,1,0]hexane.

15. Composition pharmaceutique comprenant un composé selon les revendications 1 à 14 ou un sel, un énantiomère, un polymorphe, un solvate ou un hydrate pharmaceutiquement acceptable correspondant, et un support ou un véhicule pharmaceutiquement acceptable correspondant.

16. Composition pharmaceutique selon la revendication 15, comprenant un composé choisi dans le groupe constitué de :
1-(6-fluorobenzo[*b*]thiophèn-2-yl)-3-azabicyclo[3,1,0]hexane ;
1-(benzo[*b*]thiophèn-2-yl)-3-aza-bicyclo[3,1,0]hexane ; et
1-(benzo[*b*]thiophèn-2-yl)-3-méthyl-3-aza-bicyclo[3,1,0]hexane ;
ou sel pharmaceutiquement acceptable correspondant.

17. Composition pharmaceutique selon la revendication 16, comprenant du (1S,5S)-(1-benzo[*b*]thiophèn-2-yl)-3-azabicyclo[3,1,0]hexane ou un sel pharmaceutiquement acceptable correspondant.

18. Composé selon l'une quelconque des revendications 1 à 14 ou sel, énantiomère, polymorphe, solvate ou hydrate pharmaceutiquement acceptable correspondant, ou composition selon l'une quelconque des revendications 15, 16 et 17, destinée à être utilisée dans un traitement thérapeutique.

19. Composé selon l'une quelconque des revendications 1 à 14 ou sel, énantiomère, polymorphe, solvate ou hydrate pharmaceutiquement acceptable correspondant, ou composition selon l'une quelconque des revendications 15, 16 et 17, destinée à être utilisée dans le traitement ou la prévention d'un trouble du système nerveux central (SNC) ; le trouble du SNC étant, éventuellement : une dépression, un trouble d'anxiété, un trouble déficitaire de l'attention, une obésité, un abus de drogues, un TDPM (trouble dysphorique prémenstruel) ou un trouble cognitif.

20. Composé destiné à être utilisé selon la revendication 19, ledit composé étant choisi dans le groupe constitué de :
1-(6-fluorobenzo[*b*]thiophèn-2-yl)-3-aza-bicyclo[3,1,0]hexane ;
1-(benzo[*b*]thiophèn-2-yl)-3-aza-bicyclo[3,1,0]hexane ; et
1-(benzo[*b*]thiophèn-2-yl)-3-méthyl-3-aza-bicyclo[3,1,0]hexane ;
ou sel pharmaceutiquement acceptable correspondant.

21. Composé destiné à être utilisé selon la revendication 20, le composé étant du (1S,5S)-(1-benzo[*b*]thiophèn-2-yl)-3-azabicyclo[3,1,0]hexane ou un sel pharmaceutiquement acceptable correspondant.

22. Procédé de préparation d'un 1-hétéroaryl-3-azabicyclo[3,1,0]hexane de formule III suivante, 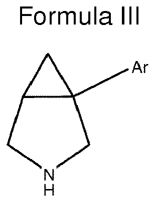 ou d'un sel pharmaceutiquement acceptable correspondant, où Ar est un groupe aryle hétérocyclice choisi parmi un furane, un méthylfurane, un pyrrole, un imidazole, un pyrazole, un triazole, un tétrazole, un thiazole, un isothiazole, une méthoxypyridine, une pyridazine, une pyrazine, une triazine, un indole, un méthylindole, un benzofurane, un benzothiophène, un benzothiazole, une isoquinoléine, une cinnoline, une phthalazine, une quinazoline, un chromane et un isochromane, et Ar est non substitué ou substitué par un ou plusieurs substituants choisis indépendamment parmi un groupe fluoro, chloro, bromo, iodo, -NO₂, -CN, - NH2, carboxy, alcényle en C₂₋₈, alcynyle en C₂₋₈, halo(C₁₋₈)alkyle, hydroxy, trifluorométhyle, cycloalkyle en C₃₋₈, alcoxy en C₁₋₃, alkoxy(C₁₋₃)alkyle en C₁₋₃, carboxy(C₁₋₃)alkyle, alkanoyle en C₁₋₃, halo(C₁₋₃)alcoxyle, alkylamino en C₁₋₈ et di(C₁₋₈)alkylamino, comprenant les étapes consistant à :
(a) faire réagir un composé de formule (i) suivante, 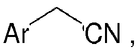 où Ar est défini tel que ci-dessus, avec de l'épichlohydrine ou un énantiomère correspondant, afin de
produire un composé de formule (ii) suivante, 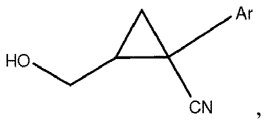 ou un énantiomère ou un diastéréomère correspondant, où Ar est tel que défini ci-dessus ;
(b) réduire le composé de formule (ii) afin de produire un composé de formule (iii) suivante, 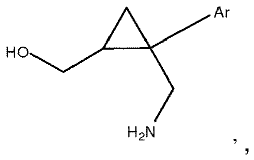 ou un énantiomère ou un diastéréomère correspondant, où Ar est défini tel que ci-dessus ; et
(c) provoquer la cyclisation du composé de formule (iii) afin de produire le 1-hétéroaryl-3-azabicyclo[3,1,0]hexane, ou un énantiomère ou un diastéréomère correspondant.

23. Procédé selon la revendication 22, consistant en outre à :
(d) alkyler le 1-hétéroaryl-3-azabicyclo[3,1,0]hexane produit à l'étape (c) afin de produire un composé de formule I suivante, 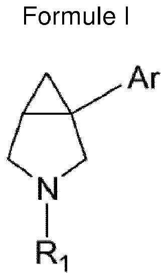 ou un sel pharmaceutiquement acceptable correspondant, oùAr est défini selon la revendication 22 et R₁ est choisi parmi l'hydrogène, un alkyle en C₁₋₁₀, un cycloalkyle en C₃₋₈, un alcényle en C₂₋₁₀ et un alcynyle en C₃₋₁₀ non substitué, et un alkyle en C₁₋₁₀, un alcényle en C₃₋₁₀ et un alcynyle en C₃₋₁₀ substitué, le substituant étant un ou plusieurs éléments choisis parmi un groupe hydroxy, cyano, halogène, alcoxy en C₁₋₆, alcoxy en C₁₋₆ substitué par de l'aryle, aryloxy, aryloxy substitué par un ou plusieurs halogènes, alkyle en C₁₋₆, alkyle en C₁₋₆ indépendamment substitué par un ou plusieurs groupes cyano et halogène, alcoxy en C₁₋₄ et haloalcoxy en C₁₋₄.

24. Procédé de préparation d'un 1-hétéroaryl-3-azabicyclo[3,1,0]hexane de formule I suivante, 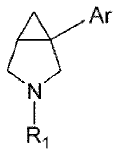 ou d'un sel pharmaceutiquement acceptable correspondant, où Ar est un groupe aryle hétérocyclice choisi parmi un furane, un méthylfurane, un pyrrole, un imidazole, un pyrazole, un triazole, un tétrazole, un thiazole, un isothiazole, une méthoxypyridine, une pyridazine, une pyrazine, une triazine, un indole, un méthylindole, un benzofurane, un benzothiophène, un benzothiazole, une isoquinoléine, une cinnoline, une phthalazine, une quinazoline, un chromane et un isochromane, aet Ar est non substitué ou substitué par un ou plusieurs substituants choisis indépendamment parmi un groupe fluoro, chloro, bromo, iodo, -N02, -CN, - NH2, carboxy, alcényle en C₂₋₈, alcynyle en C₂₋₈, halo(C₁₋₈)alkyle, hydroxy, trifluorométhyl, cycloalkyle en C₃₋₈, alcoxy en C₁₋₃, alcoxy(C₁₋₃)alkyle en C₁₋₃, carboxy(C₁₋₃)alkyle, alkanoyle en C₁₋₃, halo(C₁₋₃)alcoxyle, alkylamino en C₁₋₈ et di(C₁₋₈)alkylamino, et R₁ est choisi parmi l'hydrogène, un alkyle en C₁₋₁₀, un cycloalkyle en C₃₋₈, un alcényle en C₂₋₁₀ et un alcynyle en C₃₋₁₀ non substitué, et un alkyle en C₃₋₁₀, un alcényle en C₃₋₁₀ et un alcynyle en C₃₋₁₀ substitué, le substituant étant un ou plusieurs éléments choisis parmi un groupe hydroxy, cyano, halogène, alcoxy en C₁₋₆, alcoxy en C₁₋₆ substitué par de l'aryle, aryloxy, aryloxy substitué par un ou plusieurs halogènes, alkyle en C₁₋₆, alkyle en C₁₋₆ indépendamment substitué par un ou plusieurs groupes cyano et halogène, alcoxy en C₁₋₄ et haloalcoxy en C₁₋₄, comprenant les étapes consistant à
(a) faire réagir un composé de formule (iv) suivante, 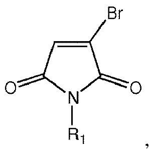 où R₁ est défini tel que ci-dessus, avec 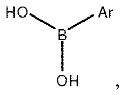 où Ar est défini tel que ci-dessus, afin de produire un composé de formule (v) suivante, 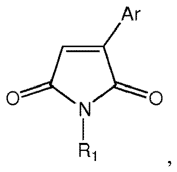 où R₁ et Ar sont tels que définis ci-dessus ;
(b) provoquer la cyclopropanation du composé de formule (v) afin de produire
un composé de formule (vi) suivante, 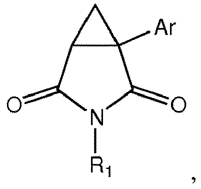 où R₁ et Ar sont tels que définis ci-dessus ; et
(c) réduire le composé de formule (vi) afin de produire le 1-hétéroaryl-3-azabicyclo[3,1,0]hexane.

25. Procédé de préparation d'un 1-hétéroaryl-3-azabicyclo[3,1,0]hexane de formule III suivante, 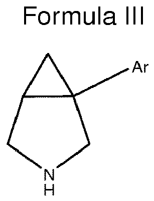 ou d'un sel pharmaceutiquement acceptable correspondant, où Ar est un groupe aryle hétérocyclice choisi parmi un furane, un méthylfurane, un pyrrole, un imidazole, un pyrazole, un triazole, un tétrazole, un thiazole, un isothiazole, une méthoxypyridine, une pyridazine, une pyrazine, une triazine, un indole, un méthylindole, un benzofurane, un benzothiophène, un benzothiazole, une isoquinoléine, une cinnoline, une phthalazine, une quinazoline, un chromane et un isochromane, et Ar est non substitué ou substitué par un ou plusieurs substituants choisis indépendamment parmi un groupe fluoro, chloro, bromo, iodo, -NO₂, -CN, - NH₂, carboxy, alcényle en C₂₋₈, alcynyle en C₂₋₈, halo(C₁₋₈)alkyle, hydroxy, trifluorométhyle, cycloalkyle en C₃₋₈, alcoxy en C₁₋₃, alkoxy(C₁₋₃)alkyle en C₁₋₃, carboxy(C₁₋₃)alkyle, alkanoyle en C₁₋₃, halo(C₁₋₃)alcoxyle, alkylamino en C₁₋₈ et di(C₁₋₈)alkylamino, comprenant les étapes consistant à :
(a) coupler un composé de formule (vii) suivante, 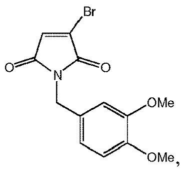 avec 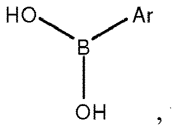 où Ar est défini tel que ci-dessus, afin de produire un composé de formule (viii) suivante, 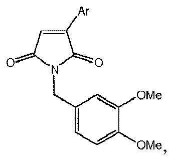 où Ar est défini tel que ci-dessus ;
(b) provoquer la cyclopropanation du composé de formule (viii) afin de
produire un composé de formule (ix) suivante, 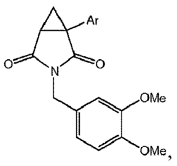 où Ar est tel que défini ci-dessus ;
(c) réduire le composé de formule (ix) afin de produire un composé de
formule (x) suivante, 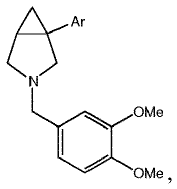 où Ar est défini tel que ci-dessus ; et (d) déprotéger le composé de formule (x) afin de produire le 1-hétéroaryl-3-azabicyclo[3,1,0]hexane.

26. Procédé selon la revendication 25, consistant en outre à :
(e) alkyler le 1-hétéroaryl-3-azabicyclo[3,1,0]hexane produit à l'étape (d) afin de produire un composé de formule I suivante, 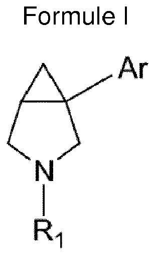 ou un sel pharmaceutiquement acceptable correspondant, où Ar est défini selon la revendication 25 et R₁ est choisi parmi l'hydrogène, un alkyle en C₁₋₁₀, un cycloalkyle en C₃₋₈, un alcényle en C₂₋₁₀ et un alcynyle en C₃₋₁₀ non substitué, et un alkyle en C₁₋₁₀, un alcényle en C₃₋₁₀ et un alcynyle en C₃₋₁₀ substitué, le substituant étant un ou plusieurs éléments choisis parmi un groupe hydroxy, cyano, halogène, alcoxy en C₁₋₆, alcoxy en C₁₋₆ substitué par de l'aryle, aryloxy, aryloxy substitué par un ou plusieurs halogènes, alkyle en C₁₋₆, alkyle en C₁₋₆ indépendamment substitué par un ou plusieurs groupes cyano et halogène, alcoxy en C₁₋₄ et haloalcoxy en C₁₋₄.
